(19) Europäisches Patentamt
European Patent Office
Office européen des brevets

(11) **EP 3 527 274 B1**

(12) **EUROPEAN PATENT SPECIFICATION**

(45) Date of publication and mention
of the grant of the patent:
**24.12.2025 Bulletin 2025/52**

(21) Application number: **18206718.1**

(22) Date of filing: **02.11.2012**

(51) International Patent Classification (IPC):
*B01D 15/08* $^{(2006.01)}$   *B01D 15/14* $^{(2006.01)}$
*B01D 15/32* $^{(2006.01)}$   *B01D 15/36* $^{(2006.01)}$
*B01D 15/38* $^{(2006.01)}$   *C07K 16/00* $^{(2006.01)}$
*C07K 1/16* $^{(2006.01)}$   *C07K 1/18* $^{(2006.01)}$
*B01D 15/42* $^{(2006.01)}$   *C07K 1/22* $^{(2006.01)}$

(52) Cooperative Patent Classification (CPC):
**B01D 15/14; B01D 15/424; C07K 1/16; C07K 1/22;**
B01D 15/327; B01D 15/363; B01D 15/3804;
B01D 15/3847; C07K 1/165; C07K 1/18

(54) **OVERLOAD AND ELUTE CHROMATOGRAPHY**

ÜBERSTROM- UND ELUTIONSCHROMATOGRAPHIE

CHROMATOGRAPHIE PAR SURCHARGE ET ÉLUTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.11.2011 US 201161554898 P**

(43) Date of publication of application:
**21.08.2019 Bulletin 2019/34**

(60) Divisional application:
**25217110.3**

(62) Document number(s) of the earlier application(s) in
accordance with Art. 76 EPC:
**17157670.5 / 3 257 564**
**12845437.8 / 2 773 438**

(73) Proprietor: **F. Hoffmann-La Roche AG**
**4070 Basel (CH)**

(72) Inventors:
• NADARAJAH, Deepa
South San Francisco, California 94080-4990 (US)
• MEHTA, Amit
South San Francisco, California 94080-4990 (US)

(74) Representative: **Mewburn Ellis LLP**
**Aurora Building**
**Counterslip**
**Bristol BS1 6BX (GB)**

(56) References cited:
WO-A1-2011/017514   WO-A1-2011/035282
WO-A1-2012/068134   US-A1- 2007 060 741

• H F LIU ET AL.: "Exploration of overloaded cation exchange chromatography for monoclonal antibody purification", JOURNAL OF CHROMATOGRAPHY A, vol. 1218, 12 August 2011 (2011-08-12), ELSEVIER SCIENCE PUBLISHERS B.V., pages 6943 - 6952, XP002668917, ISSN: 0021-9673
• ARICK BROWN ET AL: "Overloading ion-exchange membranes as a purification step for monoclonal antibodies", BIOTECHNOLOGY AND APPLIED BIOCHEMISTRY, vol. 56, no. 2, 11 June 2010 (2010-06-11), pages 59 - 70, XP055137278, ISSN: 0885-4513, DOI: 10.1042/BA20090369
• D. KELLEY BRIAN ET AL: "Weak partitioning chromatography for anion exchange purification of monoclonal antibodies", BIOTECHNOLOGY AND BIOENGINEERING, vol. 101, no. 3, 26 August 2008 (2008-08-26), Hoboken, USA, pages 553 - 566, XP093211672, ISSN: 0006-3592, DOI: 10.1002/bit.21923
• ANONYMOUS: "Ion Exchange Chromatography & Chromatofocusing Principles and Methods GE Healthcare", GE HEALTHCARE, 29 March 2023 (2023-03-29), XP093211669, Retrieved from the Internet <URL:www>

**EP 3 527 274 B1**

(Cont. next page)

- **LENHOFF ABRAHAM M.: "Protein adsorption and transport in polymer-functionalized ion-exchangers", JOURNAL OF CHROMATOGRAPHY A, vol. 1218, no. 49, 1 December 2011 (2011-12-01), AMSTERDAM, NL, pages 8748 - 8759, XP093211687, ISSN: 0021-9673, DOI: 10.1016/j.chroma.2011.06.061**
- **HAGEL, L, ET AL: "Handbook of Process Chromatography", 2008, pages: 1 - 20**
- **LENHOFF ABRAHAM M.: "Protein adsorption and transport in polymer-functionalized ion-exchangers", JOURNAL OF CHROMATOGRAPHY A, vol. 1218, no. 49, 1 December 2011 (2011-12-01), AMSTERDAM, NL, pages 8748 - 8759, XP093211711, ISSN: 0021-9673, DOI: 10.1016/j.chroma.2011.06.061**
- **HARINARAYAN C. ET AL: "An exclusion mechanism in ion exchange chromatography", BIOTECHNOLOGY AND BIOENGINEERING, vol. 95, no. 5, 8 August 2006 (2006-08-08), Hoboken, USA, pages 775 - 787, XP093211712, ISSN: 0006-3592, DOI: 10.1002/bit.21080**
- **ANONYMOUS: "High-throughput Process Development with PreDictor (TM) Plates", 1 January 2009 (2009-01-01), XP093211716, Retrieved from the Internet <URL:www>**
- **ANONYMOUS: "Chromatography", 11 July 2018 (2018-07-11), XP093211722, Retrieved from the Internet <URL:www>**
- **ANONYMOUS: "Size-exclusion chromatography", WIKIPEDIA, 1 January 2025 (2025-01-01), XP093278711, Retrieved from the Internet <URL:https://en. Wikipedia.org/w/index.php?title=Size-exclusion_chromatography&oldid=453426386>**

- **ANONYMOUS: "Hydrophobic interaction and reversed phase chromatography", GE HEALTHCARE, 1 January 2025 (2025-01-01), XP093278714, Retrieved from the Internet <URL:http>**
- **LENHOFF ABRAHAM M.: "Protein adsorption and transport in polymer-functionalized ion-exchangers", JOURNAL OF CHROMATOGRAPHY A, vol. 1218, no. 49, 22 June 2011 (2011-06-22), AMSTERDAM, NL, pages 8748 - 8759, XP093284247, ISSN: 0021-9673, DOI: 10.1016/j.chroma.2011.06.061**
- **HAGEL L. ET AL: "Handbook of Process Chromatography", 1 January 2008 (2008-01-01), XP093284257, Retrieved from the Internet <URL:www>**
- **ANONYMOUS: "High-throughput Process Development with PreDictor (TM) Plates", 1 December 2009 (2009-12-01), XP093284320, Retrieved from the Internet <URL:www>**
- **HARINARAYAN C. ET AL: "An exclusion mechanism in ion exchange chromatography", BIOTECHNOLOGY AND BIOENGINEERING, vol. 95, no. 5, 8 August 2006 (2006-08-08), Hoboken, USA, pages 775 - 787, XP093284333, ISSN: 0006-3592, DOI: 10.1002/bit.21080**

Remarks:

The file contains technical information submitted after the application was filed and not included in this specification

**Description**

RELATED APPLICATIONS

FIELD OF THE INVENTION

The present invention provides methods for purifying a product from a composition comprising the product and at least one contaminant The invention is defined by the claims and any other aspects, configurations, instances or embodiments set forth herein not falling within the scope of the claims are for information only. Any references in the description to methods of treatment (or diagnosis) refer to the compounds, pharmaceutical compositions and medicaments of the present invention for use in a method of treatment of the human (or animal) body by therapy (or for diagnosis).

BACKGROUND OF THE INVENTION

Anion exchange (AEX) chromatography is widely used in a flow-through mode as a platform polishing step for monoclonal antibodies (MAbs). Certain MAbs that bind to AEX resin under standard flow through conditions can pose plant fit challenges. For early stage clinical development where the mass requirement is typically low, these non-platform MAbs have been purified by using AEX or mixed mode resin in a bind and elute mode. However, due to low dynamic binding capacity (DBC) of these resins, the late stage implementation would require columns that are approximately 1000 L in size or multiple cycles on a smaller column thus limiting the plant throughput.

MAb purification is typically performed using bind and elute chromatography (B/E) or flow-through (F/T) chromatography. Recently weak partitioning chromatography (Kelley, BD et al., 2008 Biotechnol Bioeng 101(3):553-566; US Patent Application Publication No. 2007/ 0060741) and overload chromatography (PCT/US2011/037977) have been introduced on AEX resins and cation exchange (CEX) resins respectively to enhance MAb purification. The general mechanism and limitations of each of these chromatography modes are highlighted below.

Bind and Elute Chromatography: Under B/E chromatography the product is usually loaded to maximize DBC to the chromatography material and then wash and elution conditions are identified such that maximum product purity is attained in the eluate. A limitation of B/E chromatography is the restriction of the load density to the actual resin DBC

Flow Through Chromatography: Using F/T chromatography, load conditions are identified where impurities strongly bind to the chromatography material while the product flows through. F/T chromatography allows high load density for standard MAbs but may not be implementable for non-platform MAbs or the solution conditions that enable F/T operation for these non-platform MAbs may be such that they are not implementable in existing manufacturing plants.

Weak Partitioning Chromatography: This mode of operation enhances the F/T mode by identifying solution conditions where there is weak binding of MAb to the resin (2 to 20 g/L). Under these conditions the impurities bind stronger than in the F/T mode and thus enhanced purification is obtained. However, load conditions are targeted to a have a low product partition coefficient ($K_p$) in the range of 0.1-20.

Overload Chromatography: In this mode of chromatography the product of interest is loaded beyond the dynamic binding capacity of the chromatography material for the product, thus referred to as overload. The mode of operation has been demonstrated to provide MAb purification with cation exchange (CEX) media and particularly with membranes. However, a limitation of this approach is that there could be low yields with resin as there is no elution phase.

The large-scale, cost-effective purification of a polypeptide to sufficient purity for use as a human therapeutic remains a formidable challenge.

BRIEF SUMMARY

The invention provides methods for purifying a polypeptide from a composition comprising the polypeptide and one or more contaminants, said method comprising a) loading the composition onto a chromatography material in an amount in excess of the dynamic binding capacity of the chromatography material for the polypeptide using a loading buffer, wherein the partition coefficient of the chromatography material for the polypeptide is greater than 100,

> b) eluting the polypeptide from the chromatography material under conditions wherein the one or more contaminants remain bound to the chromatography material using an elution buffer, wherein the elution buffer has a conductivity less than the conductivity of the loading buffer, and wherein the elution buffer has a conductivity less than 4.0 mS/cm,
> c) pooling fractions comprising the polypeptide in the chromatography efflluent from steps a) and b).

In some embodiments, the polypeptide is an antibody or immunoadhesin. In some embodiments, the antibody is a monoclonal antibody; for example, but antibody, humanized antibody, or human antibody.

In some embodiments, the antibody is an antigen binding fragment; for example but not limited to a Fab fragment, a Fab' fragment, a F(ab')2 fragment, a scFv, a di-scFv, a bi-scFv, a tandem (di, tri)-scFv, a Fv, a sdAb, a tri-functional antibody, a BiTE, a diabody and a triabody.

In some embodiments, the polypeptide is an enzyme, a hormone, a fusion protein, an Fc-containing protein, an immunoconjugate, a cytokine or an interleukin.

In some embodiments, the polypeptide is purified from a composition comprising one or more contaminants; for example, Chinese Hamster Ovary Protein (CHOP), a host cell protein (HCP), leached protein A, carboxypeptidase B, nucleic acid, DNA, product variants, aggregated protein, cell culture media component, gentamicin, polypeptide fragments, endotoxins, and viral contaminant.

In some embodiments of the invention, the chromatography material is selected from a mixed mode material, an anion exchange material, a hydrophobic interaction material, and an affinity material.

In some embodiments, the composition is loaded onto the chromatography material at about the dynamic binding capacities of the chromatography materials for the one or more contaminants.

In some embodiments, the methods provide OEC wherein the elution buffer has a pH less than the pH of the loading buffer. In other embodiments, the elution buffer has a pH greater than the pH of the loading buffer.

In some embodiments, the polypeptide of the methods is in an eluent from an affinity chromatography, a cation exchange chromatography, an anion exchange chromatography, a mixed mode chromatography and a hydrophobic interaction chromatography. In some embodiments, the polypeptide is in an eluent from a Protein A chromatography.

In some embodiments, the polypeptide of the methods is further purified; for example, by virus filtration, affinity chromatography, cation exchange chromatography, anion exchange chromatography, mixes mode chromatography, and/or hydrophobic interaction chromatography. In some embodiments, the polypeptide is further concentrated; for example by ultrafiltration, diafiltration or a combination of ultrafiltration and diafiltration. In some embodiments, the polypeptide of the methods is further combined with a pharmaceutically acceptable carrier.

BRIEF DESCRIPTION OF THE DRAWINGS

Figure 1 shows high throughput screening (HTS) results on Capto Adhere resin under batch binding conditions for MAb 3. Figure 1A shows contours of constant values of $K_p$ for MAb 3. Figure 1B shows actual binding capacity of MAb 3 in the supernatant at 80 g/L product challenge to the resin. Figure 1C shows actual binding capacity of the impurity (host cell protein) in the supernatant at 80 g/L product challenge to the resin. All the contour plots were generated using a response surface model derived from the raw data.

Figure 2 shows a chromatogram for an optimized OEC mode of operation. MAb 3 was used for this run with a load density of 180 g/L.

Figure 3 shows load optimization for an OEC mode using MAb 3.

Figure 4 shows a chromatogram with target load conditions for an OEC mode of operation. Similar load and elution conditions results in tailing and thus a 45% increase in pool volume. MAb 3 was used for this run with a load density of 180 g/L.

Figure 5 shows elution optimization for OEC mode using MAb 3.

Figure 6 shows impurity analysis in fractions and cumulative analysis of impurities.

Figure 7 shows MAb 3 CHOP breakthrough analysis of Capto Adhere resin under OEC mode at a loading density of 1000 g/L.

Figure 8 shows yield analysis across pilot scale runs over a wide range of load densities from 70 g/L to 180 g/L.

Figure 9 shows a comparison of weak partition chromatography mode of operation and overload and elute chromatography mode operation. The product was MAb 3 and the chromatography material was a Capto Adhere resin.

Figure 10 shows MAb 3 CHOP analysis on QMA resin under OEC mode of operation at a load density of 150 g/L.

Figure 11 shows MAb 4 CHOP analysis on Capto Adhere resin under OEC mode of operation at a load density of 150 g/L.

Figure 12 shows MAb 4 CHOP analysis on Capto MMC resin under OEC mode of operation at a load density of 150 g/L.

Figure 13 shows MAb 3 CHOP breakthrough analysis on Capto Adhere resin under OEC mode at a loading density of 200 g/L. MAb 3 protein A pool was loaded on Capto Adhere resin to 200 g/L (which is beyond its 50 g/L product binding capacity). CHOP breakthrough analysis showed that the CHOP did not breakthrough up to 200 g/L MAb processing.

DETAILED DESCRIPTION OF THE INVENTION

*I. Definitions*

The term "product" as described herein is the substance to be purified by OEC; for example, a polypeptide.

The term "polypeptide" or "protein" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art. The terms "polypeptide" and "protein" as used herein specifically encompass antibodies.

"Purified" polypeptide (*e.g.,* antibody or immunoadhesin) means that the polypeptide has been increased in purity, such that it exists in a form that is more pure than it exists in its natural environment and/or when initially synthesized and/or amplified under laboratory conditions. Purity is a relative term and does not necessarily mean absolute purity.

The term "epitope tagged" when used herein refers to a chimeric polypeptide comprising a polypeptide fused to a "tag polypeptide." The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

"Active" or "activity" for the purposes herein refers to form(s) of a polypeptide which retain a biological and/or an immunological activity of native or naturally-occurring polypeptide, wherein "biological" activity refers to a biological function (either inhibitory or stimulatory) caused by a native or naturally-occurring polypeptide other than the ability to induce the production of an antibody against an antigenic epitope possessed by a native or naturally-occurring polypeptide and an "immunological" activity refers to the ability to induce the production of an antibody against an antigenic epitope possessed by a native or naturally-occurring polypeptide.

The term "antagonist" is used in the broadest sense, and includes any molecule that partially or fully blocks, inhibits, or neutralizes a biological activity of a native polypeptide. In a similar manner, the term "agonist" is used in the broadest sense and includes any molecule that mimics a biological activity of a native polypeptide. Suitable agonist or antagonist molecules specifically include agonist or antagonist antibodies or antibody fragments, fragments or amino acid sequence variants of native polypeptides, etc. Methods for identifying agonists or antagonists of a polypeptide may comprise contacting a polypeptide with a candidate agonist or antagonist molecule and measuring a detectable change in one or more biological activities normally associated with the polypeptide.

"Complement dependent cytotoxicity" or "CDC" refers to the ability of a molecule to lyse a target in the presence of complement. The complement activation pathway is initiated by the binding of the first component of the complement system (C1q) to a molecule (*e.g.* polypeptide (*e.g.,* an antibody)) complexed with a cognate antigen. To assess complement activation, a CDC assay, *e.g.* as described in Gazzano-Santoro et al., J. Immunol. Methods 202:163 (1996), may be performed.

A polypeptide "which binds" an antigen of interest, *e.g.* a tumor-associated polypeptide antigen target, is one that binds the antigen with sufficient affinity such that the polypeptide is useful as a diagnostic and/or therapeutic agent in targeting a cell or tissue expressing the antigen, and does not significantly cross-react with other polypeptides. In such embodiments, the extent of binding of the polypeptide to a "non-target" polypeptide will be less than about 10% of the binding of the polypeptide to its particular target polypeptide as determined by fluorescence activated cell sorting (FACS) analysis or radioimmunoprecipitation (RIA).

With regard to the binding of a polypeptide to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target, for example, an excess of non-labeled target. In this case, specific binding is indicated if the binding of the labeled target to a probe is competitively inhibited by excess unlabeled target.

The term "antibody" herein is used in the broadest sense and specifically covers monoclonal antibodies, polyclonal antibodies, multispecific antibodies (*e.g.* bispecific antibodies) formed from at least two intact antibodies, and antibody fragments so long as they exhibit the desired biological activity. The term "immunoglobulin" (Ig) is used interchangeable with antibody herein.

Antibodies are naturally occurring immunoglobulin molecules which have varying structures, all based upon the immunoglobulin fold. For example, IgG antibodies have two "heavy" chains and two "light" chains that are disulphide-bonded to form a functional antibody. Each heavy and light chain itself comprises a "constant" (C) and a "variable" (V) region. The V regions determine the antigen binding specificity of the antibody, whilst the C regions provide structural support and function in non-antigen-specific interactions with immune effectors. The antigen binding specificity of an antibody or antigen-binding fragment of an antibody is the ability of an antibody to specifically bind to a particular antigen.

The antigen binding specificity of an antibody is determined by the structural characteristics of the V region. The variability is not evenly distributed across the 110-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (*see* Kabat et al., Sequences of Proteins of Immunological Interest, 5th Ed. Public Health Service, National Institutes of Health, Bethesda, Md. (1991)). The constant domains are not involved directly in binding an antibody to an antigen, but exhibit various effector functions, such as participation of the antibody in antibody dependent cellular cytotoxicity (ADCC).

Each V region typically comprises three complementarity determining regions ("CDRs", each of which contains a "hypervariable loop"), and four framework regions. An antibody binding site, the minimal structural unit required to bind with substantial affinity to a particular desired antigen, will therefore typically include the three CDRs, and at least three, preferably four, framework regions interspersed there between to hold and present the CDRs in the appropriate conformation. Classical four chain antibodies have antigen binding sites which are defined by $V_H$ and $V_L$ domains in cooperation. Certain antibodies, such as camel and shark antibodies, lack light chains and rely on binding sites formed by heavy chains only. Single domain engineered immunoglobulins can be prepared in which the binding sites are formed by heavy chains or light chains alone, in absence of cooperation between $V_H$ and $V_L$.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (*see* Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody that are responsible for antigen binding. The hypervariable region may comprise amino acid residues from a "complementarity determining region" or "CDR" (*e.g.,* around about residues 24-34 (L1), 50-56 (L2) and 89-97 (L3) in the $V_L$, and around about 31-35B (H1), 50-65 (H2) and 95-102 (H3) in the $V_H$ (Kabat et al., Sequences of Proteins of Immunological Interest, 5th Ed. Public Health Service, National Institutes of Health, Bethesda, Md. (1991)) and/or those residues from a "hypervariable loop" (*e.g.* residues 26-32 (L1), 50-52 (L2) and 91-96 (L3) in the $V_L$, and 26-32 (H1), 52A-55 (H2) and 96-101 (H3) in the $V_H$ (Chothia and Lesk J. Mol. Biol. 196:901-917 (1987)).

"Framework" or "FR" residues are those variable domain residues other than the hypervariable region residues as herein defined.

"Antibody fragments" comprise a portion of an intact antibody, preferably comprising the antigen binding region thereof. Examples of antibody fragments include Fab, Fab', F(ab')$_2$, and Fv fragments; diabodies; tandem diabodies (taDb), linear antibodies(*e.g.,* U.S. Patent No. 5,641,870, Example 2; Zapata et al., Protein Eng. 8(10):1057-1062 (1995)); one-armed antibodies, single variable domain antibodies, minibodies, single-chain antibody molecules; multispecific antibodies formed from antibody fragments (*e.g.,* including but not limited to, Db-Fc, taDb-Fc, taDb-CH3, (scFV)4-Fc, di-scFv, bi-scFv, or tandem (di,tri)-scFv); and Bi-specific T-cell engagers (BiTEs).

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')$_2$ fragment that has two antigen-binding sites and is still capable of cross-linking antigen.

"Fv" is the minimum antibody fragment that contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the $V_H$-$V_L$ dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')$_2$ antibody fragments originally were produced as pairs of Fab' fragments that have hinge cysteines between them. Other chemical couplings of antibody fragments are also known.

The "light chains" of antibodies (immunoglobulins) from any vertebrate species can be assigned to one of two clearly distinct types, called kappa ($\kappa$) and lambda ($\lambda$), based on the amino acid sequences of their constant domains.

Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), e.g., IgG1, IgG2, IgG3, IgG4, IgA, and IgA2. The heavy chain constant domains that correspond to the different classes of antibodies are called $\alpha$, $\delta$, $\epsilon$, $\gamma$, and $\mu$, respectively. The subunit structures and three-dimensional configurations of different classes of immunoglobulins are well known.

"Single-chain Fv" or "scFv" antibody fragments comprise the $V_H$ and $V_L$ domains of antibody, wherein these domains are present in a single polypeptide chain. In some embodiments, the Fv polypeptide further comprises a polypeptide linker between the $V_H$ and $V_L$ domains that enables the scFv to form the desired structure for antigen binding. For a review of scFv see Plückthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy chain variable domain ($V_H$) connected to a light chain variable domain ($V_L$) in the same polypeptide chain ($V_H$ - $V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

The term "multispecific antibody" is used in the broadest sense and specifically covers an antibody that has polyepitopic specificity. Such multispecific antibodies include, but are not limited to, an antibody comprising a heavy chain variable domain ($V_H$) and a light chain variable domain ($V_L$), where the $V_H V_L$ unit has polyepitopic specificity, antibodies having two or more $V_L$ and $V_H$ domains with each $V_H V_L$ unit binding to a different epitope, antibodies having two or more single variable domains with each single variable domain binding to a different epitope, full length antibodies, antibody fragments such as Fab, Fv, dsFv, scFv, diabodies, bispecific diabodies, triabodies, tri-functional antibodies, antibody fragments that have been linked covalently or non-covalently. "Polyepitopic specificity" refers to the ability to specifically bind to two or more different epitopes on the same or different target(s). "Monospecific" refers to the ability to bind only one epitope. According to one embodiment the multispecific antibody is an IgG antibody that binds to each epitope with an affinity of 5 $\mu$M to 0.001 pM, 3 $\mu$M to 0.001 pM, 1 $\mu$M to 0.001 pM, 0.5 $\mu$M to 0.001 pM, or 0.1 $\mu$M to 0.001 pM.

The expression "single domain antibodies" (sdAbs) or "single variable domain (SVD) antibodies" generally refers to antibodies in which a single variable domain (VH or VL) can confer antigen binding. In other words, the single variable domain does not need to interact with another variable domain in order to recognize the target antigen. Examples of single domain antibodies include those derived from camelids (lamas and camels) and cartilaginous fish (e.g., nurse sharks) and those derived from recombinant methods from humans and mouse antibodies (Nature (1989) 341:544-546; Dev Comp Immunol (2006) 30:43-56; Trend Biochem Sci (2001) 26:230-235; Trends Biotechnol (2003):21:484-490; WO 2005/035572; WO 03/035694; Febs Lett (1994) 339:285-290; WO00/29004; WO 02/051870).

The term "monoclonal antibody" as used herein refers to an antibody obtained from a population of substantially homogeneous antibodies, i.e., the individual antibodies comprising the population are identical and/or bind the same epitope, except for possible variants that may arise during production of the monoclonal antibody, such variants generally being present in minor amounts. In contrast to polyclonal antibody preparations that typically include different antibodies directed against different determinants (epitopes), each monoclonal antibody is directed against a single determinant on the antigen. In addition to their specificity, the monoclonal antibodies are advantageous in that they are uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the methods provided herein may be made by the hybridoma method first described by Kohler et al., Nature 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA 81:6851-6855 (1984)). Chimeric antibodies of interest herein include "primatized" antibodies comprising variable domain antigen-binding sequences derived from a non-human primate (e.g. Old World Monkey, such as baboon, rhesus or cynomolgus monkey) and human constant region sequences (US Pat No. 5,693,780).

"Humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence, except for FR substitution(s) as noted above. The humanized antibody optionally also will comprise at least a portion of an immunoglobulin constant region, typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

For the purposes herein, an "intact antibody" is one comprising heavy and light variable domains as well as an Fc region. The constant domains may be native sequence constant domains (e.g. human native sequence constant domains) or amino acid sequence variant thereof. Preferably, the intact antibody has one or more effector functions.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

A "naked antibody" is an antibody (as herein defined) that is not conjugated to a heterologous molecule, such as a cytotoxic moiety or radiolabel.

In some embodiments, antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity; Fc receptor binding; antibody-dependent cell-mediated cytotoxicity (ADCC); phagocytosis; down regulation of cell surface receptors.

"Antibody-dependent cell-mediated cytotoxicity" and "ADCC" refer to a cell-mediated reaction in which nonspecific cytotoxic cells that express Fc receptors (FcRs) (e.g. Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells in summarized is Table 3 on page 464 of Ravetch and Kinet, Annu. Rev. Immunol 9:457-92 (1991). To assess ADCC activity of a molecule of interest, an in vitro ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al., Proc. Natl. Acad. Sci. (USA) 95:652-656 (1998).

"Human effector cells" are leukocytes that express one or more FcRs and perform effector functions. In some embodiments, the cells express at least FcγRIII and carry out ADCC effector function. Examples of human leukocytes that mediate ADCC include peripheral blood mononuclear cells (PBMC), natural killer (NK) cells, monocytes, cytotoxic T cells and neutrophils; with PBMCs and NK cells being preferred.

The terms "Fc receptor" or "FcR" are used to describe a receptor that binds to the Fc region of an antibody. In some embodiments, the FcR is a native sequence human FcR. Moreover, a preferred FcR is one that binds an IgG antibody (a gamma receptor) and includes receptors of the FcγRI, FcγRII, and Fcγ RIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

The term "sequential" as used herein with regard to chromatography refers to having a first chromatography followed by a second chromatography. Additional steps may be included between the first chromatography and the second chromatography.

The term "continuous" as used herein with regard to chromatography refers to having a first chromatography material and a second chromatography material either directly connected or some other mechanism which allows for continuous flow between the two chromatography materials.

"Contaminants" refer to materials that are different from the desired polypeptide product. The contaminant includes, without limitation: host cell materials, such as CHOP; leached Protein A; nucleic acid; a variant, fragment, aggregate or derivative of the desired polypeptide; another polypeptide; endotoxin; viral contaminant; cell culture media component, etc. In some examples, the contaminant may be a host cell protein (HCP) from, for example but not limited to, a bacterial cell such as an *E. coli* cell, an insect cell, a prokaryotic cell, a eukaryotic cell, a yeast cell, a mammalian cell, an avian cell, a fungal cell.

The "dynamic binding capacity" of a chromatography material is the amount of product, e.g. polypeptide, the material will bind under actual flow conditions before significant breakthrough of unbound product occurs.

"Partition coefficient", $K_p$, as used herein, refers to the molar concentration of product, *e.g.* polypeptide, in the stationary phase divided by the molar concentration of the product in the mobile phase.

"Loading density" refers to the amount, e.g. grams, of composition put in contact with a volume of chromatography material, e.g. liters. In some examples, loading density is expressed in g/L.

Reference to "about" a value or parameter herein includes (and describes) variations that are directed to that value or parameter per se. For example, description referring to "about X" includes description of "X".

As used herein and in the appended claims, the singular forms "a," "or," and "the" include plural referents unless the context clearly dictates otherwise. It is understood that aspects and variations of the invention described herein include "consisting" and/or "consisting essentially of" aspects and variations.

## II. Methods of Purification

Provided herein are methods for purifying a product, which is a polypeptiue, from a composition comprising the product and at least one contaminant using overload and elute chromatography (OEC). OEC provides a mode of operation where benefits provided by different modes of chromatography are realized within a single chromatography mode. OEC can be implemented on multiple resins while providing enhanced impurity removal and significant manufacturing advantages, such as smaller columns, better plant fit and lower cost. The mode of operation with OEC can be broken down into three different components.

1. Overload - The composition is loaded onto the chromatography material such that the product, *e.g.* a polypeptide, is loaded onto the chromatography material at an amount exceeding the dynamic binding capacity (DBC) of the material for the product. In some embodiments, loading conditions, such as pH and conductivity, are determined where impurities strongly bind to the chromatography material. In some embodiments, the chromatography conditions are chosen such that, even if product breaks through after binding most, if not all, of the impurities do not. In some embodiments the composition is loaded at or near the DBC of the material for the one or more contaminants. The overload mode allows the chromatography material to be utilized beyond the typical DBC of the material for the product.

2. Pooling - The pooling of the product, *e.g.* a polypeptide, in the eluant starts at the product breakthrough. Since the load conditions are such that impurities continue to bind during the breakthrough phase, a clean product pool may be obtained in the eluant during the load phase of chromatography.

3. Elution - Upon completion of loading of the composition on the chromatography material, the product, *e.g.* polypeptide, is eluted from the chromatography material using elution conditions that are identified such that bound product is eluted while majority of the impurities remain bound to the chromatography material.

In some aspects, OEC increases chromatography material utilization significantly beyond the DBC of the material for the product thereby providing benefit compared to other chromatography methods. For example, 10-fold higher chromatography material utilization may result in significantly lower cost.

Unlike traditional bind and elute chromatography where loading of the chromatography material is optimized to maximize binding to the product, e.g. polypeptide, to the chromatography resin, with OEC load conditions may be optimized to maximize binding of contaminants to the chromatography material and not binding of the product to the chromatography material. In some aspects, the composition is loaded onto a chromatography material at an amount exceeding the dynamic binding capacity of the chromatography material for the product. In the process of loading the chromatography material, some of the product will break-through in the wash and some of the product will remain bound to the chromatography material. Upon completion of load, the remaining product bound to the chromatography material can be eluted from the chromatography material. In some embodiments of the above, the chromatography material is a chromatography column. In some embodiments of the above, the chromatography material is a chromatography membrane.

In some aspects of the invention, a composition is loaded onto a chromatography material at about the dynamic binding capacity of the chromatography material for one or more of the contaminants in the composition. In some embodiments, a composition is loaded onto a chromatography material at an amount exceeding the binding capacity of the chromatography material for the product. In some embodiments, a composition is loaded onto a chromatography material at about the dynamic binding capacity of the chromatography material for one or more of the contaminants and exceeding the binding capacity of the chromatography material for the product. In some embodiments, the composition is loaded onto the chromatography material at 20-times the DBC of the chromatography material for the product. In some embodiments, the composition is loaded onto the chromatography material at 100-times the DBC of the chromatography material for the product. In some embodiments, the composition is loaded onto a chromatography material at about the dynamic binding capacity of the chromatography material for all of the contaminants in the composition. In some embodiments, a composition is loaded onto a chromatography material at about the dynamic binding capacity of the chromatography material for all of the contaminants and exceeding the binding capacity of the chromatography material for the product. In some embodiments, a composition is loaded onto a chromatography material at less than the dynamic binding capacity of the chromatography material for all of the contaminants and exceeding the binding capacity of the chromatography material for the product. In some embodiments of the above, the chromatography material is in a chromatography column. In some embodiments, the chromatography column is an industrial scale chromatography column. In some embodiments of the above, the chromatography material is a chromatography membrane.

The dynamic binding capacity of a chromatography material for a product and for one or more contaminants can be estimated by determining the partition coefficient ($K_p$) for the product or contaminants as a function of pH and counterion concentration for a particular chromatography material. For example, the dynamic binding capacity of a chromatography material, *e.g.* a mixed mode resin, for a polypeptide may be determined. Actual binding capacities of a chromatography material for a product or contaminant at a specific combination of pH and counterion concentration can be determined by challenging the binding with an excess of the product and/or contaminant.

The OEC is performed where the $K_p$ of the product is greater than about 100.

The conditions for OEC of a particular composition comprising a product, such as a polypeptide, and contaminant can be determined by measuring the $K_p$ and dynamic binding capacity of particular chromatography material at different pH and counterion concentration. High throughput screening can be conducted to determine OEC conditions where binding of contaminants is high and where the product may be eluted without eluting most if not all of the contaminants. For example, the composition can be incubated with a chromatography material in buffer at various pH and counterion concentrations under high throughput system; for example, in wells of a multiple-well plate. After an incubation period, the supernatant is separated from the chromatography material and the amount of product or contaminant in the supernatant is determined. In some embodiments, low concentrations of composition are used to determine $K_p$. In some embodiments, high concentrations of the composition are used to determine dynamic binding capacities.

In addition to providing information regarding $K_p$ and dynamic binding capacity of a chromatography material for particular products and contaminants, high throughput screening provides guidance to load and elute conditions in terms of pH and counterion concentration. For example, in some embodiments, the load buffer is selected by high throughput screening for a pH and counterion concentration to maximize contaminant binding to the chromatography material but to also to maximize the amount of product, *e.g.* polypeptide, in the eluent while minimizing the amount of contaminant, *e.g.* host cell protein, in the eluent. In some embodiments, the composition is loaded onto a chromatography material at a pH and conductivity determined by high throughput screening wherein about all of the contaminants in the composition bind to the chromatography material. In some embodiments, the product is eluted from the chromatography material at a pH and conductivity determined by high throughput screening wherein about all of the product elutes from the chromatography material and about all of the contaminants remain bound to the chromatography material.

Set out herein are methods for identifying the operating conditions (e.g. by using high throughput screening techniques) that cause the chromatography material to bind a maximum amount of contaminants irrespective of the amount of product bound per mL of chromatography material. The screening step is used to identify the elution conditions such that the bound product is eluted from the chromatography material and impurities remain tightly bound to the chromatography material.

In some embodiments of any of the methods described herein, the chromatography material is a mixed mode material comprising functional groups capable of one of more of the following functionalities: anionic exchange, cation exchange, hydrogen bonding, and hydrophobic interactions. In some embodiments, the mixed mode material comprises functional groups capable of anionic exchange and hydrophobic interactions. The mixed mode material may contain N-benzyl-N-methyl ethanol amine, 4-mercapto-ethyl-pyridine, hexylamine, or phenylpropylamine as ligand or contain cross-linked polyallylamine. Examples of the mixed mode materials include Capto Adhere resin, QMA resin, Capto MMC resin, MEP HyperCel resin, HEA HyperCel resin, PPA HyperCel resin, or ChromaSorb membrane or Sartobind STIC. In some embodiments, the mixed mode material is Capto Adhere resin. In some embodiments of the above, the mixed mode material is a mixed mode chromatography column. In some embodiments of the above, the mixed mode material is a mixed mode membrane.

In some embodiments of any of the methods described herein, the chromatography material is an ion exchange chromatography material; for example, an anion exchange chromatography material or a cation exchange chromatography material. In some embodiments of any of the methods described herein, the chromatography material is an anion exchange material. In some embodiments, the anion exchange chromatography material is a solid phase that is positively charged and has free anions for exchange with anions in an aqueous solution passed over or through the solid phase. In some embodiments of any of the methods described herein, the anion exchange material may be a membrane, a monolith, or resin. In an embodiment, the anion exchange material may be a resin. In some embodiments, the anion exchange material may comprise a primary amine, a secondary amine, a tertiary amine or a quarternary ammonium ion functional group, a polyamine functional group, or a diethylaminoaethyl functional group. In some embodiments of the above, the anion exchange chromatography material is an anion exchange chromatography column. In some embodiments of the above, the anion exchange chromatography material is an anion exchange chromatography membrane.

In some embodiments of any of the methods described herein, the chromatography material is a cation exchange material. In some embodiments, the cation exchange material is a solid phase that is negatively charged and has free cations for exchange with cations in an aqueous solution passed over or through the solid phase. In some embodiments of any of the methods described herein, the cation exchange material may be a membrane, a monolith, or resin. In some embodiments, the cation exchange material may be a resin. The cation exchange material may comprise a carboxylic acid functional group or a sulfonic acid functional group such as, but not limited to, sulfonate, carboxylic, carboxymethyl sulfonic acid, sulfoisobutyl, sulfoethyl, carboxyl, sulphopropyl, sulphonyl, sulphoxyethyl, or orthophosphate. In some embodiments of the above, the cation exchange chromatography material is a cation exchange chromatography column. In some embodiments of the above, the cation exchange chromatography material is a cation exchange chromatography membrane. In some embodiments of the invention, the chromatography material is not a cation exchange chromatography material.

In some embodiments of any of the methods described herein, the ion exchange material may utilize a conventional chromatography material or a convective chromatography material. The conventional chromatography materials include, for example, perfusive materials (*e.g.,* poly (styrene-divinylbenzene) resin) and diffusive materials (*e.g.,* cross-linked agarose resin). In some embodiments, the poly (styrene-divinylbenzene) resin can be Poros resin. In some embodiments, the cross-linked agarose resin may be sulphopropyl-Sepharose Fast Flow ("SPSFF") resin. The convective chromatography material may be a membrane (*e.g.,* polyethersulfone) or monolith material (*e.g.* cross-linked polymer). The polyethersulfone membrane may be Mustang. The cross-linked polymer monolith material may be cross-linked poly(glycidyl methacrylate-co-ethylene dimethacrylate).

Examples of anion exchange materials are know in the art and include, but are not limited to Poros HQ 50, Poros PI 50, Poros D, Mustang Q, Q Sepharose FF, and DEAE Sepharose.

Examples of cation exchange materials are known in the art include, but are not limited to Mustang S, Sartobind S, SO3 Monolith, S Ceramic HyperD, Poros XS, Poros HS50, Poros HS20, SPSFF, SP-Sepharose XL (SPXL), CM Sepharose Fast Flow, Capto S, Fractogel Se HiCap, Fractogel SO3, or Fractogel COO. In some embodiments of any of the methods described herein, the cation exchange material is Poros HS50. In some embodiments, the Poros HS resin may be Poros HS 50 $\mu$m or Poros HS 20 $\mu$m particles.

In some aspects of the invention, the chromatography material is a hydrophobic interaction chromatography material. Hydrophobic interaction chromatography (HIC) is a liquid chromatography technique that separates biomolecules according to hydrophobicity. Examples of HIC chromatography materials include, but are not limited to, Toyopearl hexyl 650, Toyopear butyl 650, Toyopearl phenyl 650, Toyopearl ether 650, Source, Resource, Sepharose Hi-Trap, Octyl sepharose, Phenyl sepharose. In some embodiments of the above, the HIC chromatography material is a HIC chromatography column. In some embodiments of the above, the HIC chromatography material is a HIC chromatography membrane.

In some aspects of the invention, the chromatography material is a hydroxyapatite (HAP) chromatography material. Examples of hydroxyapatite chromatography material include but are limited to HA Ultrogel, and CHT hydroxyapatite. In some embodiments of the above, the HAP chromatography material is a HAP chromatography column. In some embodiments of the above, the HAP chromatography material is a HAP chromatography membrane.

In some aspects of the invention, the chromatography material is an affinity chromatography material. Examples of affinity chromatography materials include, but are not limited to chromatography materials derivatized with protein A or protein G. Examples of affinity chromatography material include, but are not limited to, Prosep-VA, Prosep-VA Ultra Plus, Protein A sepharose fast flow, Tyopearl Protein A, MAbSelect, MAbSelect SuRe and MAbSelect SuRe LX. In some embodiments of the above, the affinity chromatography material is an affinity chromatography column. In some embodiments of the above, the affinity chromatography material is an affinity chromatography membrane.

Loading of the composition on the chromatography material may be optimized for separation of the product from contaminants by OEC. In some embodiments, the product is a polypeptide. In some embodiments, loading on the composition onto the chromatography material is optimized for binding of the contaminants to the chromatography material. For example, the composition may be loaded onto the chromatography material, *e.g.* a chromatography column, in a load buffer at a number of different pH while the conductivity of the load buffer is constant. Alternatively, the composition may be loaded onto the chromatography material in a load buffer at a number of different conductivities while the pH of the load buffer is constant. Upon completion of loading the composition on the chromatography material and elution of the product from the chromatography material into a pool fraction, the amount of contaminant in the pool fraction provides information regarding the separation of the product from the contaminants for a given pH or conductivity. In some embodiments of any of the methods described herein, the composition is loaded onto a chromatography material at a loading density of the polypeptide of greater than about any of 30 g/L, 40 g/L, 50 g/L, 60 g/L, 70 g/L, 80 g/L, 90 g/L, 100 g/L, 110 g/L, 120 g/L, 130 g/L, 140 g/L, 150 g/L, 160 g/L, 170 g/L, 180 g/L, 190 g/L, 200 g/L, 300 g/L, 400 g/L, 500 g/L, 550 g/L, 600 g/L, 650 g/L, 700 g/L, 800 g/L, 900 g/L, 1000 g/L, 2000 g/L or 5000 g/L of the chromatography material. In some embodiments, the composition is loaded onto a chromatography material at a loading density of the polypeptide of about any of about any of 30 g/L, 40 g/L, 50 g/L, 60 g/L, 70 g/L, 80 g/L, 90 g/L, 100 g/L, 110 g/L, 120 g/L, 130 g/L, 140 g/L, 150 g/L, 160 g/L, 170 g/L, 180 g/L, 190 g/L, 200 g/L, 300 g/L, 400 g/L, 500 g/L, 550 g/L, 600 g/L, 650 g/L, 700 g/L, 800 g/L, 900 g/L, 1000 g/L or 2000 g/L of the chromatography material. The composition may be loaded onto a chromatography material at a loading density of the polypeptide of between about any of 30 g/L and 2000 g/L, 30 g/L and 1000 g/L, 30 g/L and 200 g/L, 30 g/L and 180 g/L, 50 g/L and 2000 g/L, 50 g/L and 1000 g/L, 50 g/L and 200 g/L, 50 g/L and 180 g/L, 150 g/L and 2000 g/L, 150 g/L and 1500 g/L, 150 g/L and 1000 g/L, 200 g/L and 1000 g/L, 200 g/L and 1500 g/L, 300 g/L and 1500 g/L, 400 g/L and 1000 g/L, or 500 g/L and 1000 g/L of chromatography material.

In some embodiments of any of the methods described herein, the composition is loaded onto a mixed mode chromatography material at a loading density of the polypeptide of greater than about any of 30 g/L, 40 g/L, 50 g/L, 60 g/L, 70 g/L, 80 g/L, 90 g/L, 100 g/L, 110 g/L, 120 g/L, 130 g/L, 140 g/L, 150 g/L, 160 g/L, 170 g/L, 180 g/L, 190 g/L, 200 g/L, 300 g/L, 400 g/L, 500 g/L, 550 g/L, 600 g/L, 650 g/L, 700 g/L, 800 g/L, 900 g/L, 1000 g/L, 2000 g/L or 5000 g/L of the mixed mode chromatography material. In some embodiments, the composition is loaded onto a mixed mode chromatography material at a loading density of the polypeptide of about any of about any of 30 g/L, 40 g/L, 50 g/L, 60 g/L, 70 g/L, 80 g/L, 90 g/L, 100 g/L, 110 g/L, 120 g/L, 130 g/L, 140 g/L, 150 g/L, 160 g/L, 170 g/L, 180 g/L, 190 g/L, 200 g/L, 300 g/L, 400 g/L, 500 g/L, 550 g/L, 600 g/L, 650 g/L, 700 g/L, 800 g/L, 900 g/L, 1000 g/L or 2000 g/L of the mixed mode chromatography material. The composition may be loaded onto a mixed mode chromatography material at a loading density of the polypeptide of between about any of 30 g/L and 2000 g/L, 30 g/L and 1000 g/L, 30 g/L and 200 g/L, 30 g/L and 180 g/L, 50 g/L and 2000 g/L, 50 g/L and 1000 g/L, 50 g/L and 200 g/L, 50 g/L and 180 g/L, 150 g/L and 2000 g/L, 150 g/L and 1500 g/L, 150 g/L and 1000 g/L, 200 g/L and 1000 g/L, 200 g/L and 1500 g/L, 300 g/L and 1500 g/L, 400 g/L and 1000 g/L, or 500 g/L and 1000 g/L of mixed mode chromatography material. In some embodiments, the polypeptide is loaded on a chromatography material at a density of 70 g/L to 180 g/L. In some embodiments of the invention, the mixed mode chromatography is a Capto Adhere resin. In some embodiments, the polypeptide is loaded on a Capto Adhere chromatography material at a density of 70 g/L to 180 g/L. In further embodiments of the above embodiments, the polypeptide is an antibody of a fragment thereof.

In some embodiments of any of the methods described herein, the composition is loaded onto an anion exchange chromatography material at a loading density of the polypeptide of greater than about any of 30 g/L, 40 g/L, 50 g/L, 60 g/L, 70 g/L, 80 g/L, 90 g/L, 100 g/L, 110 g/L, 120 g/L, 130 g/L, 140 g/L, 150 g/L, 160 g/L, 170 g/L, 180 g/L, 190 g/L, 200 g/L, 300 g/L, 400 g/L, 500 g/L, 550 g/L, 600 g/L, 650 g/L, 700 g/L, 800 g/L, 900 g/L, 1000 g/L, 2000 g/L or 5000 g/L of the anion exchange chromatography material. In some embodiments, the composition is loaded onto an anion exchange chromatography material at a loading density of the polypeptide of about any of about any of 30 g/L, 40 g/L, 50 g/L, 60 g/L, 70 g/L, 80 g/L, 90 g/L, 100 g/L, 110 g/L, 120 g/L, 130 g/L, 140 g/L, 150 g/L, 160 g/L, 170 g/L, 180 g/L, 190 g/L, 200 g/L, 300 g/L, 400 g/L, 500 g/L, 550 g/L, 600 g/L, 650 g/L, 700 g/L, 800 g/L, 900 g/L, 1000 g/L or 2000 g/L of the an anion exchange chromatography material. The composition may be loaded onto an anion exchange chromatography material at a loading density of the polypeptide of between about any of 30 g/L and 2000 g/L, 30 g/L and 1000 g/L, 30 g/L and 200 g/L, 30 g/L and 180 g/L, 50 g/L and 2000 g/L, 50 g/L and 1000 g/L, 50 g/L and 200 g/L, 50 g/L and 180 g/L, 150 g/L and 2000 g/L, 150 g/L and 1500 g/L, 150 g/L and 1000 g/L, 200 g/L and 1000 g/L, 200 g/L and 1500 g/L, 300 g/L and 1500 g/L, 400 g/L and 1000 g/L, or 500 g/L and 1000 g/L of the anion exchange chromatography material.

In some embodiments of any of the methods described herein, the composition is loaded onto a cation exchange chromatography material at a loading density of the polypeptide of greater than about any of 30 g/L, 40 g/L, 50 g/L, 60 g/L, 70 g/L, 80 g/L, 90 g/L, 100 g/L, 110 g/L, 120 g/L, 130 g/L, 140 g/L, 150 g/L, 160 g/L, 170 g/L, 180 g/L, 190 g/L, 200 g/L, 300 g/L, 400 g/L, 500 g/L, 550 g/L, 600 g/L, 650 g/L, 700 g/L, 800 g/L, 900 g/L, 1000 g/L, 2000 g/L or 5000 g/L of the cation exchange chromatography material. In some embodiments, the composition is loaded onto a cation exchange chromatography material at a loading density of about any of about any of 30 g/L, 40 g/L, 50 g/L, 60 g/L, 70 g/L, 80 g/L, 90 g/L, 100 g/L, 110 g/L, 120 g/L, 130 g/L, 140 g/L, 150 g/L, 160 g/L, 170 g/L, 180 g/L, 190 g/L, 200 g/L, 300 g/L, 400 g/L, 500 g/L, 550 g/L, 600 g/L, 650 g/L, 700 g/L, 800 g/L, 900 g/L, 1000 g/L or 2000 g/L of the cation exchange chromatography material. The composition may be loaded onto a cation exchange chromatography material at a loading density of the polypeptide of between about any of 30 g/L and 2000 g/L, 30 g/L and 1000 g/L, 30 g/L and 200 g/L, 30 g/L and 180 g/L, 50 g/L and 2000 g/L, 50 g/L and 1000 g/L, 50 g/L and 200 g/L, 50 g/L and 180 g/L, 150 g/L and 2000 g/L, 150 g/L and 1500 g/L, 150 g/L and 1000 g/L, 200 g/L and 1000 g/L, 200 g/L and 1500 g/L, 300 g/L and 1500 g/L, 400 g/L and 1000 g/L, or 500 g/L and 1000 g/L of the cation exchange chromatography material.

The methods described above may further comprise the step of loading onto a Protein A affinity chromatography material. In some embodiments the polypeptide product is an antibody or fragment thereof that is first purified by Protein A affinity chromatography prior to OEC. The step of loading onto a Protein A affinity chromatography material is generally, but not necessarily, performed before the other chromatography step(s). In some embodiments, the step of loading onto a Protein A affinity chromatography material may be combined with the sequential steps of overloaded exchange and elute chromatography. In some embodiments, the sequential steps are continuous. In some embodiments, the continuous purification utilizes the same flow rate, conductivity, and/or pH.

Elution of the product such as a polypeptide from the chromatography material under OEC mode may be optimized for yield of product with minimal contaminants and at minimal pool volume. For example, the composition may be loaded onto the chromatography material, *e.g.* a chromatography column, in a load buffer. Upon completion of load, the product is eluted with buffers at a number of different pH while the conductivity of the elution buffer is constant. Alternatively, the product may be eluted from the chromatography material in an elution buffer at a number of different conductivities while the pH of the elution buffer is constant. Upon completion of elution of the product from the chromatography material, the amount of contaminant in the pool fraction provides information regarding the separation of the product from the contaminants for a given pH or conductivity. Elution of the product in a high number of fractions (e.g. eight column volumes) indicates "tailing" of the elution profile. In some embodiments of the invention, tailing of the elution is minimized.

Various buffers which can be employed depending, for example, on the desired pH of the buffer, the desired conductivity of the buffer, the characteristics of the protein of interest, and the purification method. In some embodiments of any of the methods described herein, the methods comprise using a buffer. The buffer can be a loading buffer, an equilibration buffer, or a wash buffer. In some embodiments, one or more of the loading buffer, the equilibration buffer, and/or the wash buffer are the same. In some embodiments, the loading buffer, the equilibration buffer, and/or the wash buffer are different. In some embodiments of any of the methods described herein, the buffer comprises a salt. The loading buffer may comprise sodium chloride, sodium acetate, or a mixture thereof. In some embodiments, the loading buffer is a sodium chloride buffer. In some embodiments, the loading buffer is a sodium acetate buffer.

Load, as used herein, is the composition loaded onto a chromatography material. Loading buffer is the buffer used to load the composition comprising the product of interest onto a chromatography material. The chromatography material may be equilibrated with an equilibration buffer prior to loading the composition which is to be purified. In some examples, the wash buffer is used after loading the composition onto a chromatography material and before elution of the polypeptide of interest from the solid phase. However, some of the product of interest, *e.g.* a polypeptide, may be removed from the chromatography material by the wash buffer (*e.g.* similar to a flow-through mode).

Elution, as used herein, is the removal of the product, *e.g.* polypeptide, from the chromatography material. Elution buffer is the buffer used to elute the polypeptide or other product of interest from a chromatography material. In many cases, an elution buffer has a different physical characteristic than the load buffer. For example, the elution buffer may have a different conductivity than load buffer or a different pH than the load buffer. In some embodiments, the elution buffer has a lower conductivity than the load buffer. In some embodiments, the elution buffer has a higher conductivity than the load buffer. In some embodiments, the elution buffer has a lower pH than the load buffer. In some embodiments, the elution buffer has a higher pH than the load buffer. In some embodiments the elution buffer has a different conductivity and a different pH than the load buffer. The elution buffer can have any combination of higher or lower conductivity and higher or lower pH.

Conductivity refers to the ability of an aqueous solution to conduct an electric current between two electrodes. In solution, the current flows by ion transport. Therefore, with an increasing amount of ions present in the aqueous solution, the solution will have a higher conductivity. The basic unit of measure for conductivity is the Siemen (or mho), mho (mS/cm), and can be measured using a conductivity meter, such as various models of Orion conductivity meters. Since electrolytic conductivity is the capacity of ions in a solution to carry electrical current, the conductivity of a solution may be altered by changing the concentration of ions therein. For example, the concentration of a buffering agent and/or the concentration of a salt (e.g. sodium chloride, sodium acetate, or potassium chloride) in the solution may be altered in order to achieve the desired conductivity. Preferably, the salt concentration of the various buffers is modified to achieve the desired conductivity.

In some embodiments of any of the methods described herein, the load buffer has a conductivity of greater than about any of 4.0 mS/cm, 4.5 mS/cm, 5.0 mS/cm, 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, or 10 mS/cm. The conductivity may be between about any of 4 mS/cm and 17 mS/cm, 4 mS/cm and 10 mS/cm, 4 mS/cm and 7 mS/cm, 5 mS/cm and 17 mS/cm, 5 mS/cm and 10 mS/cm, or 5 mS/cm and 7 mS/cm.

In some embodiments, the conductivity is about any of 4 mS/cm, 4.5 mS/cm, 5.0 mS/cm, 5.5 mS/cm, 6.0 mS/cm, 6.5 mS/cm, 7.0 mS/cm, 7.5 mS/cm, 8.0 mS/cm, 8.5 mS/cm, 9.0 mS/cm, 9.5 mS/cm, or 10 mS/cm. In one aspect, the conductivity is the conductivity of the loading buffer, the equilibration buffer, and/or the wash buffer. In some embodiments, the conductivity of one or more of the loading buffer, the equilibration buffer, and the wash buffer are the same. In some embodiments, the conductivity of the loading buffer is different from the conductivity of the wash buffer and/or equilibration buffer. In some embodiments, the composition is loaded on a mixed mode chromatography material in a buffer with a conductivity of about 5.5 mS/cm. In some embodiments, the polypeptide is an antibody or fragment thereof.

The elution buffer has a conductivity less than the conductivity The elution buffer has a conductivity of less than In some embodiments, the conductivity of the elution buffer is about any of 0.0 mS/cm, 0.5 mS/cm, 1.0 mS/cm, 1.5 mS/cm, 2.0 mS/cm, 2.5 mS/cm, 3.0 mS/cm, 3.5 mS/cm, In some embodiments, the elution buffers described above are used in mixed mode OEC, anion exchange OEC, cation exchange OEC, affinity OEC or HIC OEC.

In some aspects of any of the above embodiments, the conductivity of the elution buffer changed from the load and/or wash buffer by step gradient or by linear gradient.

In some embodiments, the load buffer has a conductivity of about 5.5 mS/cm and the elution buffer has a conductivity of about 3 mS/cm. In some embodiments, the load buffer has a conductivity of about 5.5 mS/cm and the elution buffer has a conductivity of about 2 mS/cm. In some embodiments, the load buffer has a conductivity of about 5.5 mS/cm and the elution buffer has a conductivity of about 1 mS/cm. In further embodiments of the above embodiments, the chromatography material is a Capto Adhere resin. In further embodiments of the above embodiments, the polypeptide is an antibody of fragment thereof.

In some aspects of any of the above embodiments, the conductivity of the elution buffer changed from the load and/or wash buffer by step gradient or by linear gradient. In some embodiments, the composition comprising a polypeptide is loaded onto a Capto Adhere chromatography at about 5.5 mS/cm and the polypeptide of interest is eluted from a Capto Adhere Chromatography by a linear conductivity gradient from about 5.5 mS/cm to about 1 mS/cm over about 5 column volumes (CV). In some embodiments, the composition comprising a polypeptide is loaded onto a Capto Adhere chromatography at about 5.5 mS/cm and the polypeptide of interest is eluted from a Capto Adhere Chromatography by a linear conductivity gradient from about 5.5 mS/cm to about 1mS/cm over 10.0 CV. In some embodiments, the composition comprising a polypeptide is loaded onto a Capto Adhere chromatography at about 10 mS/cm and the polypeptide of interest is eluted from a Capto Adhere chromatography by a linear conductivity gradient from about 10.0 mS/cm to about 1mS/cm over about 5 CV. In some embodiments, the composition comprising a polypeptide is loaded onto a Capto Adhere chromatography at about 10 mS/cm and the polypeptide of interest is eluted from a Capto Adhere chromatography by a linear conductivity gradient from about 10.0 mS/cm to 1 mS/cm over about 10 CV. In some embodiments, the composition comprising a polypeptide is loaded onto a Capto Adhere chromatography at about 10 mS/cm and the polypeptide of interest is eluted from a Capto Adhere chromatography by a linear conductivity gradient from about 10.0 mS/cm to abut 1mS/cm over about 15 CV.

In some aspects of any of the above embodiments, the conductivity of the elution buffer changed from the load and/or wash buffer by step gradient or by linear gradient. In some embodiments, the composition comprising a polypeptide is loaded onto a chromatography material at about 5.5mS/cm and the polypeptide of interest is eluted from a Chromatography material by a linear conductivity gradient between 5.5 mS/cm to 1mS/cm over 5 Column Volumes, 5.5 mS/cm to 1mS/cm over 10.0 Column Volumes. In some embodiments, the composition comprising a polypeptide is loaded onto a chromatography material at about 10 mS/cm and the polypeptide of interest is eluted from a Chromatography material by a linear conductivity gradient between 10.0 mS/cm to 1mS/cm over 5 Column Volumes, 10.0 mS/cm to 1 mS/cm over 10 Column Volumes, 10.0 mS/cm to 1mS/cm over 15 Column Volumes.

In some embodiments of any of the methods described herein, the load buffer has a pH of less than about any of 10, 9, 8, 7, 6, or 5. In some embodiments of any of the methods described herein, the load buffer has a pH of greater than about any of 4, 5, 6, 7, 8, or 9. The load buffer may have a pH of between about any of 4 and 9, 4 and 8, 4 and 7, 5 and 9, 5 and 8, 5 and 7, 5 and 6. In some embodiments, the pH of the load buffer is about any of 4, 4.5, 5, 5.5, 6, 6.5, 7, 7.5, or 8. The pH can be the pH of the loading buffer, the equilibration buffer, or the wash buffer. In some embodiments, the pH of one or more of the loading buffer, the equilibration buffer, and/or the wash buffer are the same. In some embodiments, the pH of the loading buffer is different from the pH of the equilibration buffer and/or the wash buffer.

In some embodiments, the elution buffer has a pH less than the pH of the load buffer. In some embodiments of any of the methods described herein, the elution buffer has a pH of less than about any of 8, 7, 6, 5, 4, 3 or 2. The pH of the elution buffer may be between about any of 4 and 9, 4 and 8, 4 and 7, 4 and 6, 4 and 5, 5 and 9, 5 and 8, 5 and 7, 5 and 6, 6 and 9, 6 and 8, 6 and 7. In some embodiments, the pH of the elution buffer is about any of 4.0, 4.5, 5.0. 5.5, 6.0, 6.5, 7/0, 7.5, 8.0, 8.5 or 9.0.

In some embodiments, the elution buffer has a pH greater than the pH of the load buffer. In some embodiments of any of the methods described herein, the elution buffer has a pH of greater than about any of 5, 6, 7, 8, or 9. The pH of the elution buffer may be between about any of 4 and 9, 5 and 9, 6 and 9, 7 and 9, 8 and 9, 4 and 8, 5 and 8, 6 and 8, 7 and 8, 4 and 7, 5 and 7, and 6 and 7. In some embodiments, the pH of the elution buffer is about any of 4.0, 4.5, 5.0. 5.5, 6.0, 6.5, 7/0, 7.5, 8.0, 8.5 or 9.0

In some aspects of any of the above embodiments, the pH of the elution buffer changed from the load and/or wash buffer by step gradient or by linear gradient.

In some embodiments of any of the methods described herein, the flow rate is less than about any of 50 CV/hr, 40 CV/hr, or 30 CV/hr. The flow rate may be between about any of 5 CV/hr and 50 CV/hr, 10 CV/hr and 40 CV/hr, or 18 CV/hr and 36 CV/hr. In some embodiments, the flow rate is about any of 9 CV/hr, 18 CV/hr, 25 CV/hr, 30 CV/hr, 36 CV/hr, or 40 CV/hr. In some embodiments of any of the methods described herein, the flow rate is less than about any of 100 cm/hr, 75 cm/hr, or 50 cm/hr. The flow rate may be between about any of 25 cm/hr and 150 cm/hr, 25 cm/hr and 100 cm/hr, 50 cm/hr and 100 cm/hr, or 65 cm/hr and 85 cm/hr.

Bed height is the height of chromatography material used. In some embodiments of any of the method described herein, the bed height is greater than about any of 3 cm, 10 cm, or 15 cm. The bed height may be between about any of 3 cm and 35 cm, 5 cm and 15 cm, 3 cm and 10 cm, or 5 cm and 8 cm. In some embodiments, the bed height is about any of 3 cm, 5 cm, 10 cm, or 15 cm. In some embodiments, bed height is determined based on the amount of polypeptide or contaminants in the load.

In some embodiments, the chromatography is in a column of vessel with a volume of greater than about 1 mL, 2 mL, 3 mL, 4 mL, 5 mL, 6 mL, 7 mL, 8 mL, 9 mL, 10 mL, 15 mL, 20 mL, 25 mL, 30 mL, 40 mL, 50 mL, 75 mL, 100 mL, 200 mL, 300 mL, 400 mL, 500 mL, 600 mL, 700 mL, 800 mL, 900 mL, 1 L, 2 L, 3 L, 4 L, 5 L, 6 L, 7 L, 8 L, 9 L, 10 L, 25 L, 50 L, 100 L, 200 L, 300 L, 400 L, 500 L, 600 L, 700 L, 800 L, 900 L or 100 L.

In some embodiments of the invention, fractions are collected from the chromatography. In some embodiments, fractions collected are greater than about 0.01 CV, 0.02 CV, 0.03 CV, 0.04 CV, 0.05 CV, 0.06 CV, 0.07 CV, 0.08 CV, 0.09 CV, 0.1 CV, 0.2 CV, 0.3 CV, 0.4 CV, 0.5 CV, 0.6 CV, 0.7 CV, 0.8 CV, 0.9 CV, 1.0 CV, 2.0 CV, 3.0 CV, 4.0 CV, 5.0 CV, 6.0 CV, 7.0 CV, 8.0 CV, 9.0 CV, or 10.0 CV. In some embodiments, fractions containing the product, e.g. polypeptide, are pooled. In some embodiments, fractions containing the polypeptide from the load fractions and from the elution fractions are pooled. The amount of polypeptide in a fraction can be determined by one skilled in the art; for example, the amount of polypeptide in a fraction can be determined by UV spectroscopy. In some embodiments, fractions containing detectable polypeptide fragment are pooled.

In some embodiments of any of the methods described herein, the at least one contaminant is any one or more of host cell materials, such as CHOP; leached Protein A; nucleic acid; a variant, fragment, aggregate or derivative of the desired polypeptide; another polypeptide; endotoxin; viral contaminant; cell culture media component, carboxypeptidase B, gentamicin, etc. In some examples, the contaminant may be a host cell protein (HCP) from, for example but not limited to, a bacterial cell such as an E. coli cell, an insect cell, a prokaryotic cell, a eukaryotic cell, a yeast cell, a mammalian cell, an avian cell, a fungal cell.

Host cell proteins (HCP) are proteins from the cells in which the polypeptide was produced. For example, CHOP are proteins from host cells, *i.e.,* Chinese Hamster Ovary Proteins. The amount of CHOP may be measured by enzyme-linked immunosorbent assay ("ELISA") or Meso Scale Discovery ("MSO"). In some embodiments of any of the methods described herein, the amount of HCP (*e.g.* CHOP) is reduced by greater than about any of 10 %, 20 %, 30 %, 40 %, 50 %, 60 %, 70 %, 80 %, 90 %, or 95 %. The amount of HCP may be reduced by between about any of 10 % and 99 %, 30% and 95%, 30 % and 99 %, 50% and 95%, 50 % and 99 %, 75 % and 99 %, or 85 % and 99 %. In some embodiments, the amount of HCP is reduced by about any of 10 %, 20 %, 30 %, 40 %, 50 %, 60 %, 70 %, 80 %, 85 %, 90 %, 95 %, or 98 %. In some embodiments, the reduction is determined by comparing the amount of HCP in the composition recovered from a purification step(s) to the amount of HCP in the composition before the purification step(s).

Aggregated polypeptide can be high molecular weight (HMW) protein. In some embodiments, the aggregated polypeptide is multimers of the polypeptide of interest. The HMW protein may be a dimer, up to 8x monomer, or larger of the polypeptide of interest. Methods of measuring aggregated protein (e.g., HMW protein) are known in the art and described in the examples section. In some embodiments of any of the methods described herein, the amount of aggregated protein is reduced by greater than about any of 5%, 10 %, 20 %, 30 %, 40 %, 50 %, 60 %, 70 %, 80 %, 90 %, or 95 %. The amount of aggregated protein may be reduced by between about any of 10 % and 99 %, 30% and 95%, 30 % and 99 %, 50% and 95%, 50 % and 99 %, 75 % and 99 %, or 85 % and 99 %. The amount of aggregated protein may be reduced by about any of 5 %, 10 %, 20 %, 30 %, 40 %, 50 %, 60 %, 70 %, 80 %, 90 %, or 95 %. In some embodiments, the reduction is determined by comparing the amount of aggregated protein (*e.g.,* HMW protein) in the composition recovered from a purification step(s) to the amount of aggregated protein (*e.g.,* HMW protein) in the composition before the purification step(s).

Fragment polypeptide can be low molecular weight (LMW) protein. In some embodiments, the fragmented polypeptide is a fragment of the polypeptide of interest. Examples of LMW protein include, but not limited to, a Fab (Fragment antigen binding), Fc (fragment, crystallizable) regions or combination of both or any random fragmented part of an antibody of interest. Methods of measuring fragmented protein (e.g., LMW protein) are known in the art and described in the examples section. In some embodiments of any of the methods described herein, the amount of LMW protein is reduced by greater than about any of 5%, 10 %, 20 %, 30 %, 40 %, 50 %, 60 %, 70 %, 80 %, 90 %, or 95 %. The amount of LMW protein may be reduced by between about any of 10 % and 99 %, 30% and 95%, 30 % and 99 %, 50% and 95%, 50 % and 99 %, 75 % and 99 %, or 85 % and 99 %. The amount of LMW protein may be reduced by about any of 5 %, 10 %, 20 %, 30 %, 40 %, 50 %, 60 %, 70 %, 80 %, 90 %, or 95 %. In some embodiments, the reduction is determined by comparing the amount of fragmented protein (*e.g.,* LMW protein) in the composition recovered from a purification step(s) to the amount of fragmented protein (*e.g.,* LMW protein) in the composition before the purification step(s).

Leached Protein A is Protein A detached or washed from a solid phase to which it is bound. For example, leached Protein A can be leached from Protein A chromatography column. The amount of Protein A may be measured, for example, by ELISA. In some embodiments of any of the methods described herein, the amount of leached Protein A is reduced by greater than about any of 10 %, 20 %, 30 %, 40 %, 50 %, 60 %, 70 %, 80 %, or 90 %. The amount of leached Protein A may be reduced by between about any of 10 % and 99 %, 30% and 95%, 30 % and 99 %, 50% and 95%, 50 % and 99 %, 75 % and 99 %, or 85 % and 99 %. In some embodiments, the amount of leached Protein A is reduced by about any of 10 %, 20 %, 30 %, 40 %, 50 %, 60 %, 70 %, 80 %, 90 %, or 95 %. In some embodiments, the reduction is determined by comparing the amount of leached Protein A in the composition recovered from a purification step(s) to the amount of leached Protein A in the composition before the purification step(s).

Methods of measuring DNA such as host cell DNA are known in the art and described in the examples section. In some embodiments of any of the methods described herein, the amount of DNA is reduced by greater than about any of 10 %, 20 %, 30 %, 40 %, 50 %, 60 %, 70 %, 80 %, or 90 %. The amount of DNA may be reduced by between about any of 10 % and 99 %, 30% and 95%, 30 % and 99 %, 50% and 95%, 50 % and 99 %, 75 % and 99 %, or 85 % and 99 %. The amount of DNA may be reduced by about any of 10 %, 20 %, 30 %, 40 %, 50 %, 60 %, 70 %, 80 %, 90 %, 95 %, or 99 %. In some embodiments, the reduction is determined by comparing the amount of DNA in the composition recovered from a purification step(s) to the amount of DNA in the composition before the purification step(s).

Cell culture media component refers to a component present in a cell culture media. A cell culture media may be a cell culture media at the time of harvesting cells. In some embodiments, the cell culture media component is gentamicin. The amount of gentamicin may be measured by ELISA. In some embodiments of any of the methods described herein, the amount of cell culture media component is reduced by greater than about any of 10 %, 20 %, 30 %, 40 %, 50 %, 60 %, 70 %, 80 %, or 90 %. The amount of cell culture media component may be reduced by between about any of 10 % and 99 %, 30% and 95%, 30 % and 99 %, 50% and 95%, 50 % and 99 %, 75 % and 99 %, or 85 % and 99 %. In some embodiments, the amount of cell culture media component is reduced by about any of 10 %, 20 %, 30 %, 40 %, 50 %, 60 %, 70 %, 80 %, 90 %, 95 %, or 98 %. In some embodiments, the reduction is determined by comparing the amount of cell culture media component in the composition recovered from a purification step(s) to the amount of cell culture media component in the composition before the purification step(s).

In some embodiments of any of the methods described herein, the methods may further comprise one or more purification steps either prior to, or after, any of the OEC described herein. Other purification procedures include, for example, ion exchange chromatography such as anion exchange chromatography and cation exchange chromatography, affinity chromatography such as protein A chromatography and protein G chromatography, mixed mode chromatography, hydroxylapatite chromatography; gel filtration chromatography; affinity chromatography; gel electrophoresis; dialysis; ethanol precipitation; reverse phase HPLC; chromatography on silica; chromatofocusing; SDS-PAGE; ammonium sulfate precipitation; and metal chelating columns to bind epitope-tagged forms of the polypeptide.

In some embodiments of any of the methods described herein, the methods further comprise recovering the purified polypeptide. In some embodiments, the purified polypeptide is recovered from any of the purification steps described herein. The chromatography step may be cation exchange chromatography, mixed mode chromatography, or Protein A chromatography. In some embodiments, the OEC chromatography is a mixed mode chromatography and the further chromatography is an anion exchange chromatography. In some embodiments, the OEC chromatography is a mixed mode chromatography and the further chromatography is a cation exchange chromatography. In some embodiments, the OEC chromatography is a mixed mode chromatography and the further chromatography is a HIC chromatography. In some embodiments, the OEC chromatography is an anion exchange chromatography and the further chromatography is a cation exchange chromatography. In some embodiments, the OEC chromatography is an anion exchange chromatography and the further chromatography is a mixed mode chromatography. In some embodiments, the OEC chromatography is an anion exchange chromatography and the further chromatography is a HIC chromatography. In some embodiments, the OEC chromatography is a cation exchange chromatography and the further chromatography is an anion exchange chromatography. In some embodiments, the OEC chromatography is a cation exchange chromatography and the further chromatography is a mixed mode chromatography. In some embodiments, the OEC chromatography is a cation exchange chromatography and the further chromatography is a HIC chromatography. In some embodiments, the OEC chromatography is a HIC chromatography and the further chromatography is a mixed mode chromatography. In some embodiments, the OEC chromatography is a HIC chromatography and the further chromatography is an anion exchange chromatography. In some embodiments, the OEC chromatography is a HIC chromatography and the further chromatography is a cation exchange chromatography.

In some embodiments, the polypeptide is further purified following OEC by viral filtration. Viral filtration is the removal of viral contaminants in a polypeptide purification feedstream. Examples of viral filtration include ultrafiltration and microfiltration. In some embodiments the polypeptide is purified using a parvovirus filter.

In some embodiments, the polypeptide is concentrated after chromatography by OEC mode. Examples of concentration methods are known in the art and include but are not limited to ultrafiltration and diafiltration.

In some embodiments of any of the methods described herein, the methods further comprise combining the purified polypeptide of the methods of purification with a pharmaceutically acceptable carrier.

In some embodiments, the invention provides methods to purify an antibody comprising a) loading a composition comprising the antibody on a Capto Adhere resin at a load density of 150 - 200 g antibody per liter of Capto Adhere resin in a loading buffer with a pH of about 6.5 and a conductivity of about 5.3 mS/cm to about 5.6 mS/cm; b) eluting the antibody from the resin with an elution buffer comprising 100 mM 2-(N-morpholino)ethanesulfonic acid (MES) with a pH of about 6.5 and a conductivity of about 1 mS/cm; and collecting a pool comprising the antibody.

In some embodiments, the invention provides methods to purify an antibody comprising a) loading a composition comprising the antibody on a Capto Adhere resin in a 1.6 - 10.8 L column at a load density of 70-180 g antibody per liter of Capto Adhere resin in a loading buffer with a pH of about 6.5 and a conductivity of about 5.3 mS/cm to about 5.6 mS/cm; b) eluting the antibody from the resin with an elution buffer comprising 100 mM MES with a pH of about 6.5 and a conductivity of about 1 mS/cm; and collecting a pool comprising the antibody.

In some embodiments, the invention provides methods to purify an antibody comprising a) loading a composition comprising the antibody on a Capto Adhere resin at a load density of about 200 g antibody per liter of Capto Adhere resin in a loading buffer with a pH of about 8.6 and a conductivity of about less than 6 mS/cm; b) eluting the antibody from the resin with an elution buffer of 20 mM MES with a pH of about 6.5 and a conductivity of about 1 mS/cm; and collecting a pool comprising the antibody.

In some embodiments, the invention provides methods to purify an antibody comprising a) loading a composition comprising the antibody on a Capto Adhere resin at a load density of about 200 g antibody per liter of Capto Adhere resin in a loading buffer with a pH of about 6.1 and a conductivity of about less than 6 mS/cm; b) eluting the antibody from the resin with an elution buffer of 20 mM MES with a pH of about 6.0 and a conductivity of about 0.65 mS/cm; and collecting a pool comprising the antibody.

In some embodiments, the invention provides methods to purify an antibody comprising a) loading a composition comprising the antibody on a Capto Adhere resin at a load density of about 200 g antibody per liter of Capto Adhere resin in a loading buffer with a pH of about 5.5 and a conductivity of about less than 6 mS/cm; b) eluting the antibody from the resin with an elution buffer of 20 mM MES with a pH of about 4.9 and a conductivity of about 1.1 mS/cm; and collecting a pool comprising the antibody.

In some embodiments, the invention provides methods to purify an antibody comprising a) loading a composition comprising the antibody on a Capto Adhere resin at a load density of about 200 g antibody per liter of Capto Adhere resin in a loading buffer with a pH of about 6.5 and a conductivity of about less than 6 mS/cm; b) eluting the antibody from the resin with an elution buffer of 20 mM MES with a pH of about 6.5 and a conductivity of about 1 mS/cm; and collecting a pool comprising the antibody.

In some embodiments, the invention provides methods to purify an antibody comprising a) loading a composition comprising the antibody on a QMA resin at a load density of about 103 g antibody per liter of QMA resin in a loading buffer with a pH of about 6.5 and a conductivity of about less than 5.5 mS/cm; b) eluting the antibody from the resin with an elution buffer of 20 mM MES with a pH of about 6.5 and a conductivity of about 1 mS/cm; and collecting a pool comprising the antibody.

In some embodiments, the invention provides methods to purify an antibody comprising a) loading a composition comprising the antibody on a Poros XS resin at a load density of about 200 g antibody per liter of Poros XS resin in a loading buffer with a pH of about 5.5 and a conductivity of about less than 6 mS/cm; b) eluting the antibody from the resin with a 50-350 mM acetate elution buffer with a pH of about 5.5; and collecting a pool comprising the antibody.

In some embodiments, the invention provides methods to purify an antibody comprising a) loading a composition comprising the antibody on a Capto MMC resin at a load density of about 147 g antibody per liter of Capto MMC resin in a loading buffer with a pH of about 7.0 and a conductivity of about less than 6 mS/cm; b) eluting the antibody from the resin with an elution buffer of 20 mM MES with a pH of about 6.5 and a conductivity of about 1 mS/cm; and collecting a pool comprising the antibody.

*III. Polypeptides*

Polypeptides are provided for use in any of the methods of purifying polypeptides and formulations comprising the polypeptides purified by the methods described herein.

The invention provides methods to purify a polypeptide by using overload and elute chromatography. In some embodiments, the polypeptide is a therapeutic polypeptide. In some embodiments, the polypeptide is an antagonist. In some embodiments, the polypeptide is an agonist. In some embodiments, the polypeptide is an antibody. In some embodiments, the polypeptide is epitope tagged. In some embodiments, the polypeptide retains a biological and/or immunological activity. In some embodiments, the polypeptide is an antagonist. In some embodiments, the polypeptide initiates complement dependent cytotoxicity. In some embodiments the polypeptide is an antibody or immunoadhesin. In further embodiments of the above embodiments, the polypeptide is purified by OEC using a mixed mode chromatography media. In further embodiments of the above embodiments, the polypeptide is purified by OEC using an anion exchange chromatography media. In further embodiments of the above embodiments, the polypeptide is purified by OEC using a cation exchange chromatography media. In further embodiments of the above embodiments, the polypeptide is purified by OEC using a HIC chromatography media. In further embodiments of the above embodiments, the polypeptide is purified by OEC using a HAP chromatography media. In further embodiments of the above embodiments, the polypeptide is purified by OEC using an affinity chromatography media. In further embodiments of the above embodiments, the polypeptide is purified by OEC using a chromatography media that is not, or does not include, a cation exchange chromatography.

In some embodiments, the polypeptide has a molecular weight of greater than about any of 5,000 Daltons, 10,000 Daltons, 15,000 Daltons, 25,000 Daltons, 50,000 Daltons, 75,000 Daltons, 100,000 Dalton, 125,000 Daltons, or 150,000 Daltons. The polypeptide may have a molecular weight between about any of 50,000 Daltons to 200,000 Daltons or 100,000 Daltons to 200,000 Daltons. Alternatively, the polypeptide for use herein may have a molecular weight of about 120,000 Daltons or about 25,000 Daltons.

pI is the isoelectric point and is the pH at which a particular molecule or surface carries no net electrical charge. In some embodiments of any of the methods described herein, the pI of the polypeptide may be between about any of 6 to 10, 7 to 9, or 8 to 9. In some embodiments, the polypeptide has a pI of about any of 6, 7, 7.5, 8, 8.5, 9, 9.5, or 10.

The polypeptides to be purified using the methods described herein is generally produced using recombinant techniques. Methods for producing recombinant proteins are described, e.g., in U.S. Pat Nos. 5,534,615 and 4,816,567. In some embodiments, the protein of interest is produced in a CHO cell (see, e.g. WO 94/11026). When using recombinant techniques, the polypeptides can be produced intracellularly, in the periplasmic space, or directly secreted into the medium.

The polypeptides may be recovered from culture medium or from host cell lysates. Cells employed in expression of the polypeptides can be disrupted by various physical or chemical means, such as freeze-thaw cycling, sonication, mechanical disruption, or cell lysing agents. If the polypeptide is produced intracellularly, as a first step, the particulate debris, either host cells or lysed fragments, are removed, for example, by centrifugation or ultrafiltration. Carter et al., BiolTechnology 10: 163-167 (1992) describe a procedure for isolating polypeptides which are secreted to the periplasmic space of E. coli. Briefly, cell paste is thawed in the presence of sodium acetate (pH 3.5), EDTA, and phenylmethylsulfonylfluoride (PMSF) over about 30 min. Cell debris can be removed by centrifugation. Where the polypeptide is secreted into the medium, supernatants from such expression systems are generally first concentrated using a commercially available polypeptide concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

Examples of polypeptides that may be purified by the methods of the invention include but are not limited to immunoglobulins, immunoadhesins, antibodies, enzymes, hormones, fusion proteins, Fc-containing proteins, immunoconjugates, cytokines and interleukins. Examples of polypeptide include, but are not limited to, mammalian proteins, such as, e.g., renin; a hormone; a growth hormone, including human growth hormone and bovine growth hormone; growth hormone releasing factor; parathyroid hormone; thyroid stimulating hormone; lipoproteins; alpha-1-antitrypsin; insulin A-chain; insulin B-chain; proinsulin; follicle stimulating hormone; calcitonin; luteinizing hormone; glucagon; clotting factors such as factor VIIIC, factor IX, tissue factor, and von Willebrands factor; anti-clotting factors such as Protein C; atrial natriuretic factor; lung surfactant; a plasminogen activator, such as urokinase or human urine or tissue-type plasminogen activator (t-PA); bombesin; thrombin; hemopoietic growth factor; tumor necrosis factor-alpha and -beta; enkephalinase; RANTES (regulated on activation normally T-cell expressed and secreted); human macrophage inflammatory protein (MIP-1-alpha); a serum albumin such as human serum albumin; Muellerian-inhibiting substance; relaxin A-chain; relaxin B-chain; prorelaxin; mouse gonadotropin-associated peptide; an enzyme; a microbial protein, such as beta-lactamase; DNase; IgE; a cytotoxic T-lymphocyte associated antigen (CTLA), such as CTLA-4; inhibin; activin; vascular endothelial growth factor (VEGF); receptors for hormones or growth factors; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-b; platelet-derived growth factor (PDGF); fibroblast growth factor such as aFGF and bFGF; epidermal growth factor (EGF); transforming growth factor (TGF) such as TGF-alpha and TGF-beta, including TGF-β1, TGF-β2, TGF-β3, TGF-β4, or TGF-β5; insulin-like growth factor-I and -II (IGF-I and IGF-II); des(1-3)-IGF-I (brain IGF-I), insulin-like growth factor binding proteins (IGFBPs); a cytokine; CD proteins such as CD3, CD4, CD8, CD19 and CD20; erythropoietin; osteoinductive factors; immunotoxins; a fusion polypeptide, i.e. a polypeptide comprised on two or more heterologous polypeptides or fragments thereof and encoded by a recombinant nucleic acid; an Fc-containing polypeptide, for example, a fusion protein comprising an immunoglobulin Fc region, or fragment thereof, fused to a second polypeptide; an immunoconjugate; a bone morphogenetic protein (BMP); an interferon such as interferon-alpha, -beta, and -gamma; colony stimulating factors (CSFs), e.g., M-CSF, GM-CSF, and G-CSF; interleukins (ILs), e.g., IL-1 to IL-10;

superoxide dismutase; T-cell receptors; surface membrane proteins; decay accelerating factor; viral antigen such as, for example, a portion of the AIDS envelope; transport proteins; homing receptors; addressins; regulatory proteins; integrins such as CD11a, CD11b, CD11c, CD18, an ICAM, VLA-4 and VCAM; a tumor associated antigen such as CA125 (ovarian cancer antigen) or HER2, HER3 or HER4 receptor; immunoadhesins; and fragments and/or variants of any of the above-listed proteins as well as antibodies, including antibody fragments, binding to a protein, including, for example, any of the above-listed proteins.

*(A) Antibodies*

In some embodiments of any of the methods described herein, the polypeptide for use in any of the methods of purifying polypeptides and formulations comprising the polypeptides purified by the methods described herein is an antibody.

Molecular targets for antibodies include CD proteins and their ligands, such as, but not limited to: (i) CD3, CD4, CD8, CD19, CD11a, CD20, CD22, CD34, CD40, CD79$\alpha$ (CD79a), and CD79$\beta$ (CD79b); (ii) members of the ErbB receptor family such as the EGF receptor, HER2, HER3 or HER4 receptor; (iii) cell adhesion molecules such as LFA-1, Mac1, p150,95, VLA-4, ICAM-1, VCAM and $\alpha$v/$\beta$3 integrin, including either alpha or beta subunits thereof (*e.g.,* anti-CD11a, anti-CD18 or anti-CD11b antibodies); (iv) growth factors such as VEGF; IgE; blood group antigens; flk2/flt3 receptor; obesity (OB) receptor; *mpl* receptor; CTLA-4; protein C, BR3, c-met, tissue factor, $\beta$7 etc; and (v) cell surface and transmembrane tumor-associated antigens (TAA), such as those described in U.S. Patent No. 7,521,541.

Other exemplary antibodies include those selected from, and without limitation, anti-estrogen receptor antibody, anti-progesterone receptor antibody, anti-p53 antibody, anti-HER-2/neu antibody, anti-EGFR antibody, anti-cathepsin D antibody, anti-Bcl-2 antibody, anti-E-cadherin antibody, anti-CA125 antibody, anti-CA15-3 antibody, anti-CA19-9 antibody, anti-c-erbB-2 antibody, anti-P-glycoprotein antibody, anti-CEA antibody, anti-retinoblastoma protein antibody, anti-ras oncoprotein antibody, anti-Lewis X antibody, anti-Ki-67 antibody, anti-PCNA antibody, anti-CD3 antibody, anti-CD4 antibody, anti-CD5 antibody, anti-CD7 antibody, anti-CD8 antibody, anti-CD9/p24 antibody, anti-CD10 antibody, anti-CD11a antibody, anti-CD11c antibody, anti-CD13 antibody, anti-CD14 antibody, anti-CD15 antibody, anti-CD19 antibody, anti-CD20 antibody, anti-CD22 antibody, anti-CD23 antibody, anti-CD30 antibody, anti-CD31 antibody, anti-CD33 antibody, anti-CD34 antibody, anti-CD35 antibody, anti-CD38 antibody, anti-CD41 antibody, anti-LCA/CD45 antibody, anti-CD45RO antibody, anti-CD45RA antibody, anti-CD39 antibody, anti-CD100 antibody, anti-CD95/Fas antibody, anti-CD99 antibody, anti-CD106 antibody, anti-ubiquitin antibody, anti-CD71 antibody, anti-c-myc antibody, anti-cytokeratins antibody, anti-vimentins antibody, anti-HPV proteins antibody, anti-kappa light chains antibody, anti-lambda light chains antibody, anti-melanosomes antibody, anti-prostate specific antigen antibody, anti-S-100 antibody, anti-tau antigen antibody, anti-fibrin antibody, anti-keratins antibody and anti-Tn-antigen antibody.

*(i) Polyclonal antibodies*

In some embodiments, the antibodies are polyclonal antibodies. Polyclonal antibodies are preferably raised in animals by multiple subcutaneous (sc) or intraperitoneal (ip) injections of the relevant antigen and an adjuvant. It may be useful to conjugate the relevant antigen to a polypeptide that is immunogenic in the species to be immunized, *e.g.*, keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl$_2$, or R$^1$N=C=NR, where R and R$^1$ are different alkyl groups.

Animals are immunized against the antigen, immunogenic conjugates, or derivatives by combining, *e.g.*, 100 $\mu$g or 5 $\mu$g of the polypeptide or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. In some embodiments, the animal is boosted with the conjugate of the same antigen, but conjugated to a different polypeptide and/or through a different cross-linking reagent. Conjugates also can be made in recombinant cell culture as polypeptide fusions. Also, aggregating agents such as alum are suitably used to enhance the immune response.

*(ii) Monoclonal antibodies*

In some embodiments, the antibodies are monoclonal antibodies. Monoclonal antibodies are obtained from a population of substantially homogeneous antibodies, *i.e.,* the individual antibodies comprising the population are identical and/or bind the same epitope except for possible variants that arise during production of the monoclonal antibody, such variants generally being present in minor amounts. Thus, the modifier "monoclonal" indicates the character of the antibody as not being a mixture of discrete or polyclonal antibodies.

For example, the monoclonal antibodies may be made using the hybridoma method first described by Kohler et al., Nature 256:495 (1975), or may be made by recombinant DNA methods (U.S. Patent No. 4,816,567).

In the hybridoma method, a mouse or other appropriate host animal, such as a hamster, is immunized as herein described to elicit lymphocytes that produce or are capable of producing antibodies that will specifically bind to the polypeptide used for immunization. Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

In some embodiments, the myeloma cells are those that fuse efficiently, support stable high-level production of antibody by the selected antibody-producing cells, and are sensitive to a medium such as HAT medium. Among these, in some embodiments, the myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol. 133:3001 (1984); Brodeur et al., Monoclonal Antibody Production Techniques and Applications pp. 51-63 (Marcel Dekker, Inc., New York, 1987)).

Culture medium in which hybridoma cells are growing is assayed for production of monoclonal antibodies directed against the antigen. In some embodiments, the binding specificity of monoclonal antibodies produced by hybridoma cells is determined by immunoprecipitation or by an in vitro binding assay, such as radioimmunoassay (RIA) or enzyme-linked immunoabsorbent assay (ELISA).

The binding affinity of the monoclonal antibody can, for example, be determined by the Scatchard analysis of Munson et al., Anal. Biochem. 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice pp. 59-103 (Academic Press, 1986)). Suitable culture media for this purpose include, for example, D-MEM or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional immunoglobulin purification procedures such as, for example, polypeptide A-Sepharose, hydroxylapatite chromatography, gel electrophoresis, dialysis, or affinity chromatography.

DNA encoding the monoclonal antibodies is readily isolated and sequenced using conventional procedures (*e.g.,* by using oligonucleotide probes that are capable of binding specifically to genes encoding the heavy and light chains of murine antibodies). In some embodiments, the hybridoma cells serve as a source of such DNA. Once isolated, the DNA may be placed into expression vectors, which are then transfected into host cells such as E. coli cells, simian COS cells, Chinese Hamster Ovary (CHO) cells, or myeloma cells that do not otherwise produce immunoglobulin polypeptide, to obtain the synthesis of monoclonal antibodies in the recombinant host cells. Review articles on recombinant expression in bacteria of DNA encoding the antibody include Skerra et al., Curr. Opinion in Immunol. 5:256-262 (1993) and Plückthun, Immunol. Revs., 130:151-188 (1992).

In a further embodiment, antibodies or antibody fragments can be isolated from antibody phage libraries generated using the techniques described in McCafferty et al., Nature 348:552-554 (1990). Clackson et al., Nature 352:624-628 (1991) and Marks et al., J. Mol. Biol. 222:581-597 (1991) describe the isolation of murine and human antibodies, respectively, using phage libraries. Subsequent publications describe the production of high affinity (nM range) human antibodies by chain shuffling (Marks et al., BiolTechnology 10:779-783 (1992)), as well as combinatorial infection and in vivo recombination as a strategy for constructing very large phage libraries (Waterhouse et al., Nuc. Acids. Res. 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy- and light chain constant domains in place of the homologous murine sequences (U.S. Patent No. 4,816,567; Morrison et al., Proc. Natl Acad. Sci. USA 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

In some embodiments of any of the methods described herein, the antibody is IgA, IgD, IgE, IgG, or IgM. In some embodiments, the antibody is an IgG monoclonal antibody.

*(iii) Humanized antibodies*

In some embodiments, the antibody is a humanized antibody. Methods for humanizing non-human antibodies have been described in the art. In some embodiments, a humanized antibody has one or more amino acid residues introduced into it from a source that is non-human. These non-human amino acid residues are often referred to as "import" residues, which are typically taken from an "import" variable domain. Humanization can be essentially performed following the method of Winter and co-workers (Jones et al., Nature 321:522-525 (1986); Riechmann et al., Nature 332:323-327 (1988); Verhoeyen et al., Science 239:1534-1536 (1988)), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence that is closest to that of the rodent is then accepted as the human framework region (FR) for the humanized antibody (Sims et al., J. Immunol. 151:2296 (1993); Chothia et al., J. Mol. Biol. 196:901 (1987)). Another method uses a particular framework region derived from the consensus sequence of all human antibodies of a particular subgroup of light or heavy chain variable regions. The same framework may be used for several different humanized antibodies (Carter et al., Proc. Natl. Acad. Sci. USA 89:4285 (1992); Presta et al., J. Immunol. 151:2623 (1993)).

It is further important that antibodies be humanized with retention of high affinity for the antigen and other favorable biological properties. To achieve this goal, in some embodiments of the methods, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available that illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

*(v) Human antibodies*

In some embodiments, the antibody is a human antibody. As an alternative to humanization, human antibodies can be generated. For example, it is now possible to produce transgenic animals (*e.g.,* mice) that are capable, upon immunization, of producing a full repertoire of human antibodies in the absence of endogenous immunoglobulin production. For example, it has been described that the homozygous deletion of the antibody heavy chain joining region ($J_H$) gene in chimeric and germ-line mutant mice results in complete inhibition of endogenous antibody production. Transfer of the human germ-line immunoglobulin gene array in such germ-line mutant mice will result in the production of human antibodies upon antigen challenge. *See, e.g.,* Jakobovits et al., Proc. Natl. Acad. Sci. USA 90:2551 (1993); Jakobovits et al., Nature 362:255-258 (1993); Bruggermann et al., Year in Immuno. 7:33 (1993); and US Patent Nos. 5,591,669; 5,589,369; and 5,545,807.

Alternatively, phage display technology (McCafferty et al., Nature 348:552-553 (1990)) can be used to produce human antibodies and antibody fragments *in vitro,* from immunoglobulin variable (V) domain gene repertoires from unimmunized donors. According to this technique, antibody V domain genes are cloned in-frame into either a major or minor coat polypeptide gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review *see, e.g.,* Johnson, Kevin S. and Chiswell, David J., Current Opinion in Structural Biology 3:564-571 (1993). Several sources of V-gene segments can be used for phage display. Clackson et al., Nature 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). *See also,* US Patent Nos. 5,565,332 and 5,573,905.

Human antibodies may also be generated by *in vitro* activated B cells (*see* US Patents 5,567,610 and 5,229,275).

*(v) Antibody fragments*

In some embodiments, the antibody is an antibody fragment. Various techniques have been developed for the production of antibody fragments. Traditionally, these fragments were derived via proteolytic digestion of intact antibodies (*see, e.g.,* Morimoto et al., Journal of Biochemical and Biophysical Methods 24:107-117 (1992) and Brennan et al., Science 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form $F(ab')_2$ fragments (Carter et al., BiolTechnology 10:163-167 (1992)). According to another approach, $F(ab')_2$ fragments can be isolated directly from recombinant host cell culture. Other techniques for the production of antibody fragments will be apparent to the skilled practitioner. In other embodiments, the antibody of choice is a single chain Fv fragment (scFv). *See* WO 93/16185; US Patent No. 5,571,894; and US Patent No. 5,587,458. The antibody fragment may also be a "linear antibody," *e.g.*, as described in US Patent 5,641,870 for example. Such linear antibody fragments may be monospecific or bispecific.

In some embodiments, fragments of the antibodies described herein are provided. In some embodiments, the antibody fragment is an antigen binding fragment. In some embodiments, the antigen binding fragment is selected from the group consisting of a Fab fragment, a Fab' fragment, a $F(ab')_2$ fragment, a scFv, a Fv, and a diabody.

*(vi) Bispecific antibodies*

In some embodiments, the antibody is a bispecific antibody. Bispecific antibodies are antibodies that have binding specificities for at least two different epitopes. Exemplary bispecific antibodies may bind to two different epitopes. Alternatively, a bispecific antibody binding arm may be combined with an arm that binds to a triggering molecule on a leukocyte such as a T-cell receptor molecule (*e.g.* CD2 or CD3), or Fc receptors for IgG (FcγR), such as FcγRI (CD64), FcγRII (CD32) and FcγRIII (CD16) so as to focus cellular defense mechanisms to the cell. Bispecific antibodies can be prepared as full length antibodies or antibody fragments (*e.g.* $F(ab')_2$ bispecific antibodies).

Methods for making bispecific antibodies are known in the art. Traditional production of full length bispecific antibodies is based on the coexpression of two immunoglobulin heavy chain-light chain pairs, where the two chains have different specificities (Millstein et al., Nature 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991).

According to a different approach, antibody variable domains with the desired binding specificities (antibody-antigen combining sites) are fused to immunoglobulin constant domain sequences. In some embodiments, the fusion is with an immunoglobulin heavy chain constant domain, comprising at least part of the hinge, CH2, and CH3 regions. In some embodiments, the first heavy chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In some embodiments of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies *see,* for example, Suresh et al., Methods in Enzymology 121:210 (1986).

According to another approach described in US Patent No. 5,731,168, the interface between a pair of antibody molecules can be engineered to maximize the percentage of heterodimers that are recovered from recombinant cell culture. In some embodiments, the interface comprises at least a part of the $C_H3$ domain of an antibody constant domain. In this method, one or more small amino acid side chains from the interface of the first antibody molecule are replaced with larger side chains (*e.g.* tyrosine or tryptophan). Compensatory "cavities" of identical or similar size to the large side chain(s) are created on the interface of the second antibody molecule by replacing large amino acid side chains with smaller ones (*e.g.* alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229: 81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy chain variable domain ($V_H$) connected to a light chain variable domain ($V_L$) by a linker that is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. *See* Gruber et al., J. Immunol. 152:5368 (1994).

Antibodies with more than two valencies are contemplated. For example, trispecific antibodies can be prepared. Tutt et al., J. Immunol. 147: 60 (1991).

*(vii) Multivalent Antibodies*

In some embodiments, the antibodies are multivalent antibodies. A multivalent antibody may be internalized (and/or catabolized) faster than a bivalent antibody by a cell expressing an antigen to which the antibodies bind. The antibodies provided herein can be multivalent antibodies (which are other than of the IgM class) with three or more antigen binding sites (*e.g.,* tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody will comprise an Fc region and three or more antigen binding sites amino-terminal to the Fc region. The preferred multivalent antibody herein comprises (or consists of) three to about eight, but preferably four, antigen binding sites. The multivalent antibody comprises at least one polypeptide chain (and preferably two polypeptide chains), wherein the polypeptide chain(s) comprise two or more variable domains. For instance, the polypeptide chain(s) may comprise VD1-(X1)n-VD2-(X2) n-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

In some embodiments, the antibody is a multispecific antibody. Example of multispecific antibodies include, but are not limited to, an antibody comprising a heavy chain variable domain ($V_H$) and a light chain variable domain ($V_L$), where the $V_HV_L$ unit has polyepitopic specificity, antibodies having two or more $V_L$ and $V_H$ domains with each $V_HV_L$ unit binding to a different epitope, antibodies having two or more single variable domains with each single variable domain binding to a different epitope, full length antibodies, antibody fragments such as Fab, Fv, dsFv, scFv, diabodies, bispecific diabodies, triabodies, tri-functional antibodies, antibody fragments that have been linked covalently or non-covalently. In some embodiment that antibody has polyepitopic specificity; for example, the ability to specifically bind to two or more different epitopes on the same or different target(s). In some embodiments, the antibodies are monospecific; for example, an antibody that binds only one epitope. According to one embodiment the multispecific antibody is an IgG antibody that binds to each epitope with an affinity of 5 $\mu$M to 0.001 pM, 3 $\mu$M to 0.001 pM, 1 $\mu$M to 0.001 pM, 0.5 $\mu$M to 0.001 pM, or 0.1 $\mu$M to 0.001 pM.

*(viii) Other Antibody Modifications*

It may be desirable to modify the antibody provided herein with respect to effector function, *e.g.*, so as to enhance antigen-dependent cell-mediated cyotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). *See* Caron et al., J. Exp Med. 176:1191-1195 (1992) and Shopes, B. J., Immunol. 148:2918-2922 (1992). Homodimeric antibodies with enhanced anti-tumor activity may also be prepared using heterobifunctional cross-linkers as described in Wolff et al., Cancer Research 53:2560-2565 (1993). Alternatively, an antibody can be engineered which has dual Fc regions and may thereby have enhanced complement mediated lysis and ADCC capabilities. *See* Stevenson et al., Anti-Cancer Drug Design 3:219-230 (1989).

For increasing serum half the serum half life of the antibody, amino acid alterations can be made in the antibody as described in US 2006/0067930.

*(B) Polypeptide Variants and Modifications*

Amino acid sequence modification(s) of the polypeptides, including antibodies, described herein may be used in the methods of purifying polypeptides (*e.g.,* antibodies) described herein.

*(i) Variant Polypeptides*

"Polypeptide variant" means a polypeptide, preferably an active polypeptide, as defined herein having at least about 80% amino acid sequence identity with a full-length native sequence of the polypeptide, a polypeptide sequence lacking the signal peptide, an extracellular domain of a polypeptide, with or without the signal peptide. Such polypeptide variants include, for instance, polypeptides wherein one or more amino acid residues are added, or deleted, at the N or C-terminus of the full-length native amino acid sequence. Ordinarily, a TAT polypeptide variant will have at least about 80% amino acid sequence identity, alternatively at least about any of 85%, 90%, 95%, 96%, 97%, 98%, or 99% amino acid sequence identity, to a full-length native sequence polypeptide sequence, a polypeptide sequence lacking the signal peptide, an extracellular domain of a polypeptide, with or without the signal peptide. Optionally, variant polypeptides will have no more than one conservative amino acid substitution as compared to the native polypeptide sequence, alternatively no more than about any of 2, 3, 4, 5, 6, 7, 8, 9, or 10 conservative amino acid substitution as compared to the native polypeptide sequence.

The variant polypeptide may be truncated at the N-terminus or C-terminus, or may lack internal residues, for example, when compared with a full length native polypeptide. Certain variant polypeptides may lack amino acid residues that are not essential for a desired biological activity. These variant polypeptides with truncations, deletions, and insertions may be prepared by any of a number of conventional techniques. Desired variant polypeptides may be chemically synthesized. Another suitable technique involves isolating and amplifying a nucleic acid fragment encoding a desired variant polypeptide, by polymerase chain reaction (PCR). Oligonucleotides that define the desired termini of the nucleic acid fragment are employed at the 5' and 3' primers in the PCR. Preferably, variant polypeptides share at least one biological and/or immunological activity with the native polypeptide disclosed herein.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N- or C-terminus of the antibody to an enzyme or a polypeptide which increases the serum half-life of the antibody.

For example, it may be desirable to improve the binding affinity and/or other biological properties of the polypeptide. Amino acid sequence variants of the polypeptide are prepared by introducing appropriate nucleotide changes into the antibody nucleic acid, or by peptide synthesis. Such modifications include, for example, deletions from, and/or insertions into and/or substitutions of, residues within the amino acid sequences of the polypeptide. Any combination of deletion, insertion, and substitution is made to arrive at the final construct, provided that the final construct possesses the desired characteristics. The amino acid changes also may alter post-translational processes of the polypeptide (*e.g.,* antibody), such as changing the number or position of glycosylation sites.

Guidance in determining which amino acid residue may be inserted, substituted or deleted without adversely affecting the desired activity may be found by comparing the sequence of the polypeptide with that of homologous known polypeptide molecules and minimizing the number of amino acid sequence changes made in regions of high homology.

A useful method for identification of certain residues or regions of the polypeptide (*e.g.,* antibody) that are preferred locations for mutagenesis is called "alanine scanning mutagenesis" as described by Cunningham and Wells, Science 244:1081-1085 (1989). Here, a residue or group of target residues are identified (*e.g.,* charged residues such as Arg, Asp, His, Lys, and Glu) and replaced by a neutral or negatively charged amino acid (most preferably Alanine or Polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed antibody variants are screened for the desired activity.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. Conservative substitutions are shown in the Table 1 below under the heading of "preferred substitutions." If such substitutions result in a change in biological activity, then more substantial changes, denominated "exemplary substitutions" in the Table 1, or as further described below in reference to amino acid classes, may be introduced and the products screened.

*Table 1.*

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in the biological properties of the polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, Biochemistry second ed., pp. 73-75, Worth Publishers, New York (1975)):

(1) non-polar: Ala (A), Val (V), Leu (L), Ile (I), Pro (P), Phe (F), Trp (W), Met (M)
(2) uncharged polar: Gly (G), Ser (S), Thr (T), Cys (C), Tyr (Y), Asn (N), Gln (Q)
(3) acidic: Asp (D), Glu (E)
(4) basic: Lys (K), Arg (R), His(H)

Alternatively, naturally occurring residues may be divided into groups based on common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

Any cysteine residue not involved in maintaining the proper conformation of the antibody also may be substituted, generally with serine, to improve the oxidative stability of the molecule and prevent aberrant crosslinking. Conversely, cysteine bond(s) may be added to the polypeptide to improve its stability (particularly where the antibody is an antibody fragment such as an Fv fragment).

A particularly preferred type of substitutional variant involves substituting one or more hypervariable region residues of a parent antibody (e.g., a humanized antibody). Generally, the resulting variant(s) selected for further development will have improved biological properties relative to the parent antibody from which they are generated. A convenient way for generating such substitutional variants involves affinity maturation using phage display. Briefly, several hypervariable region sites (e.g., 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (e.g., binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and target. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the polypeptide alters the original glycosylation pattern of the antibody. The polypeptide may comprise non-amino acid moieties. For example, the polypeptide may be glycosylated. Such glycosylation may occur naturally during expression of the polypeptide in the host cell or host organism, or may be a deliberate modification arising from human intervention. By altering is meant deleting one or more carbohydrate moieties found in the polypeptide, and/or adding one or more glycosylation sites that are not present in the polypeptide.

Glycosylation of polypeptide is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the polypeptide is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Removal of carbohydrate moieties present on the polypeptide may be accomplished chemically or enzymatically or by mutational substitution of codons encoding for amino acid residues that serve as targets for glycosylation. Enzymatic cleavage of carbohydrate moieties on polypeptides can be achieved by the use of a variety of endo- and exo-glycosidases.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the $\alpha$-amino groups of lysine, arginine, and histidine side chains, acetylation of the N-terminal amine, and amidation of any C-terminal carboxyl group.

*(ii) Chimeric Polypeptides*

The polypeptide described herein may be modified in a way to form chimeric molecules comprising the polypeptide fused to another, heterologous polypeptide or amino acid sequence. In some embodiments, a chimeric molecule comprises a fusion of the polypeptide with a tag polypeptide which provides an epitope to which an anti-tag antibody can selectively bind. The epitope tag is generally placed at the amino- or carboxyl-terminus of the polypeptide. The presence of such epitope-tagged forms of the polypeptide can be detected using an antibody against the tag polypeptide. Also, provision of the epitope tag enables the polypeptide to be readily purified by affinity purification using an anti-tag antibody or another type of affinity matrix that binds to the epitope tag.

In an alternative embodiment, the chimeric molecule may comprise a fusion of the polypeptide with an immunoglobulin or a particular region of an immunoglobulin. A bivalent form of the chimeric molecule is referred to as an "immunoadhesin."

As used herein, the term "immunoadhesin" designates antibody-like molecules which combine the binding specificity of a heterologous polypeptide with the effector functions of immunoglobulin constant domains. Structurally, the immunoadhesins comprise a fusion of an amino acid sequence with the desired binding specificity which is other than the antigen recognition and binding site of an antibody (*i.e.,* is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

The Ig fusions preferably include the substitution of a soluble (transmembrane domain deleted or inactivated) form of a polypeptide in place of at least one variable region within an Ig molecule. In a particularly preferred embodiment, the immunoglobulin fusion includes the hinge, $CH_2$ and $CH_3$, or the hinge, $CH_1$, $CH_2$ and $CH_3$ regions of an IgG1 molecule.

*(iii) Polypeptide Conjugates*

The polypeptide for use in polypeptide formulations may be conjugated to a cytotoxic agent such as a chemotherapeutic agent, a growth inhibitory agent, a toxin (e.g., an enzymatically active toxin of bacterial, fungal, plant, or animal origin, or fragments thereof), or a radioactive isotope (*i.e.,* a radioconjugate).

Chemotherapeutic agents useful in the generation of such conjugates can be used. In addition, enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated polypeptides. Examples include $^{212}Bi$, $^{131}I$, $^{131}In$, $^{90}Y$, and $^{186}Re$. Conjugates of the polypeptide and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis(p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the polypeptide.

Conjugates of a polypeptide and one or more small molecule toxins, such as a calicheamicin, maytansinoids, a trichothene, and CC1065, and the derivatives of these toxins that have toxin activity, are also contemplated herein.

Maytansinoids are mitototic inhibitors which act by inhibiting tubulin polymerization. Maytansine was first isolated from the east African shrub *Maytenus serrata.* Subsequently, it was discovered that certain microbes also produce maytansinoids, such as maytansinol and C-3 maytansinol esters. Synthetic maytansinol and derivatives and analogues thereof are also contemplated. There are many linking groups known in the art for making polypeptide-maytansinoid conjugates, including, for example, those disclosed in U.S. Pat. No. 5,208,020. The linking groups include disufide groups, thioether groups, acid labile groups, photolabile groups, peptidase labile groups, or esterase labile groups, as disclosed in the above-identified patents, disulfide and thioether groups being preferred.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hyrdoxymethyl, the C-15 position modified with a hydroxyl group, and the C-20 position having a hydroxyl group. In a preferred embodiment, the linkage is formed at the C-3 position of maytansinol or a maytansinol analogue.

Another conjugate of interest comprises a polypeptide conjugated to one or more calicheamicin molecules. The calicheamicin family of antibiotics are capable of producing double-stranded DNA breaks at sub-picomolar concentrations. For the preparation of conjugates of the calicheamicin family, *see, e.g.,* U.S. Pat. No. 5,712,374. Structural analogues of calicheamicin which may be used include, but are not limited to, $\gamma_1^I$, $\alpha_2^I$, $\alpha_3^I$, N-acetyl-$\gamma_1^I$, PSAG and $\theta_1^I$. Another anti-tumor drug that the antibody can be conjugated is QFA which is an antifolate. Both calicheamicin and QFA have intracellular sites of action and do not readily cross the plasma membrane. Therefore, cellular uptake of these agents through polypeptide (*e.g.*, antibody) mediated internalization greatly enhances their cytotoxic effects.

Other antitumor agents that can be conjugated to the polypeptides described herein include BCNU, streptozoicin, vincristine and 5-fluorouracil, the family of agents known collectively LL-E33288 complex, as well as esperamicins.

In some embodiments, the polypeptide may be a conjugate between a polypeptide and a compound with nucleolytic activity (*e.g.*, a ribonuclease or a DNA endonuclease such as a deoxyribonuclease; DNase).

In yet another embodiment, the polypeptide (*e.g.*, antibody) may be conjugated to a "receptor" (such streptavidin) for utilization in tumor pre-targeting wherein the polypeptide receptor conjugate is administered to the patient, followed by removal of unbound conjugate from the circulation using a clearing agent and then administration of a "ligand" (*e.g.*, avidin) which is conjugated to a cytotoxic agent (*e.g.*, a radionucleotide).

In some embodiments, the polypeptide may be conjugated to a prodrug-activating enzyme which converts a prodrug (*e.g.*, a peptidyl chemotherapeutic agent) to an active anti-cancer drug. The enzyme component of the immunoconjugate includes any enzyme capable of acting on a prodrug in such a way so as to covert it into its more active, cytotoxic form.

Enzymes that are useful include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galactosidase and neuraminidase useful for converting glycosylated prodrugs into free drugs; β-lactamase useful for converting drugs derivatized with β-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs into free active drugs.

*(iv) Other*

Another type of covalent modification of the polypeptide comprises linking the polypeptide to one of a variety of nonproteinaceous polymers, *e.g.*, polyethylene glycol, polypropylene glycol, polyoxyalkylenes, or copolymers of polyethylene glycol and polypropylene glycol. The polypeptide also may be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, 18th edition, Gennaro, A.R., Ed., (1990).

### IV. Obtaining Polypeptides for Use in the Formulations and Methods

The polypeptides used in the methods of purification described herein may be obtained using methods well-known in the art, including the recombination methods. The following sections provide guidance regarding these methods.

*(A) Polynucleotides*

"Polynucleotide," or "nucleic acid," as used interchangeably herein, refer to polymers of nucleotides of any length, and include DNA and RNA.

Polynucleotides encoding polypeptides may be obtained from any source including, but not limited to, a cDNA library prepared from tissue believed to possess the polypeptide mRNA and to express it at a detectable level. Accordingly, polynucleotides encoding polypeptide can be conveniently obtained from a cDNA library prepared from human tissue. The polypeptide-encoding gene may also be obtained from a genomic library or by known synthetic procedures (*e.g.*, automated nucleic acid synthesis).

For example, the polynucleotide may encode an entire immunoglobulin molecule chain, such as a light chain or a heavy chain. A complete heavy chain includes not only a heavy chain variable region ($V_H$) but also a heavy chain constant region ($C_H$), which typically will comprise three constant domains: $C_H1$, $C_H2$ and $C_H3$; and a "hinge" region. In some situations, the presence of a constant region is desirable.

Other polypeptides which may be encoded by the polynucleotide include antigen-binding antibody fragments such as single domain antibodies ("dAbs"), Fv, scFv, Fab' and F(ab')$_2$ and "minibodies." Minibodies are (typically) bivalent antibody fragments from which the $C_H1$ and $C_K$ or $C_L$ domain has been excised. As minibodies are smaller than conventional antibodies they should achieve better tissue penetration in clinical/diagnostic use, but being bivalent they should retain higher binding affinity than monovalent antibody fragments, such as dAbs. Accordingly, unless the context dictates otherwise, the term "antibody" as used herein encompasses not only whole antibody molecules but also antigen-binding antibody fragments of the type discussed above. Preferably each framework region present in the encoded polypeptide will comprise at least one amino acid substitution relative to the corresponding human acceptor framework. Thus, for example, the framework regions may comprise, in total, three, four, five, six, seven, eight, nine, ten, eleven, twelve, thirteen, fourteen, or fifteen amino acid substitutions relative to the acceptor framework regions.

Suitably, the polynucleotides described herein may be isolated and/or purified. In some embodiments, the polynucleotides are isolated polynucleotides.

The term "isolated polynucleotide" is intended to indicate that the molecule is removed or separated from its normal or natural environment or has been produced in such a way that it is not present in its normal or natural environment. In some embodiments, the polynucleotides are purified polynucleotides. The term purified is intended to indicate that at least some contaminating molecules or substances have been removed.

Suitably, the polynucleotides are substantially purified, such that the relevant polynucleotides constitutes the dominant (*i.e.,* most abundant) polynucleotides present in a composition.

*(B) Expression of Polynucleotides*

The description below relates primarily to production of polypeptides by culturing cells transformed or transfected with a vector containing polypeptide-encoding polynucleotides. It is, of course, contemplated that alternative methods, which are well known in the art, may be employed to prepare polypeptides. For instance, the appropriate amino acid sequence, or portions thereof, may be produced by direct peptide synthesis using solid-phase techniques (*see, e.g.,* Stewart et al., Solid-Phase Peptide Synthesis W.H. Freeman Co., San Francisco, Calif. (1969); Merrifield, J. Am. Chem. Soc. 85:2149-2154 (1963)). In vitro protein synthesis may be performed using manual techniques or by automation. Automated synthesis may be accomplished, for instance, using an Applied Biosystems Peptide Synthesizer (Foster City, Calif.) using manufacturer's instructions. Various portions of the polypeptide may be chemically synthesized separately and combined using chemical or enzymatic methods to produce the desired polypeptide.

Polynucleotides as described herein are inserted into an expression vector(s) for production of the polypeptides. The term "control sequences" refers to DNA sequences necessary for the expression of an operably linked coding sequence in a particular host organism. The control sequences include, but are not limited to, promoters (*e.g.,* naturally-associated or heterologous promoters), signal sequences, enhancer elements, and transcription termination sequences.

A polynucleotide is "operably linked" when it is placed into a functional relationship with another polynucleotide sequence. For example, nucleic acids for a presequence or secretory leader is operably linked to nucleic acids for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the nucleic acid sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

For antibodies, the light and heavy chains can be cloned in the same or different expression vectors. The nucleic acid segments encoding immunoglobulin chains are operably linked to control sequences in the expression vector(s) that ensure the expression of immunoglobulin polypeptides.

The vectors containing the polynucleotide sequences (*e.g.,* the variable heavy and/or variable light chain encoding sequences and optional expression control sequences) can be transferred into a host cell by well-known methods, which vary depending on the type of cellular host. For example, calcium chloride transfection is commonly utilized for prokaryotic cells, whereas calcium phosphate treatment, electroporation, lipofection, biolistics or viral-based transfection may be used for other cellular hosts. (*See generally* Sambrook et al., Molecular Cloning: A Laboratory Manual (Cold Spring Harbor Press, 2nd ed., 1989). Other methods used to transform mammalian cells include the use of polybrene, protoplast fusion, liposomes, electroporation, and microinjection. For production of non-human transgenic animals, transgenes can be microinjected into non-human fertilized oocytes, or can be incorporated into the genome of non-human embryonic stem cells, and the nuclei of such cells transferred into enucleated non-human oocytes.

*(C) Vectors*

The term "vector" includes expression vectors and transformation vectors and shuttle vectors.

The term "expression vector" means a construct capable of *in vivo* or *in vitro* expression.

The term "transformation vector" means a construct capable of being transferred from one entity to another entity - which may be of the species or may be of a different species. If the construct is capable of being transferred from one species to another - such as from an *Escherichia coli* plasmid to a bacterium, such as of the genus *Bacillus,* then the transformation vector is sometimes called a "shuttle vector". It may even be a construct capable of being transferred from an *E. coli* plasmid to an *Agrobacterium* to a plant.

Vectors may be transformed into a suitable host cell as described below to provide for expression of a polypeptide. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Construction of suitable vectors containing one or more of these components employs standard ligation techniques which are known to the skilled artisan.

The vectors may be for example, plasmid, virus or phage vectors provided with an origin of replication, optionally a promoter for the expression of the said polynucleotide and optionally a regulator of the promoter. Vectors may contain one or more selectable marker genes which are well known in the art.

These expression vectors are typically replicable in the host organisms either as episomes or as an integral part of the host chromosomal DNA.

*(D) Host Cells*

The host cell may be a bacterium, a yeast or other fungal cell, insect cell, a plant cell, or a mammalian cell, for example.

A transgenic multicellular host organism which has been genetically manipulated may be used to produce a polypeptide. The organism may be, for example, a transgenic mammalian organism (*e.g.,* a transgenic goat or mouse line).

Suitable prokaryotes include but are not limited to eubacteria, such as Gram-negative or Gram-positive organisms, for example, *Enterobacteriaceae* such as *E. coli.* Various E. coli strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include *Enterobacteriaceae* such as *Escherichia, e.g., E. coli, Enterobacter, Erwinia, Klebsiella, Proteus, Salmonella, e.g., Salmonella typhimurium, Serratia, e.g., Serratia marcescans,* and *Shigella,* as well as *Bacilli* such as *B. subtilis and B. licheniformis* (*e.g., B. licheniformis* 41P), *Pseudomonas* such as *P. aeruginosa,* and *Streptomyces.* These examples are illustrative rather than limiting. Strain W3110 is one particularly preferred host or parent host because it is a common host strain for recombinant polynucleotide product fermentations. Preferably, the host cell secretes minimal amounts of proteolytic enzymes. For example, strain W3110 may be modified to effect a genetic mutation in the genes encoding polypeptides endogenous to the host, with examples of such hosts including *E. coli* W3110 strain 1A2, which has the complete genotype *tonA; E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT kan'; E. coli* W3110 strain 37D6, which has the complete genotype *tonA ptr3 phoA E15 (argF-lac)169 degP ompT rbs7 ilvG kan'; E. coli* W3110 strain 40B4, which is strain 37D6 with a non-kanamycin resistant *degP* deletion mutation; and an *E. coli* strain having mutant periplasmic protease. Alternatively, in vitro methods of cloning, *e.g.,* PCR or other nucleic acid polymerase reactions, are suitable.

In these prokaryotic hosts, one can make expression vectors, which will typically contain expression control sequences compatible with the host cell (*e.g.,* an origin of replication). In addition, any number of a variety of well-known promoters will be present, such as the lactose promoter system, a tryptophan (trp) promoter system, a beta-lactamase promoter system, or a promoter system from phage lambda. The promoters will typically control expression, optionally with an operator sequence, and have ribosome binding site sequences and the like, for initiating and completing transcription and translation.

Eukaryotic microbes may be used for expression. Eukaryotic microbes such as filamentous fungi or yeast are suitable cloning or expression hosts for polypeptide-encoding vectors. *Saccharomyces cerevisiae* is a commonly used lower eukaryotic host microorganism. Others include *Schizosaccharomyces pombe; Kluyveromyces hosts* such as, *e.g., K. lactis* (MW98-8C, CBS683, CBS4574), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans,* and *K. marxianus; yarrowia* (EP 402,226); *Pichia pastoris; Candida; Trichoderma reesia; Neurospora crassa; Schwanniomyces* such as *Schwanniomyces occidentalis;* and filamentous fungi such as, *e.g., Neurospora, Penicillium, Tolypocladium,* and Aspergillus hosts such as *A. nidulans,* and *A. niger.* Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula. Saccharomyces* is a preferred yeast host, with suitable vectors having expression control sequences (*e.g.*, promoters), an origin of replication, termination sequences and the like as desired. Typical promoters include 3-phosphoglycerate kinase and other glycolytic enzymes. Inducible yeast promoters include, among others, promoters from alcohol dehydrogenase, isocytochrome C, and enzymes responsible for maltose and galactose utilization.

In addition to microorganisms, mammalian tissue cell culture may also be used to express and produce the polypeptides as described herein and in some instances are preferred (*See* Winnacker, From Genes to Clones VCH Publishers, N.Y., N.Y. (1987). For some embodiments, eukaryotic cells may be preferred, because a number of suitable host cell lines capable of secreting heterologous polypeptides (*e.g.*, intact immunoglobulins) have been developed in the art, and include CHO cell lines, various Cos cell lines, HeLa cells, preferably, myeloma cell lines, or transformed B-cells or hybridomas. In some embodiments, the mammalian host cell is a CHO cell.

In some embodiments, the host cell is a vertebrate host cell. Examples of useful mammalian host cell lines are monkey kidney CV1 line transformed by SV40 (COS-7, ATCC CRL 1651); human embryonic kidney line (293 or 293 cells subcloned for growth in suspension culture); baby hamster kidney cells (BHK, ATCC CCL 10); Chinese hamster ovary cells/- DHFR(CHO or CHO-DP-12 line); mouse sertoli cells; monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells; MRC 5 cells; FS4 cells; and a human hepatoma line (Hep G2).

## V. Formulations and Methods of Making of the Formulations

Provided herein are also formulations and methods of making the formulation comprising the polypeptides (*e.g.*, antibodies) purified by the methods described herein. For example, the purified polypeptide may be combined with a pharmaceutically acceptable carrier.

The polypeptide formulations in some embodiments may be prepared for storage by mixing a polypeptide having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution.

Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as TWEEN™, PLURONICS™ or polyethylene glycol (PEG).

In some embodiments, the polypeptide in the polypeptide formulation maintains functional activity.

The formulations to be used for *in vivo* administration must be sterile. This is readily accomplished by filtration through sterile filtration membranes.

The formulations herein may also contain more than one active compound as necessary for the particular indication being treated, preferably those with complementary activities that do not adversely affect each other. For example, in addition to a polypeptide, it may be desirable to include in the one formulation, an additional polypeptide (*e.g.*, antibody). Alternatively, or additionally, the composition may further comprise a chemotherapeutic agent, cytotoxic agent, cytokine, growth inhibitory agent, anti-hormonal agent, and/or cardioprotectant. Such molecules are suitably present in combination in amounts that are effective for the purpose intended.

## V. Articles of Manufacture

The polypeptides purified by the methods described herein and/or formulations comprising the polypeptides purified by the methods described herein may be contained within an article of manufacture. The article of manufacture may comprise a container containing the polypeptide and/or the polypeptide formulation. Preferably, the article of manufacture comprises:(a) a container comprising a composition comprising the polypeptide and/or the polypeptide formulation described herein within the container; and (b) a package insert with instructions for administering the formulation to a subject.

The article of manufacture comprises a container and a label or package insert on or associated with the container. Suitable containers include, for example, bottles, vials, syringes, etc. The containers may be formed from a variety of materials such as glass or plastic. The container holds or contains a formulation and may have a sterile access port (for example the container may be an intravenous solution bag or a vial having a stopper pierceable by a hypodermic injection needle). At least one active agent in the composition is the polypeptide. The label or package insert indicates that the composition's use in a subject with specific guidance regarding dosing amounts and intervals of polypeptide and any other drug being provided. The article of manufacture may further include other materials desirable from a commercial and user standpoint, including other buffers, diluents, filters, needles, and syringes. In some embodiments, the container is a syringe. In some embodiments, the syringe is further contained within an injection device. In some embodiments, the injection device is an autoinjector.

A "package insert" is used to refer to instructions customarily included in commercial packages of therapeutic products, that contain information about the indications, usage, dosage, administration, contraindications, other therapeutic products to be combined with the packaged product, and/or warnings concerning the use of such therapeutic products.

## *VI. Exemplary embodiments*

The invention provides methods for purifying a polypeptide from a composition comprising the polypeptide and one or more contaminants, said method comprising

> a) loading the composition onto a chromatography material in an amount in excess of the dynamic binding capacity of the chromatography material for the polypeptide using a loading buffer, wherein the partition coefficient of the chromatography material for the polypeptide is greater than 100,
> b) eluting the polypeptide from the chromatography material under conditions wherein the one or more contaminants remain bound to the chromatography material using an elution buffer, wherein the elution buffer has a conductivity less than the conductivity of the loading buffer, and wherein the elution buffer has a conductivity less than 4.0 mS/cm,
> c) pooling fractions comprising the polypeptide in the chromatography effluent from steps a) and b). contaminants remain bound to the chromatography material, and c) pooling fractions comprising the polypeptide in the chromatography effluent from steps a) and b).

In further embodiments of the above embodiment, polypeptide is an antibody or immunoadhesin.
In further embodiments of the above embodiment, the polypeptide is an immunoadhesin.
In further embodiments of the above embodiment, the polypeptide is an antibody.
In further embodiments of the above embodiment, the antibody is a monoclonal antibody.
In yet a further embodiment of the above embodiment, the monoclonal antibody is a chimeric antibody, humanized antibody, or human antibody.
In further embodiments of the above embodiment, the monoclonal antibody is an IgG monoclonal antibody.
In further embodiments of the above embodiment, the antibody is an antigen binding fragment.
In yet further embodiments of the above embodiment, the antigen binding fragment is selected from the group consisting of a Fab fragment, a Fab' fragment, a F(ab')2 fragment, a scFv, a di-scFv, a bi-scFv, a tandem (di, tri)-scFv, a Fv, a sdAb, a tri-functional antibody, a BiTE, a diabody and a triabody.
In further embodiments of the above embodiment, the polypeptide is selected from an enzyme, a hormone, a fusion protein, an Fc-containing protein, an immunoconjugate, a cytokine and an interleukin.
In further embodiments of the above embodiment, the at least one contaminant is any one or more of Chinese Hamster Ovary Protein (CHOP), a host cell protein (HCP), leached protein A, carboxypeptidase B, nucleic acid, DNA, product variants, aggregated protein, cell culture media component, gentamicin, polypeptide fragment, endotoxin and viral contaminant.
In further embodiments of the above embodiment, the chromatography material is selected from a mixed mode material, an anion exchange material, a cation exchange material, a hydrophobic interaction material, and an affinity material.
In further embodiments of the above embodiment, the loading density is between about 50 g/L to about 2000 g/L.
In yet further embodiments of the above embodiment; the loading density is between about 200 g/L to about 1000 g/L.
In further embodiments of the above embodiment, the composition is loaded onto the chromatography material at about the dynamic binding capacities of the chromatography materials for the one or more contaminants.
In further embodiments of the above embodiment, the composition is loaded on the chromatography material at 20-times the dynamic binding capacity of the chromatography material for the polypeptide.

The partition coefficient of the chromatography material for the polypeptide is greater than 100.

In further embodiments of the above embodiment, the method further comprises the use of a loading buffer and an elution buffer.

The elution buffer has a conductivity less than the conductivity of the loading buffer.

In further embodiments of the above embodiment, the loading buffer has a conductivity of about 4.0 mS to about 7.0 mS.

In further embodiments of the above embodiment, the conductivity of the elution buffer decreases in a gradient from about 5.5 mS to about 1.0 mS over about 10 column volumes (CVs).

In further embodiments of the above embodiment, the conductivity of the elution buffer decreases in a gradient from about 5.5 mS to about 1.0 mS over about 15 CVs.

In further embodiments of the above embodiment, the conductivity of the elution buffer decreases in a gradient from about 10.0 mS to about 1.0 mS over about 5 CVs.

In further embodiments of the above embodiment, the conductivity of the elution buffer decreases in a gradient from about 10.9 mS to about 1.0 mS over about 10 CVs.

In further embodiments of the above embodiment, the elution buffer has a pH less than the pH of the loading buffer.

In further embodiments of the above embodiment, the loading buffer has a pH of about 4 to about 9.

In further embodiments of the above embodiment, the elution buffer has a pH of about 4 to about 9.

In further embodiments of the above embodiment, the elution buffer has a pH greater than the pH of the loading buffer.

In further embodiments of the above embodiment, the load buffer has a pH of about 4 to about 9.

In further embodiments of the above embodiment, the elution buffer has a pH of about 4 to about 9.

In further embodiments of the above embodiment, the composition is an eluent from an affinity chromatography, a cation exchange chromatography, an anion exchange chromatography, a mixed mode chromatography and a hydrophobic interaction chromatography.

In further embodiments of the above embodiment, the affinity chromatography is a Protein A chromatography.

In further embodiments of the above embodiment, the polypeptide is further purified.

In yet further embodiments of the above embodiment, the polypeptide is further purified by virus filtration.

In yet further embodiments of the above embodiment, the polypeptide is further purified by one or more of an affinity chromatography, a cation exchange chromatography, an anion exchange chromatography, a mixed mode chromatography or a hydrophobic interaction chromatography.

In further embodiments of the above embodiment, the polypeptide is further concentrated.

In yet further embodiments of the above embodiment, the polypeptide is concentrated by ultrafiltration, diafilteration or a combination of ultrafiltration and diafiltration.

In further embodiments of the above embodiment, the methods further comprising combining the polypeptide with a pharmaceutically acceptable carrier.

Further details of the invention are illustrated by the following non-limiting Examples.

EXAMPLES

The examples below are intended to be purely exemplary of the invention and should therefore not be considered to limit the invention in any way. The following examples and detailed description are offered by way of illustration and not by way of limitation.

*Materials and Methods*

Materials and methods for all Examples were performed as indicated below unless otherwise noted in the Example.

*MAb feedstocks*

MAb feedstocks for all examples were selected from industrial, pilot or small scale cell culture batches at Genentech (South San Francisco, CA, U.S.A.). After a period of cell culture fermentation, the cells were separated and the clarified fluid was purified by Protein A chromatography. The Protein A pool was used to investigate the mechanism of impurity clearance. Table 2 shows feedstock characteristics for each mAb used in the examples.

Table2. Characteristics of MAb feedstocks.

| MAb | MAb indentification | pI |
|---|---|---|
| MAb 1 | Rituxan® | 8.8-9.3 |
| MAb 2 | anti-oxLDL | 9.3 |
| MAb 3 | anti-IFNα | 8.7 |
| MAb 4 | Xolair® | 7.6 |
| MAb 5 | anti-FGFR3 | 8.1 |

*MAb quantification*

The concentration of antibody was determined via absorbance at 280 and 320 nm using a UV-visible spectrophotometer (8453 model G1103A; Agilent Technologies; Santa Clara, CA, U.S.A.) or NanoDrop 1000 model ND-1000 (Thermo Fisher Scientific; Waltham, MA, U.S.A.). Species other than antibody (*i.e.* impurities) were too low in concentration to have an appreciable effect on UV absorbance. As needed, samples were diluted with an appropriate non-interfering diluent in the range of 0.1-1.0 absorbance unit. Sample preparation and UV measurements were performed in duplicate and the average value was recorded. The MAb absorption coefficients ranged from 1.42 to 1.645/mg·ml·cm.

*CHO host cell protein (CHOP) quantification*

An ELISA was used to quantify the levels of the host cell protein called CHOP. Anti-CHOP antibodies were immobilized on microtiter plate wells. Dilutions of the samples containing CHOP, standards and controls were incubated in the wells, followed by incubation with anti-CHOP antibodies conjugated with horseradish peroxidase (HRP). The HRP enzymatic activity was detected with o-phenylenediamine, and the CHOP was quantified by reading absorbance at 490 nm in a microtiter plate reader. Based on the principles of sandwich ELISA, the concentration of peroxidase corresponded to the CHOP concentration. The assay range for the ELISA was typically 5-320 ng/ml with intra-assay variability <10 %. CHOP values were reported in units of ng/ml. Alternatively, CHOP values were divided by the MAb concentration and the results were reported in PPM (parts per million; *e.g.* ng of CHOP/mg of MAb). The CHOP ELISA may be used to quantify total CHOP levels in a sample but does not quantify the concentration of individual proteins.

*Chromatography Operating Conditions*

Capto Adhere and Capto MMC Capto Adhere resins were obtained from GE Healthcare (Uppsala, Sweden). Strong cation exchange resins (Poros XS and Poros 50 HS) were obtained from Applied Biosystems (Address). All laboratory chromatographic experiments were carried out using an AKTA FPLC chromatographic system from GE Healthcare (Uppsala, Sweden) utilizing UNICORN software. Laboratory columns were 0.66 cm in diameter and 10-20 cm in height. The columns were equilibrated to the specified operating conditions of pH and conductivity prior to loading. Protein A pools were then loaded on to the column followed by the elution buffer as required to elute off the bound protein. The flowthrough or eluate during the load, overload and elution phases were collected as fractions and then analyzed for impurities. MAb load density varied from 100 to 1000 g per liter of resin.

*Chromatography Pool Analysis*

The flow-through pools during the load, overload and the elution phases were collected in fractions (1 column volume each) and analyzed for MAb concentration, CHOP concentration, aggregates, Leached Protein A, CHO DNA and yield. Cumulative plots were generated as a function of the elution pool fractions. Cumulative yield was obtained using Equation 1.
where, for fraction i, Ci is the Mab concentration (mg/ml), Vi is the volume of the fraction (ml), Mp is the mass of protein loaded (mg).

$$\text{Cumulative \% Yield} = \frac{\sum_{i=1}^{y} C_i * V_i}{M_p} * 100 \qquad \text{Equation 1}$$

*Size-Exclusion Chromatography*

The size heterogeneity of the monoclonal antibodies was determined by a high-performance size exclusion chromatography assay. A TSK G3000SWXL SEC column (diameter=7.8 mm, height=300 mm; part number 08541) manufactured by Tosoh Bioscience (Tokyo, Japan) was operated at ambient temperature on a 1200 series HPLC instrument (Agilent Technologies) and used to determine the relative levels of MAb monomer for the collected samples. The column was operated at a flow rate of 0.3 mL/min using a 200 mM potassium phosphate, 250 mM potassium chloride pH 6.2 mobile phase. 20 $\mu$g of antibody was injected for each sample. UV absorbance at 280 nm was used to monitor the separation of monomer, LMW proteins and HMW proteins. Percentages of monomer, LMW proteins and HMW proteins were analyzed manually using ChemStation software (Agilent Technologies).

*CHO DNA Quantification*

CHO DNA in product samples was quantified using real-time PCR (TaqMan PCR). DNA from samples and controls were first extracted using Qiagen's Virus Biorobot kit. The extracted samples, controls, and standard DNA, were subject to TaqMan real time Polymerase chain reaction (PCR) using PCR primers and probe in a 96-well plate with ABI's sequence detection system. The primers were defined by a 110 base pair segment of a repetitive DNA sequence in the *Cricetulus griseus* genome. The probe was labeled with a fluorescent reporter dye at 5' end and a quencher dye at the 3' end. When the probe is intact, the emission spectrum of the reporter is suppressed by the quencher. The 5' nuclease activity of polymerase hydrolyzes the probe and releases the report, which results in an increase in fluorescence emission. The sequence detector quantified the amplified product in direct proportion to the increase in fluorescence emission measured continuously during the DNA amplification. Cycle numbers at which DNA had amplified past the threshold (CT) were calculated for the standard curve. A standard curve ranging 1 pg/mL-10,000 pg/mL was generated, which was used for quantifying DNA in samples.

*Leached Protein A quantification*

The level of leached Protein-A in the Protein A pools was determined by a sandwich Protein-A ELISA. Chicken anti-staphylococcal protein A antibodies were immobilized on microtiter plate wells. The sample treatment procedure included sample dilution and then dissociation of the Protein A/IgG complex using microwave assisted heating as a pretreatment step before running the samples on a sandwich ELISA. Protein A, if present in the sample, bound to the coated antibody. Bound protein A was detected using horseradish peroxidase conjugated anti-protein antibodies. Horseradish peroxidase enzymatic activity was quantified with a 2 component TMB substrate solution which produces a colorimetric signal.

Feedstock Conditioning

Feedstocks used for experiments in this study were either fresh or were removed from cold storage (2-8 °C or -70 °C) and allowed to equilibrate to room temperature. Subsequently, they were pH and/or conductivity adjusted as necessary using a titrating agent (1.5 M Tris base or 1 M Acetic acid) or diluent (purified water, 5 M sodium chloride, or 5 M sodium acetate). All feedstocks were 0.2 $\mu$m filtered using a Millipak 20 (Millipore), AcroPakTM 20 (Pall Corporation) or a vacuum filter (Thermo Fisher Scientific, Rochester, NY, U.S.A.).

*High Throughput Screening*

A Tecan Freedom Evo 200 robot (Tecan US, Research Triangle Park, NC) was used for liquid and resin handling. A 96-well filter plate (Seahorse 800 $\mu$L polypropylene 0.45$\mu$m short drip filter plates, E&K Scientific EK-2223) was used to incubate resin with the protein and buffer. After incubating the protein solution with the resin, the filter plate was centrifuged at 1200 x g for 3 minutes to separate the solution from the resin. For each stage, 300 mL of solution was contacted with 50 mL of resin, resulting in a phase volume ratio of 6:1. Each well was equilibrated to the appropriate pH and sodium acetate concentration. The pH ranged from 5.00 to 7.5 at 0.5 pH unit intervals (acetate was used to buffer the pH 5.00 to 5.5 conditions, MES was used to buffer the pH 6.00 to 6.5 conditions, and MOPS was used to buffer the 7.00 to 7.5 conditions). All experiments were performed at room temperature. Partially purified Protein A pool, concentrated to 5 g/L or 97 mg/mL, and buffer exchanged into 15 mM NaOAc was used as a load for these experiments. Resin was challenged to 5 g/L for the experiments to determine product $K_p$ values. However, for the actual product binding capacity the resin was challenged to 97 g/L. The filtrate solution was captured in a collection plate and then analyzed using the Infinite M200 plate reader. The bound protein was subsequently stripped from the resin using two stages of a 2 M NaCl buffer to close the mass balance.

*Virus Clearance Studies*

The objective of this study was to evaluate virus removal capability of the Capto Adhere for the 2 model viruses (MMV and X-MuLV). Columns, 0.66 cm diameter, were packed with naïve resins to a 20 cm bed height. The MAb feed was spiked with 1% virus and then processed over the Capto Adhere resin. The pools were collected immediately and load and elution samples were assayed for viral counts. Multiple dilutions of pools with Complete Medium (1:10 and 1:100) were made to determine any potential interference between the buffer components and the viruses.

**Example 1. High throughput screening**

This example describes high throughput screening methods to determine binding capacities of chromatography material. High throughput screening was performed on Capto Adhere resin under batch binding conditions for monoclonal antibody MAb3.

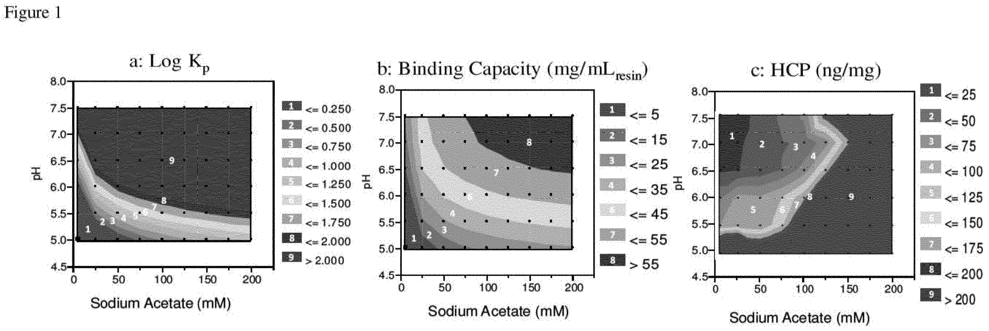

The results of high throughput screening of binding conditions are presented in Figure 1. In the response surface figures (Figures 1A and 1B), product-binding regions are indicated by red (region 8 of Figure 1A and region 7 in Figure 1B) and the product, e.g. polypeptide, nonbinding regions are green (indicated as region 1). The actual host cell protein (HCP) contents (ng/mg) in the supernatant are shown in the Figure 1C contour plot. Product $K_p$ as a function of pH and counterion concentration for MAb3 is shown in Figure 1A. The resin was loaded to 5 g/L and the raw data was analyzed using a response surface model. The model was then used to estimate the $K_p$ for any combination of pH and counterion concentration in the experimental space. Data shows that as the pH increases and as the conductivity increases, the log $K_p$ increases. Increases in log $K_p$ reflect increases in the product binding to the resin. In order to find out the actual product binding capacity on the resin, the resin was challenged to 80 g/L at 48 different conditions combining different pHs and counterion concentrations (Figure 1B). The supernatant from the same experimental plate was also analyzed for HCP (ng/mg) and the data was plotted in the form of contour (Figure1C). Partially purified Protein A pool containing several thousand ppm of CHOP was used as a load for the high throughput screening experiments. Green regions (region 1) in the figure indicate the lower amount of CHOP in the supernatant and the red regions (region 8 of Figure 1B and region 9 of Figure 1C) indicate higher amount of CHOP in the supernatant.

Design space for optimal loading and elution conditions can be determined from the CHOP and the binding data contour plots. These plots enable the design of either a flow through mode of operation or a bind and elute mode of operation. Loading conditions for a complete Flow Through chromatography are generally conditions where product binding minimized, e.g. polypeptide binding, and the impurity (CHOP) binding is maximized. Complete overlapping green regions (region 1) between Figures 1B and 1C suggest that a F/T mode is possible. However, the green region (region 1) in this plot is a region where the conductivity is really low ~1mS. Such conditions require about 4-fold dilution of the Protein A pool resulting in potential plant fit challenges at scale.

The red regions (region 7 in Figure 1B and region 8 in Figure 1C) indicate binding regions for both MAb and CHOP. There are some overlapping red regions in both the plots. However, FigureIB shows that, within the operable pHs and counterion concentrations, a maximum binding capacity of 55 g/L was possible. For a high titer process with a cell culture titer of ~3.5 g/L, a 1000 L column would be needed to recover all the product, such as a polypeptide, in one cycle or multiple cycles would have to be performed on a smaller column.

In an OEC mode, a loading condition can be chosen based on the impurity (e.g. CHOP) behavior on the column. The amount of MAb that binds to the column is less of a concern because the bound MAb can be recovered by the elution phase. Thus there is an entire experimental space to design the loading condition so as to get maximum impurity clearance without being limited by the resin's product binding capacity. While bind and elute mode allows 55 g/L load density, OEC enables about a10-fold higher loading capacity thereby allowing implementation of a smaller column which in turn can reduce resin cost per gram of product and offers a good plant fit.

**Example 2. Optimized OEC mode**

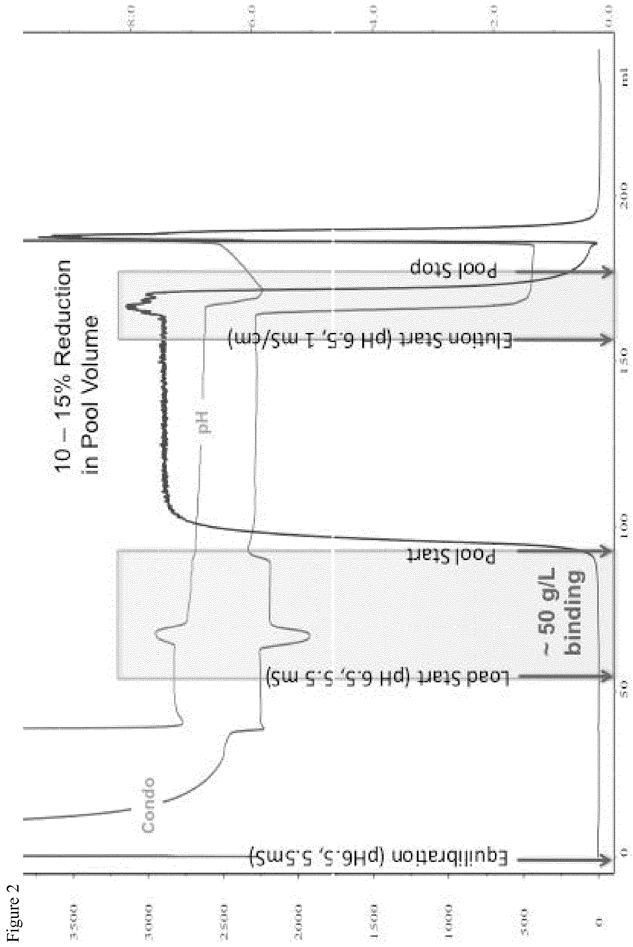

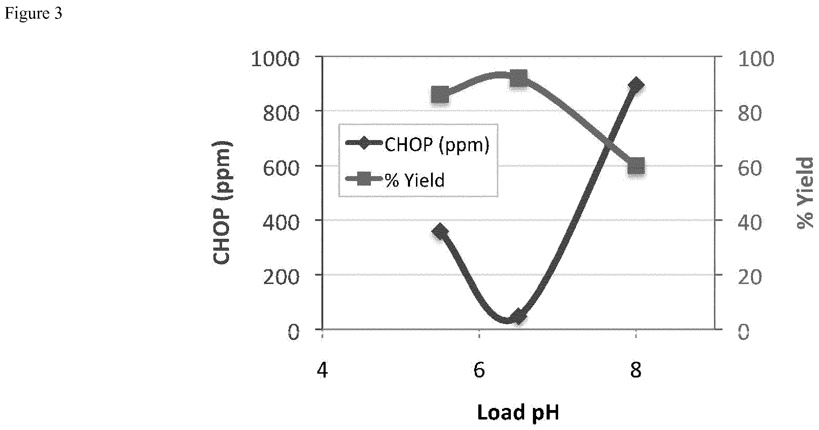

HTS data was used for parameter determination to operate in an OEC mode. Based on load density requirements, plant fit and impurity clearance, the load conditions for MAb 3 were selected to be pH 6.5 and 5.5 mS/cm. The load density for the optimized chromatography run shown in Figure 2 was 180 g/L. About 50 g/L polypeptide product binding to the resin during the load phase. The product pool was collected starting at about 0.5 OD, and the product was overloaded on the resin up to 180 g/L. After completion of the load phase, elution phase with low conductivity buffer of 1 mS/cm (elution buffer: 20 mM MES pH 6.5) was used to elute the bound protein resulting in 10-15% pool volume reduction compared to flow-through chromatography. Yield and impurity clearance for this chromatography run is shown in Figure 3.

**Example 3. Load Optimization**

This study was conducted to compare the HTS data and actual column performance data in the OEC mode of operation. Columns were loaded at three different pH's. pH 6.5 was selected to be the best condition based on the initial CHOP clearance and the yield data. Concentrations of CHOP in the pools ranged from 900 to 50 to 400 ppm as a function of load pH. From this study, pH 6.5 was determined to be the best condition to load the composition. In addition, although the yield wasn't optimized, pH 6.5 also provided the maximum yield (Figure 3, Table 3).

Table 3. Load condition optimization for an OEC mode

| Load | | Elution | | Pool CHOP (ppm) | MAb Bound (g/L) | Yield (%) |
|---|---|---|---|---|---|---|
| pH | Conductivity (mS/cm) | pH | Conductivity (mS/cm) | | | |
| 8 | 5.4 | 8 | 2.7 | 895 | 63 | 60 |
| 6.5 | 5.6 | 6.5 | 2.1 | 48 | 49 | 92 |
| 5.5 | 5.3 | 5.5 | 3.3 | 359 | 29 | 86 |
| Load CHOP: 20000 ppm Load Density: 150 g/L | | | | | | |

**Example 4. Elution optimization**

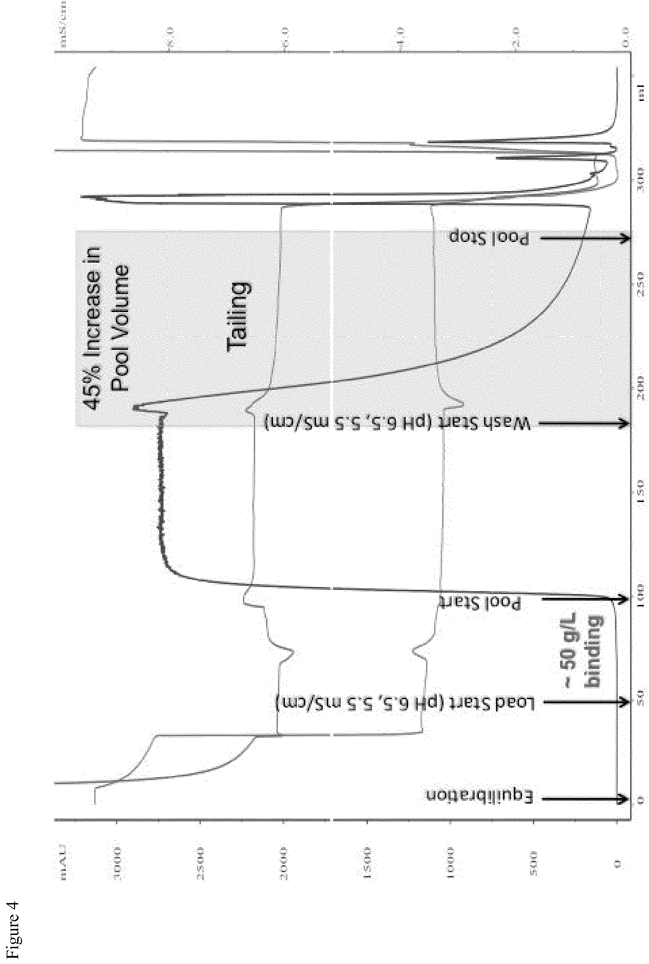

This study was conducted to optimize the elution conditions for OEC. In order to recover the product (MAb 3, ~50 g/L) that was bound during the load phase, a few column volumes of wash buffer (50 mM MES, 30 mM NaAcetate, pH 6.5, ~5.5 mS/cm) were passed through the column after completion of the load phase. A large amount of tailing was observed resulting in an increase in pool volume at the end of load phase. The increase in pool volume was about 45% using a wash buffer with a similar pH and conductivity as the load buffer. This increase in pool volume may result in plant fit challenges (Figure 4).

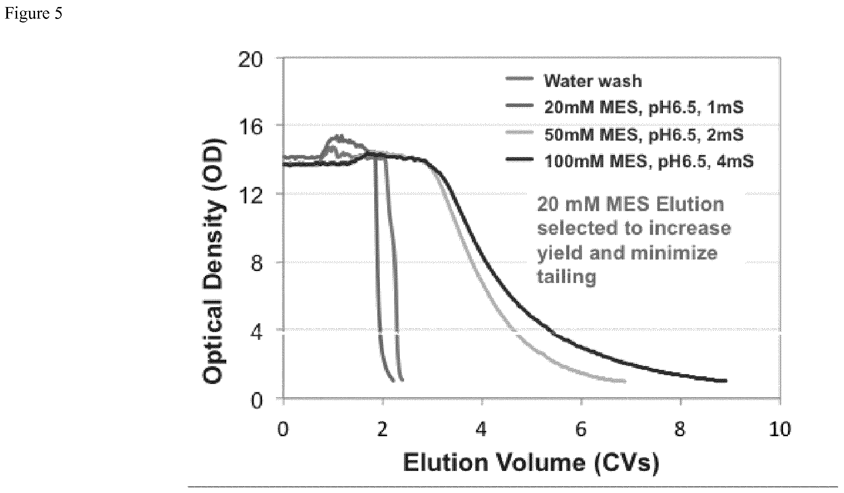

The objective of elution optimization study was to obtain maximum yield with minimum CHOP and maximum pool volume reduction. The elution phase was developed for eluting the 50 g/L of bound product from the column. Figure 5 shows the elution phase of the chromatograms. Lower conductivity buffer elutes the product off the column within 2 column volumes resulting in higher yield and lower pool volume (Table 4). Higher conductivity elution buffers, on the other hand, resulted in tailing of greater than 8 column volumes. As shown in Table 4, pool CHOP was less than 20 ppm in all the elution buffers tried. Therefore 20 mM MES was selected as elution buffer to increase yield and to minimize tailing.

Table 4. Elution optimization for OEC

| Elution Buffer | CHOP (ppm) | % Yield |
|---|---|---|
| 100 mM MES, pH 6.5, 4 mS | 13 | 91 |
| 100 mM MES, pH 6.5, 2 mS | 11 | 93 |
| 100 mM MES, pH 6.5, 1 mS | 17 | 94 |
| Water | 15 | 90 |
| Load CHOP: 3200 ppm Load conditions: pH 6.5, 5.5 mS/cm Load density: 180 g/L | | |

**Example 5. Impurity Analysis on OEC**

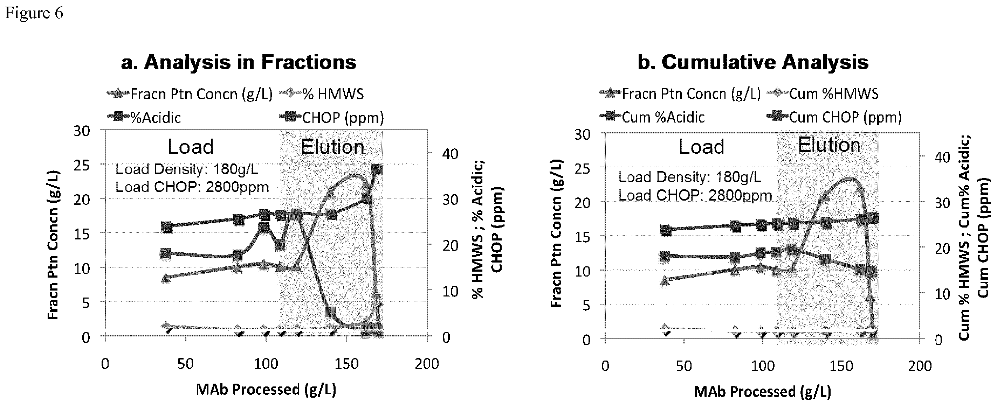

Fractions from OEC were analyzed for impurities. Figure 6A shows the MAb concentrations and CHOP levels in fractions collected during load phase and during elution phase. During the load phase, CHOP remained between 20-25 ppm and during the elution phase where only the bound MAb is being eluted, the fractional CHOP level decreases. Cumulative analysis demonstrates that the CHOP and other impurities remain fairly consistent across the entire load and the elution phases (Figure 6B). The load density for this run was 180 g/L. Virus clearance, using X-MuLV and MMV as model viruses, was also studied for the same load density (Table 5). For this experiment, load pH was 6.5 and load conductivity was 5.5 mS/cm. Elution conditions were 20 mM MES pH 6.5 and conductivity of ~1 mS/cm.

Table 5. Virus clearance for OEC mode chromatography.

| Capto Adhere resin load density | X-MuLV LRV | MMV LRV |
|---|---|---|
| 180 g/L | 3.6 | 3.3 |

### Example 6. Maximum Impurity Binding Capacity Determination

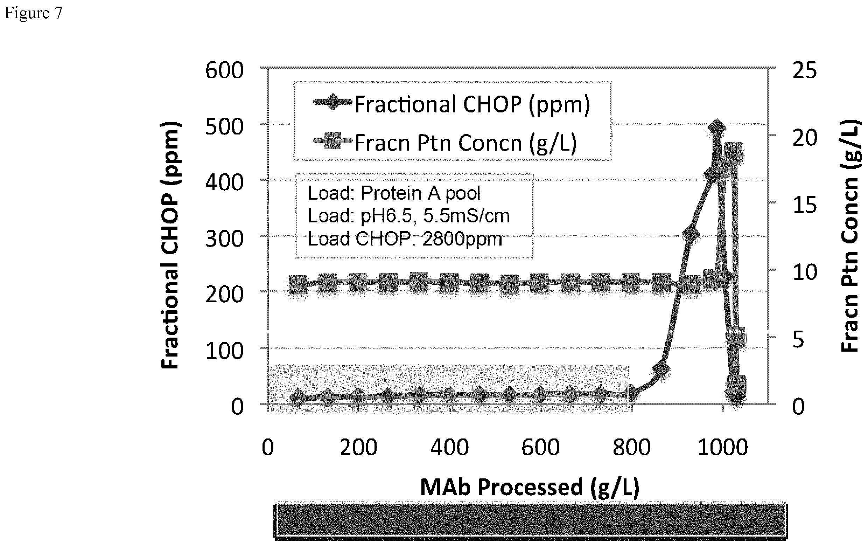

To find the maximum impurity binding capacity on the resin loaded with MAb3 by OEC mode, the Capto Adhere resin was challenged to 1000 g/L with Protein A pool. Loading buffer was pH 6.5, ~5.5 mS/cm; elution buffer was 20 mM MES pH 6.5, ~1 mS/cm. Pool sample was collected every 50 g/L and analyzed for CHOP and protein concentration (Figure 7). For loading densities of up to 800 g/L, CHOP did not break through and the CHOP in the eluate was less than 20 ng/mg.

### Example 7. Implementation at Pilot Scale

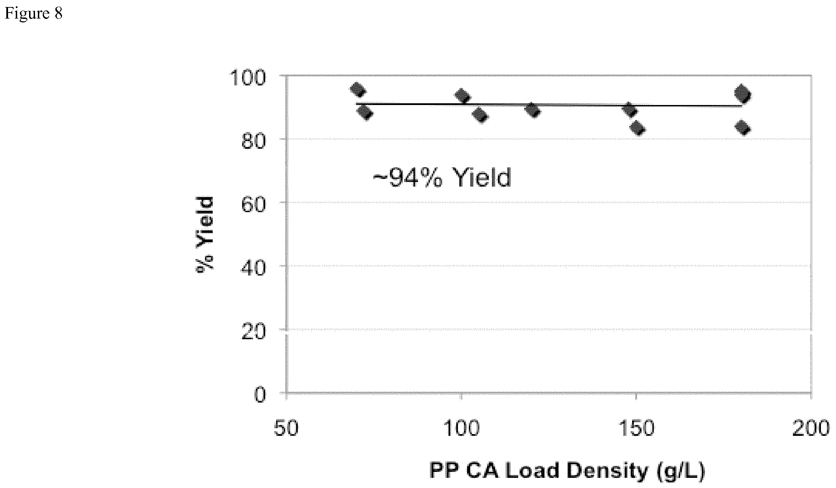

The OEC mode of operation was implemented on pilot scale columns ranging in size from 1.6 L to 10.8 L. The column diameters ranged from 10 cm to 25 cm with bed heights ranging from 20 - 22 cm (Table 6). Samples were loaded at pH 6.5, ~5.5 mS/cm and eluted with 20 mM MES pH 6.5, ~ 1 mS/cm. Yield across the pilot scale runs are shown in Figure 8. The load densities in the pilot scale runs covered a range of load densities anywhere from 70 to 180 g/L. Across all the load densities, an average yield of 94% was achieved. There was no impact on yield over the range of load densities tested. Across all pilot scale runs, the OEC mode pool CHOP was less than 25 ppm which was then cleared downstream to <2 ppm CHOP in the final polypeptide product (UFDF pool) (Table 7). On average, there was about 1.1% reduction in HMW proteins across all the pilot scale runs (Table 8) and 0.19% reduction in LMW proteins across all the pilot scale runs (Table 9).

Table 6. Capto Adhere column size for pilot scale runs

| Pilot Scale Run | BH | Diameter (cm) | CV (L) |
|---|---|---|---|
| Run 1 | 21 | 10 | 1.6 |
| Run 2 | 21 | 10 | 1.6 |
| Run 3 | 20 | 14 | 3.1 |
| Run 4 | 19 | 14 | 2.9 |
| Run 5 | 19 | 14 | 2.9 |
| Run 6 | 22 | 25 | 10.8 |
| Run 7 | 22 | 25 | 10.8 |
| Run 8 | 21.5 | 10 | 1.7 |
| Run 9 | 20 | 14 | 3.1 |
| Run 10 | 22 | 25 | 10.8 |

Table 7. Pilot Scale CHOP data

| Steps | Run 1 | Run 2 | Run 3 | Run 4 | Run 5 | Run 6 | Run 7 | Run 8 | Run 9 | Run 10 | Run 11 | Run 12 |
|---|---|---|---|---|---|---|---|---|---|---|---|---|
| HCCF | 467000 | 446000 | 242000 | 343000 | 223000 | 310000 | 424000 | 355000 | 218000 | 329000 | 29700 | 21500 |
| Protein A Pool | 11360 | 2200* | 5712 | 9100 | 5100 | 4800 | 5400 | 6200 | 9000 | 7300 | 5300 | 4800 |
| Capto Adhere Resin (OEC) | 13 | 14 | 21 | 11 | 22 | 17 | 17 | 13 | 15 | 25 | 17 | 9 |

* CHOP after depth filtration at pH 6.5
HCCF is harvested cell culture fluid

Table 8. Average % HMW proteins reduction across 12 Pilot Scale runs.

| Avg % HMW protein Reduction | 0.95 ± 0.49 |
|---|---|

Table 9. Average % LMW proteins reduction across 12 Pilot Scale runs.

| Avg % LMW protein Reduction | 0.19 ± 0.08 |
|---|---|

CHO DNA and leached Protein A were less than detectable in the pool after OEC.

## Example 8. Implementation at Manufacturing Scale

OEC mode of operation was implemented on a manufacturing scale column with a size of 157 L (100 cm diameter × 20 cm height). At manufacturing scale, the column was loaded to ~96 g/L. % yield across the two runs at manufacturing scale was ≥95% for both runs. Across all the manufacturing scale runs, the OEC mode pool CHOP was less than 17 ppm (Table 10) which was cleared downstream to less than the detectable level of CHOP. In an average, there was about 1.1% reduction in HMWs, 0.1% reduction in LMWs, and 1.65% reduction in acidics across the two manufacturing scale runs.

Table 10. Manufacturing Scale CHOP (ppm) data

| Steps | Run 1 | Run 2 |
|---|---|---|
| HCCE | 30500 | 32500 |
| Protein A Pool | 5749 | 6157 |
| Capto Adhere Resin (OEC) | 16 | 17 |

CHO DNA and leached Protein A were less than detectable in the pool after OEC mode.

## Example 9. OEC Applicability to outher MAbs

Protein A pools were used as loads for these runs. Load and elution conditions were selected so as to enable OEC mode. Load densities, % yield, MAb bound to the resin (g/L), impurities in the load and pool are shown in the following tables (Table 11, 12, 13 and 14).

Table 11. OEC applicability to other MAbs: CHOP

| MAb, Load pH (conductivity <6mS/cm) | Load Density (g/L) | Elution pH and conductivity | MAb Bound (g/L) | Load CHOP (ppm) | Pool CHOP (ppm) | % Yield |
|---|---|---|---|---|---|---|
| MAb 1, pH 8.6 | 200 | pH 6.5, 1 mS/cm | 35 | 2825 | 9 | 93 |
| MAb 2, pH 8.6 | 200 | pH 6.5, 1 mS/cm | 24 | 5057 | 12 | 100 |
| MAb 3, pH 6.5 | 200 | pH 6.5, 1 mS/cm | 45 | 3200 | 15 | 97 |
| MAb 4, pH 6.1 | 200 | pH 6.0, 0.65 mS/cm | 59 | 193 | 4 | 93 |
| MAb 5, pH 5.5 | 200 | pH 4.9, 1.1 mS/cm | 59 | 4560 | 145 | 92 |

Table 12. OEC applicability to other MAbs: % HMW proteins

| MAb, Load pH (conductivity < 6 mS/cm) | Load Density (g/L) | Elution pH and conductivity | MAb Bound (g/L) | Load % HMW proteins | Pool % HMW proteins |
|---|---|---|---|---|---|
| MAb 1, pH 8.6 | 200 | pH 6.5, 1 mS/cm | 35 | 5.4 | 1.6 |
| MAb 2, pH 8.6 | 200 | pH 6.5, 1 mS/cm | 24 | 5.4 | 3.8 |
| MAb 3, pH 6.5 | 200 | pH 6.5, 1 mS/cm | 45 | 3.5 | 1.8 |
| MAb 4, pH 6.1 | 200 | pH 6.0, 0.65 mS/cm | 59 | 1.9 | 0.5 |

(continued)

| MAb, Load pH (conductivity < 6 mS/cm) | Load Density (g/L) | Elution pH and conductivity | MAb Bound (g/L) | Load % HMW proteins | Pool % HMW proteins |
|---|---|---|---|---|---|
| MAb 5, pH 5.5 | 200 | pH 4.9, 1.1 mS/cm | 59 | 2.4 | 1.7 |

Table 13. OEC applicability to other MAbs: % leached protein A

| MAb, Load pH (conductivity <6mS/cm) | Load Density (g/L) | Elution pH and conductivity | MAb Bound (g/L) | Load Leached Protein A (ppm) | Pool Leached Protein A (ppm) |
|---|---|---|---|---|---|
| MAb 1, pH 8.6 | 200 | pH 6.5, 1 mS/cm | 35 | 2 | LTD* |
| MAb 2, pH 8.6 | 200 | pH 6.5, 1 mS/cm | 24 | 2 | LTD |
| MAb 3, pH 6.5 | 200 | pH 6.5, 1 mS/cm | 45 | 3 | LTD |
| MAb 4, pH 6.1 | 200 | pH 6.0, 0.65 mS/cm | 59 | 2 | LTD |
| MAb 5, pH 5.5 | 200 | pH 4.9, 1.1 mS/cm | 59 | 6 | LTD |
| *Less than detectable | | | | | |

Table 14. OEC applicability to other MAbs: CHO DNA

| MAb, Load pH (conductivity <6 mS/cm) | Load Density (g/L) | Elution pH and conductivity | MAb Bound (g/L) | Load CHO DNA (pg/mL) | Pool CHO DNA (pg/mL) |
|---|---|---|---|---|---|
| MAb 1, pH 8.6 | 200 | pH 6.5, 1 mS/cm | 35 | 253 | LTD |
| MAb 2, pH 8.6 | 200 | pH 6.5, 1 mS/cm | 24 | 297 | LTD |
| MAb 3, pH 6.5 | 200 | pH 6.5, 1 mS/cm | 45 | 106 | LTD |
| MAb 4, pH 6.1 | 200 | pH 6.0, 0.65 mS/cm | 59 | 17 | LTD |
| MAb 5, pH 5.5 | 200 | pH 4.9, 1.1 mS/cm | 59 | 4 | LTD |

## Example 10. Modes of AEX chromatography operations based on $K_p$ values

In complete flow through (F/T) chromatography, $K_p$ is <0.1 and there is no protein binding to the resin. In weak partitioning chromatography (WPC), $K_p$ is 0.1 to 20 and there is weak partitioning between the product and the chromatography media. In a bind and elute mode, product is tightly bound to the resin, and the $K_p$ is >100 but the load density is limited to the product binding capacity. However, in an overload and elute mode of chromatography (MAb 3), load conditions were found such that the product and the impurities $K_p$ were >100 and although the product flows through after reaching its binding capacity, the impurities keep binding to the resin and does not break through until they reach their binding capacity, which could be higher than the product binding capacity.

Elution conditions were found such that the polypeptide product $K_p$ < 2. The bound product is recovered but majority of the impurities remain bound (impurity $K_p$ > 100). As such, this mode enables good impurity clearance while providing very high yield (*e.g.* ~95%) (Table 15).

Table 15. MAb 3 as a model antibody on Capto Adhere resin

| Parameters | WPC | OEC |
|---|---|---|
| Load | Protein A Pool (MAb 3) | |
| Load Conditions | pH 5, 4.4 mS/cm | pH 6.5, 5.5 mS/cm |
| Kp | 2.6 | >100 |
| Log Kp | 0.4 | 4.15 |
| Load CHOP | 20000 ppm | |

(continued)

| Parameters | WPC | OEC |
|---|---|---|
| Product Binding (g/L resin) | 20 | 50 |
| Pool CHOP (ppm) | 360 | 50 |
| % Yield | 86 | 95 |

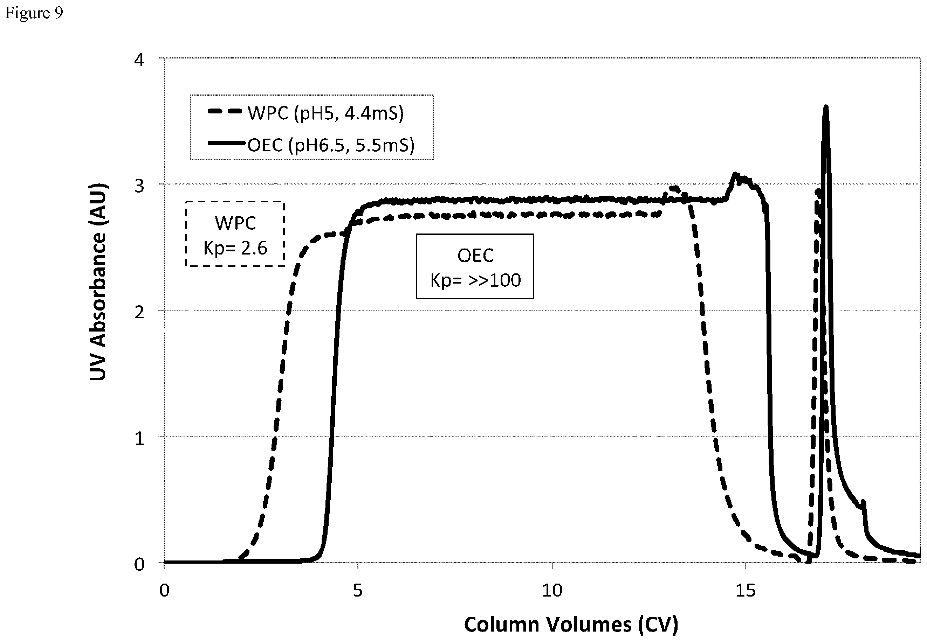

Chromatograms from column runs under weak partitioning conditions ($K_p$ = 2.4) and overload and elute conditions ($K_p$ >100) display the effects of increasing mAb $K_p$ on the product breakthrough regions of the chromatogram (Figure 9). WPC load conditions: pH 5.5, 4.4 mS/cm; WPC wash conditions: 20 mM Acetate pH 5, 4.4 mS/cm. OEC load conditions: pH 6.5, 5.5 mS/cm; OEC elution conditions: 20 mM MES pH 6.5, ~1 mS/cm.

Table 16. Analysis of an AEX polishing step for a MAb that binds to AEX resin at typical flow through process conditions

| Mode of Operation | F/T | B/E | OEC |
|---|---|---|---|
| Load Density (g/L$_{resin}$) | ~200 | 50 | 800 |
| Column Size* | 220 L | 850 L | 52 L |
| Pool Volume | 11,000 L | ~4CV Elution (3400L) | ~2500 L |
| Plant Fit (pool Tank limitation) | Very large pool volume due to ~5X load dilution | Large column or multiple cycles | Better plant fit. Reduces pool volume by ~ 10 - 40% |
| Cost of Resin** | $ 0.7 Million | $2.98 Million | $ 0.18 Million |
| Model antibody: MAb 3<br>* Assume 12,500 L harvest at 3.5 g/L titer.<br>** CA resin at $ 3500/L | | | |

## Example 11. OEC applicability to different resins

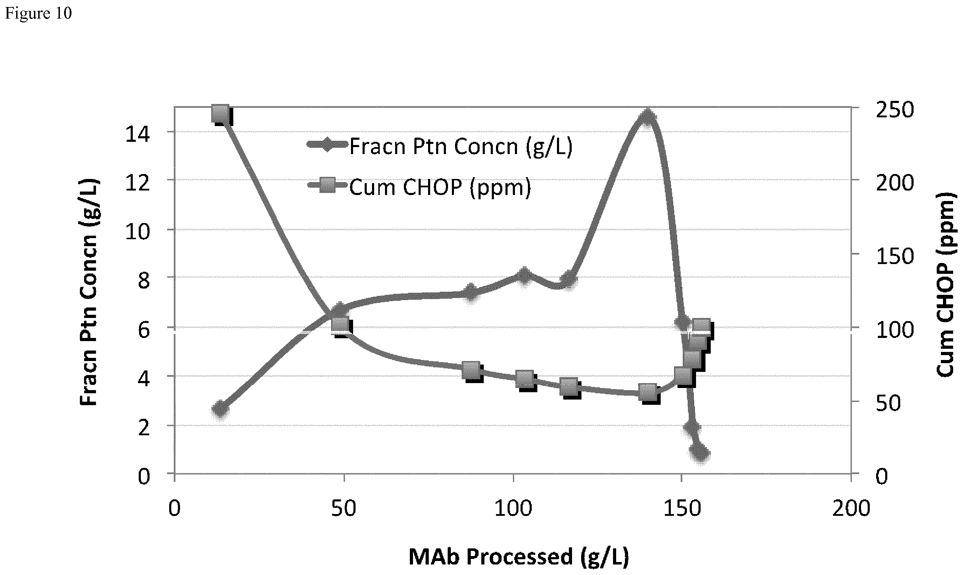

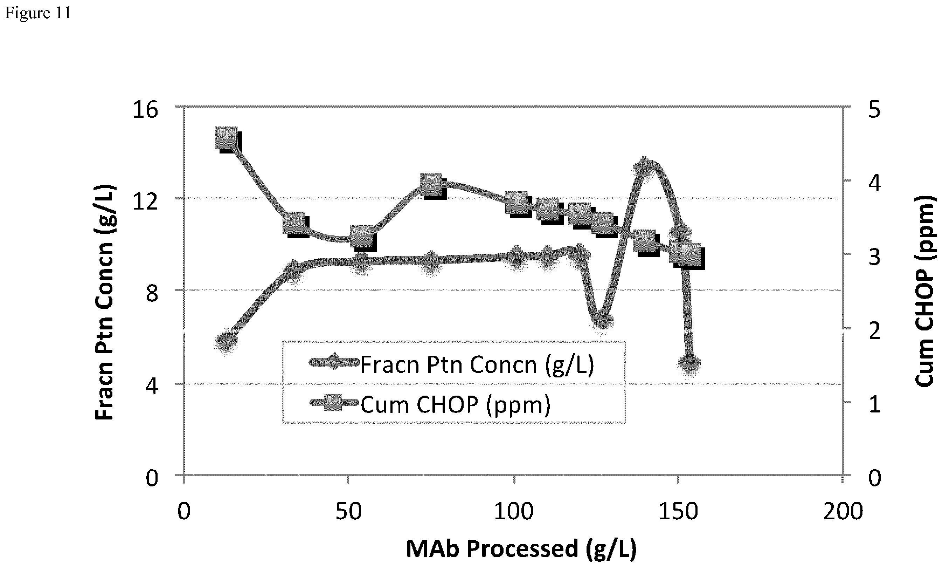

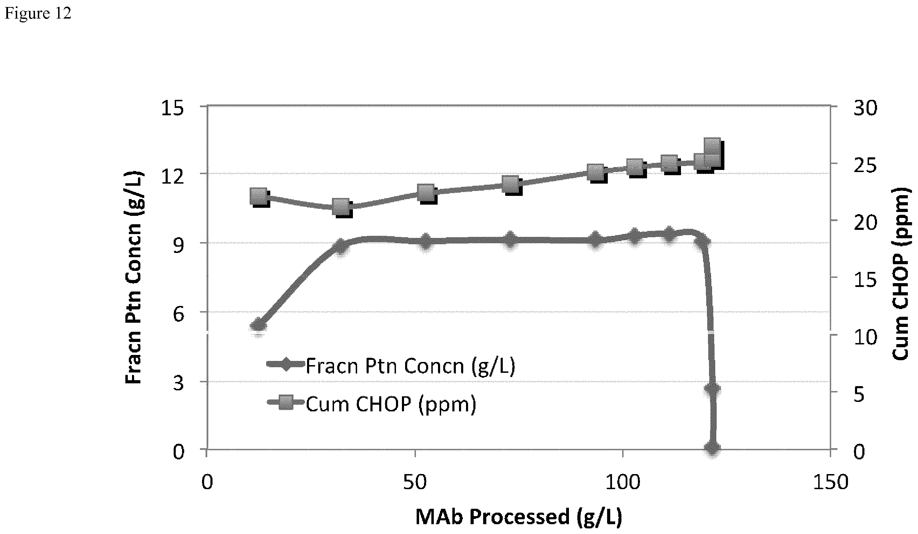

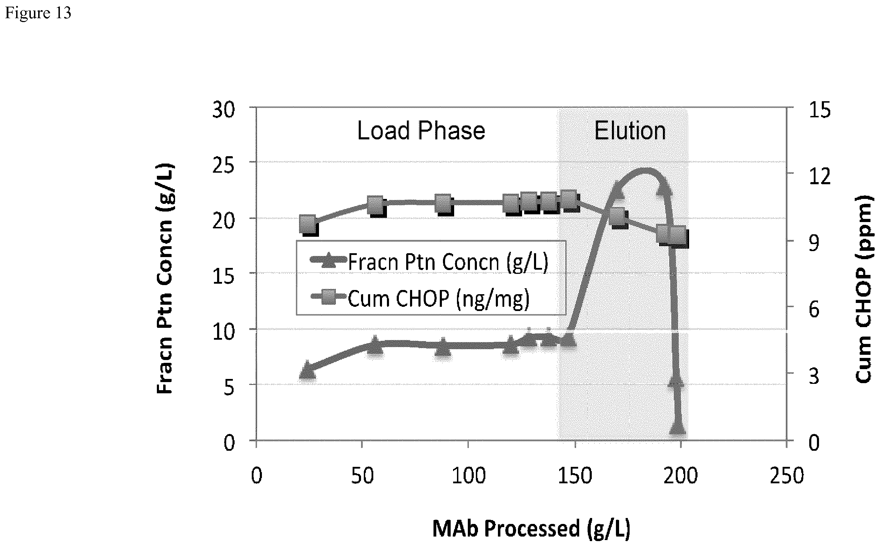

Depending on the pI of the molecule, OEC can be applied to the following Multi mode Resins (Capto Adhere, QMA, MEP Hypercel, HEA Hypercel, PPA Hypercel, Capto MMC). Polypeptide product binding on the Capto Adhere resin was more hydrophobic in nature and the bound product could be eluted from the column by lowering the conductivity of the elution buffer (Figure 5). However, the mode of product binding on the resin and product elution from the resin is not be limited to hydrophobic interactions and thus OEC mode of operation can be widely used in other chromatography materials as well. Table 17 demonstrates that OEC can be applied to other CEX Resins and IEX resins. Elution buffers were 20 mM MES unless otherwise indicted. The potential of OEC is not limited to the above resins and is currently being evaluated. Breakthrough analysis of MAb 3 on QMA resin is shown in Figure 10. Breakthrough analysis of MAb 4 on Capto Adhere resin is shown in Figure 11. Breakthrough analysis of MAb 4 on Capto MMC resin is shown in Figure 12. Breakthrough analysis of MAb 3 on Capto Adhere resin is shown in Figure 13.

Table 17. OEC applicability to different resins.

| MAb | Resin | Load | | Elution | | Load Density (g/L) | MAb Bound (g/L) | Load CHOP (ppm) | Pool CHOP (ppm) | % Yield |
|---|---|---|---|---|---|---|---|---|---|---|
| | | pH | Condo (mS/cm) | pH | Condo (mS/cm) | | | | | |
| MAb 3 | Capto Adhere | 6.5 | <5.5 | 6.5 | 1 | 200 | 50 | 3200 | 15 | 97 |
| | QMA | 6.5 | <5.5 | 6.5 | 1 | 103 | 17 | 1800 | 99 | 93 |
| MAb 3* | Poros XS | 5.5 | <6 | 5.5 | ** | 200 | 100 | 9900 | 370 | 92 |

(continued)

| MAb | Resin | Load | | Elution | | Load Density (g/L) | MAb Bound (g/L) | Load CHOP (ppm) | Pool CHOP (ppm) | % Yield |
|---|---|---|---|---|---|---|---|---|---|---|
| | | pH | Condo (mS/cm) | pH | Condo (mS/cm) | | | | | |
| MAb 4 | Capto Adhere | 6.1 | <5.5 | 6.0 | 0.65 | 200 | 59 | 193 | 4 | 93 |
| | Capto MMC | 7 | <6 | 6.5 | 1 | 147 | 10 | 187 | 27 | 93 |

**Poros XS MAb 3 load conditions: pH 5.5, < 6 mS/cm; Elution conditions: Buffer A - 50 mM acetate pH 5.5, ~3 mS/cm, Buffer B- 350 mM acetate pH 5.5, ~24 mS/cm, Gradient: 20-85% over 10 CVs, Pooling: 1-8 CVs.

**Example 12. Gradient Elution on OEC**

Another objective of elution optimization study was to evaluate the effect of gradient elution conditions on OEC mode on Capto Adhere resin. Elution phase was developed for eluting the bound product (Table 18). In a gradient elution run, ionic strength, pH, composition and concentration of the mobile phase can be varied based on the requirements. Table 18 shows the run conditions: both the load (pH and conductivity) and the elution (pH and the conductivity gradient) conditions. All the data shown in the table were obtained from chromatography runs loaded at 150 g/L load density with MAb 3. These runs were done as proof of concept for demonstrating that gradient elution can be performed on OEC mode of chromatography and the gradient slope (concentration of the salt (mM)/Column Volume) can be optimized. It can be seen that the %HMWS is reduced by 38% on an average when compared to the load (Table 18). CHOP was reduced to <20 ppm in 5.5 mS/cm conductivity run and the higher conductivity run at 10 mS/cm, resulted in pool CHOP of ~150 ppm (Table 19).

Table 18. Gradient Elution runs on OEC: %HMWs data

| Load pH | Load Conductivity | Gradient Elution Conditions | Product Bound (g/L) | % HMWS Load | % HMWS Pool |
|---|---|---|---|---|---|
| 6.5 | 5.5 mS/cm | 5.5 mS/cm to 1 mS/cm over 10 CVs | 43 | 4.0 | 2.4 |
| 6.5 | 5.5 mS/cm | 5.5 mS/cm to 1mS/cm over 15 CVs | 43 | 4.0 | 2.8 |
| 6.5 | 10 mS/cm | 10 mS/cm to 1mS/cm over 5 CVs | 48 | 3.8 | 2.4 |
| 6.5 | 10 mS/cm | 10 mS/cm to 1mS/cm over 10 CVs | 48 | 3.9 | 2.2 |

Table 19. Gradient Elution runs on OEC: CHOP data

| Load pH | Load Conductivity | Gradient Elution Conditions | Load CHOP (ppm) | Pool CHOP (ppm) |
|---|---|---|---|---|
| 6.5 | 5.5 mS/cm | 5.5 mS/cm to 1 mS/cm over 10 CVs | 3100 | 17 |
| 6.5 | 5.5 mS/cm | 5.5 mS/cm to 1mS/cm over 15 CVs | 3100 | 19 |
| 6.5 | 10 mS/cm | 10 mS/cm to 1mS/cm over 5 CVs | 3100 | 160 |
| 6.5 | 10 mS/cm | 10 mS/cm to 1mS/cm over 10 CVs | 3100 | 143 |

**Claims**

1.  A method for purifying a polypeptide from a composition comprising the polypeptide and one or more contaminants, said method comprising

    a) loading the composition onto a chromatography material in an amount in excess of the dynamic binding capacity of the chromatography material for the polypeptide using a loading buffer, wherein the partition coefficient of the chromatography material for the polypeptide is greater than 100,
    b) eluting the polypeptide from the chromatography material under conditions wherein the one or more contaminants remain bound to the chromatography material using an elution buffer, wherein the elution buffer has a conductivity less than the conductivity of the loading buffer, and wherein the elution buffer has a conductivity less than 4.0 mS/cm,

c) pooling fractions comprising the polypeptide in the chromatography effluent from steps a) and b).

2. The method of claim 1, wherein the loading buffer has a conductivity greater than 5 mS/cm.

3. The method of claim 1, wherein the polypeptide is (i) an antibody or immunoadhesin, or (ii) an enzyme, a hormone, a fusion protein, an Fc-containing protein, an immunoconjugate, a cytokine or an interleukin.

4. The method of claim 3, wherein the antibody is (i) a monoclonal antibody, in particular wherein the monoclonal antibody is a chimeric antibody, humanized antibody, or human antibody, or (ii) an antigen binding fragment, optionally wherein the antigen binding fragment is a Fab fragment, a Fab' fragment, a F(ab')$_2$ fragment, a scFv, a di-scFv, a bi-scFv, a tandem (di, tri)-scFv, a Fv, asdAb, a tri-functional antibody, a BiTE, a diabody or a triabody.

5. The method of any one of claims 1-4, wherein the at least one contaminant is any one or more of Chinese Hamster Ovary Protein (CHOP), a host cell protein (HCP), leached protein A, carboxypeptidase B, nucleic acids, DNA, product variants, aggregated protein, cell culture media component, gentamicin, polypeptide fragment, endotoxin and viral contaminant.

6. The method of claim 5 wherein the at least one contaminant is any one of CHOP, HCP, leached protein A, DNA, aggregated protein, cell culture media component or gentamicin, and wherein the contaminant is reduced by greater than any of 10%, 20%, 30%, 40%, 50%, 60%, 70%, 80%, 90%, or 95%.

7. The method of any one of claims 1-6, wherein the chromatography material is a mixed mode material, an ion exchange material, or a hydrophobic interaction material.

8. The method of claim 7, wherein the ion exchange material is an anion exchange material or a cation exchange material.

9. The method of claim 7, wherein the mixed mode material:

(a) comprises functional groups capable of one or more of the following functionalities: anionic exchange, cation exchange, hydrogen bonding, and hydrophobic interactions; or
(b) comprises functional groups capable of anionic exchange and hydrophobic interactions.

10. The method of any one of claims 1-7, wherein the chromatography material is not a hydroxyapatite material.

11. The method of any one of claims 1-10, wherein the loading density of the polypeptide is (a) greater than about 30 g/L of the chromatography material or (b) is between about 50 g/L to about 2000 g/L of the chromatography material.

12. The method of any one of claims 1-11 wherein the composition is loaded onto the chromatography material at about the dynamic binding capacities of the chromatography materials for the one or more contaminants.

13. The method of any one of claims 1-11, wherein the composition is loaded onto the chromatography material at less than the dynamic binding capacity of the chromatography material for the one or more contaminants and exceeding the binding capacity of the chromatography material for the product.

14. The method of any one of claims 1-11, wherein the composition is loaded on the chromatography material at 20-times the dynamic binding capacity of the chromatography material for the polypeptide.

15. The method of any one of the preceding claims providing at least about 95% yield.

16. The method of any one of claims 1-15, wherein (i) the elution buffer has a pH less than the pH of the loading buffer, in particular wherein the load buffer has a pH of about 4 to about 9 and the elution buffer has a pH of about 4 to about 9, or (ii) the elution buffer has a pH greater than the pH of the loading buffer, in particular wherein the load buffer has a pH of about 4 to about 9 and the elution buffer has a pH of about 4 to about 9.

17. The method of any one of claims 1-16, wherein the composition is an eluent from an affinity chromatography, a cation exchange chromatography, an anion exchange chromatography, a mixed mode chromatography or a hydrophobic interaction chromatography.

18. The method of claim 17, wherein the affinity chromatography is a Protein A chromatography.

19. The method of any one of the preceding claims, wherein the chromatography material is a chromatography column.

20. The method of claim 19, wherein the chromatography column is an industrial scale chromatography column.

**Patentansprüche**

1. Verfahren zum Reinigen eines Polypeptids aus einer Zusammensetzung, umfassend das Polypeptid und eine oder mehrere Verunreinigungen, wobei das Verfahren Folgendes umfasst:

   a) Laden der Zusammensetzung unter Verwendung eines Ladepuffers auf ein Chromatographiematerial in einer Menge, die die dynamische Bindungskapazität des Chromatographiematerials für das Polypeptid übersteigt, wobei der Verteilungskoeffizient des Chromatographiematerials für das Polypeptid größer als 100 ist,
   b) Eluieren des Polypeptids von dem Chromatographiematerial unter Bedingungen, bei denen die eine oder die mehreren Verunreinigungen unter Verwendung eines Elutionspuffers an das Chromatographiematerial gebunden bleiben, wobei der Elutionspuffer eine Leitfähigkeit aufweist, die geringer als die Leitfähigkeit des Ladepuffers ist, und wobei der Elutionspuffer eine Leitfähigkeit von weniger als 4,0 mS/cm aufweist,
   c) Vereinigen von Fraktionen, umfassend das Polypeptid in dem Chromatographieeffluenten aus den Schritten a) und b).

2. Verfahren nach Anspruch 1, wobei der Ladepuffer eine Leitfähigkeit von mehr als 5 mS/cm aufweist.

3. Verfahren nach Anspruch 1, wobei das Polypeptid (i) ein Antikörper oder Immunadhäsin oder (ii) ein Enzym, ein Hormon, ein Fusionsprotein, ein Fc-enthaltendes Protein, ein Immunkonjugat, ein Zytokin oder ein Interleukin ist.

4. Verfahren nach Anspruch 3, wobei der Antikörper (i) ein monoklonaler Antikörper ist, insbesondere wobei der monoklonale Antikörper ein chimärer Antikörper, humanisierter Antikörper oder humaner Antikörper ist, oder (ii) ein antigenbindendes Fragment ist, optional wobei das antigenbindende Fragment ein Fab-Fragment, ein Fab'-Fragment, ein F(ab')$_2$-Fragment, ein scFv, ein di-scFv, ein bi-scFv, ein Tandem(di, tri)-scFv, ein Fv, asdAb, ein trifunktioneller Antikörper, ein BiTE, ein Diabody oder ein Triabody ist.

5. Verfahren nach einem der Ansprüche 1-4, wobei die mindestens eine Verunreinigung eine oder mehrere aus Protein aus dem Eierstock des chinesischen Hamsters (CHOP), einem Wirtszellprotein (HCP), ausgelaugtem Protein A, Carboxypeptidase B, Nukleinsäuren, DNA, Produktvarianten, aggregiertem Protein, Zellkulturmedienkomponente, Gentamicin, Polypeptidfragment, Endotoxin und viraler Verunreinigung ist.

6. Verfahren nach Anspruch 5, wobei die mindestens eine Verunreinigung eine aus CHOP, HCP, ausgelaugtem Protein A, DNA, aggregiertem Protein, Zellkulturmedienkomponente oder Gentamicin ist, und wobei die Verunreinigung um mehr als 10 %, 20 %, 30 %, 40 %, 50 %, 60 %, 70 %, 80 %, 90 % oder 95 % reduziert ist.

7. Verfahren nach einem der Ansprüche 1-6, wobei das Chromatographiematerial ein Mischmodusmaterial, ein Ionenaustauschmaterial oder ein hydrophobes Interaktionsmaterial ist.

8. Verfahren nach Anspruch 7, wobei das Ionenaustauschmaterial ein Anionenaustauschmaterial oder ein Kationenaustauschmaterial ist.

9. Verfahren nach Anspruch 7, wobei das Mischmodusmaterial:

   (a) funktionelle Gruppen umfasst, die zu einer oder mehreren der folgenden Funktionalitäten fähig sind: Anionenaustausch, Kationenaustausch, Wasserstoffbindung und hydrophobe Interaktionen; oder
   (b) funktionelle Gruppen umfasst, die zu Anionenaustausch und hydrophoben Interaktionen fähig sind.

10. Verfahren nach einem der Ansprüche 1-7, wobei das Chromatographiematerial kein Hydroxyapatitmaterial ist.

11. Verfahren nach einem der Ansprüche 1-10, wobei die Ladedichte des Polypeptids (a) größer als etwa 30 g/l des Chromatographiematerials ist oder (b) zwischen etwa 50 g/l bis etwa 2000 g/l des Chromatographiematerials liegt.

12. Verfahren nach einem der Ansprüche 1-11, wobei die Zusammensetzung bei etwa den dynamischen Bindungskapazitäten der Chromatographiematerialien für die eine oder die mehreren Verunreinigung(en) auf das Chromatographiematerial geladen wird.

13. Verfahren nach einem der Ansprüche 1-11, wobei die Zusammensetzung bei weniger als der dynamischen Bindungskapazität des Chromatographiematerials für die eine oder die mehreren Verunreinigung(en) auf das Chromatographiematerial geladen wird und die Bindungskapazität des Chromatographiematerials für das Produkt übersteigt.

14. Verfahren nach einem der Ansprüche 1-11, wobei die Zusammensetzung bei dem 20-Fachen der dynamischen Bindungskapazität des Chromatographiematerials für das Polypeptid auf das Chromatographiematerial geladen wird.

15. Verfahren nach einem der vorhergehenden Ansprüche, das eine Ausbeute von mindestens etwa 95 % liefert.

16. Verfahren nach einem der Ansprüche 1-15, wobei (i) der Elutionspuffer einen pH-Wert aufweist, der geringer als der pH-Wert des Ladepuffers ist, insbesondere wobei der Ladepuffer einen pH-Wert von etwa 4 bis etwa 9 aufweist und der Elutionspuffer einen pH-Wert von etwa 4 bis etwa 9 aufweist, oder (ii) der Elutionspuffer einen pH-Wert aufweist, der größer als der pH-Wert des Ladepuffers ist, insbesondere wobei der Ladepuffer einen pH-Wert von etwa 4 bis etwa 9 aufweist und der Elutionspuffer einen pH-Wert von etwa 4 bis etwa 9 aufweist.

17. Verfahren nach einem der Ansprüche 1-16, wobei die Zusammensetzung ein Eluent aus einer Affinitätschromatographie, einer Kationenaustauschchromatographie, einer Anionenaustauschchromatographie, einer Mischmoduschromatographie oder einer hydrophoben Interaktionschromatographie ist.

18. Verfahren nach Anspruch 17, wobei die Affinitätschromatographie eine Protein A-Chromatographie ist.

19. Verfahren nach einem der vorhergehenden Ansprüche, wobei das Chromatographiematerial eine Chromatographiesäule ist.

20. Verfahren nach Anspruch 19, wobei die Chromatographiesäule eine Chromatographiesäule im industriellen Maßstab ist.

**Revendications**

1. Procédé de purification d'un polypeptide à partir d'une composition comprenant le polypeptide et un ou plusieurs contaminants, ledit procédé comprenant

   a) le chargement de la composition sur un matériau de chromatographie en une quantité en excès par rapport à la capacité de liaison dynamique du matériau de chromatographie pour le polypeptide en utilisant un tampon de charge, dans lequel le coefficient de partage du matériau de chromatographie pour le polypeptide est supérieur à 100,
   b) l'élution du polypeptide du matériau de chromatographie dans des conditions dans lesquelles le ou les contaminants restent liés au matériau de chromatographie en utilisant un tampon d'élution, dans lequel le tampon d'élution a une conductivité inférieure à la conductivité du tampon de charge, et dans lequel le tampon d'élution a une conductivité inférieure à 4,0 mS/cm,
   c) le rassemblement des fractions comprenant le polypeptide dans l'effluent de chromatographie des étapes a) et b).

2. Procédé selon la revendication 1, dans lequel le tampon de charge a une conductivité supérieure à 5 mS/cm.

3. Procédé selon la revendication 1, dans lequel le polypeptide est (i) un anticorps ou une immunoadhésine, ou (ii) une enzyme, une hormone, une protéine de fusion, une protéine contenant Fc, un immunoconjugué, une cytokine ou une interleukine.

4. Procédé selon la revendication 3, dans lequel l'anticorps est (i) un anticorps monoclonal, en particulier dans lequel l'anticorps monoclonal est un anticorps chimère, un anticorps humanisé ou un anticorps humain, ou (ii) un fragment de liaison à l'antigène, éventuellement dans lequel le fragment de liaison à l'antigène est un fragment Fab, un fragment Fab', un fragment $F(ab')_2$, un scFv, un di-scFv, un bi-scFv, un (di, tri)-scFv tandem, un Fv, un sdAb, un anticorps tri-fonctionnel, un BiTE, un diacorps ou un triacorps.

5. Procédé selon l'une quelconque des revendications 1 à 4, dans lequel l'au moins un contaminant est l'un quelconque ou plusieurs parmi une protéine d'ovaire d'hamster chinois (CHOP), une protéine de cellule hôte (HCP), une protéine A lixiviée, une carboxypeptidase B, des acides nucléiques, de l'ADN, des variantes de produits, une protéine agrégée, un composant de milieu de culture cellulaire, la gentamicine, un fragment de polypeptide, une endotoxine et un contaminant viral.

6. Procédé selon la revendication 5 dans lequel l'au moins un contaminant est l'un quelconque parmi une CHOP, une HCP, une protéine A lixiviée, de l'ADN, une protéine agrégée, un composant de milieu de culture cellulaire ou la gentamicine, et dans lequel le contaminant est réduit de plus d'un quelconque parmi 10 %, 20 %, 30 %, 40 %, 50 %, 60 %, 70 %, 80 %, 90 % ou 95 %.

7. Procédé selon l'une quelconque des revendications 1 à 6, dans lequel le matériau de chromatographie est un matériau en mode mixte, un matériau d'échange d'ions ou un matériau d'interaction hydrophobe.

8. Procédé selon la revendication 7, dans lequel le matériau d'échange d'ions est un matériau d'échange d'anions ou un matériau d'échange de cations.

9. Procédé selon la revendication 7, dans lequel le matériau en mode mixte :

(a) comprend des groupes fonctionnels susceptibles d'une ou plusieurs des fonctionnalités suivantes : échange d'anions, échange de cations, liaison hydrogène et interactions hydrophobes ; ou
(b) comprend des groupes fonctionnels susceptibles d'un échange d'anions et d'interactions hydrophobes.

10. Procédé selon l'une quelconque des revendications 1 à 7, dans lequel le matériau de chromatographie n'est pas un matériau d'hydroxyapatite.

11. Procédé selon l'une quelconque des revendications 1 à 10, dans lequel la densité de charge du polypeptide est (a) supérieure à environ 30 g/L du matériau de chromatographie ou (b) est entre environ 50 g/L et environ 2000 g/L du matériau de chromatographie.

12. Procédé selon l'une quelconque des revendications 1 à 11 dans lequel la composition est chargée sur le matériau de chromatographie à des capacités correspondant à environ les capacités de liaison dynamique des matériaux de chromatographie pour le ou les contaminants.

13. Procédé selon l'une quelconque des revendications 1 à 11, dans lequel la composition est chargée sur le matériau de chromatographie à une capacité inférieure à la capacité de liaison dynamique du matériau de chromatographie pour le ou les contaminants et excédant la capacité de liaison du matériau de chromatographie pour le produit.

14. Procédé selon l'une quelconque des revendications 1 à 11, dans lequel la composition est chargée sur le matériau de chromatographie à une capacité correspondant à 20 fois la capacité de liaison dynamique du matériau de chromatographie pour le polypeptide.

15. Procédé selon l'une quelconque des revendications précédentes fournissant un rendement d'au moins environ 95 %.

16. Procédé selon l'une quelconque des revendications 1 à 15, dans lequel (i) le tampon d'élution a un pH inférieur au pH du tampon de charge, en particulier dans lequel le tampon de charge a un pH d'environ 4 à environ 9 et le tampon d'élution a un pH d'environ 4 à environ 9, ou (ii) le tampon d'élution a un pH supérieur au pH du tampon de charge, en particulier dans lequel le tampon de charge a un pH d'environ 4 à environ 9 et le tampon d'élution a un pH d'environ 4 à environ 9.

17. Procédé selon l'une quelconque des revendications 1 à 16, dans lequel la composition est un éluant d'une chromatographie d'affinité, d'une chromatographie à échange de cations, d'une chromatographie à échange d'anions, d'une chromatographie en mode mixte ou d'une chromatographie à interaction hydrophobe.

18. Procédé selon la revendication 17, dans lequel la chromatographie d'affinité est une chromatographie à protéine A.

19. Procédé selon l'une quelconque des revendications précédentes, dans lequel le matériau de chromatographie est une colonne de chromatographie.

20. Procédé selon la revendication 19, dans lequel la colonne de chromatographie est une colonne de chromatographie à l'échelle industrielle.

Figure 1

Figure 2

Figure 3

EP 3 527 274 B1

Figure 4

EP 3 527 274 B1

Figure 5

Figure 6

## a. Analysis in Fractions

## b. Cumulative Analysis

EP 3 527 274 B1

Figure 7

Figure 8

EP 3 527 274 B1

Figure 9

Figure 10

Figure 11

Figure 12

EP 3 527 274 B1

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070060741
- US 2011037977 W
- US 5641870 A
- EP 404097 A
- WO 9311161 A
- WO 2005035572 A
- WO 03035694 A
- WO 0029004 A
- WO 02051870 A
- US 4816567 A
- US 5693780 A
- US 5500362 A
- US 5821337 A
- US 5534615 A
- WO 9411026 A
- US 7521541 B
- US 5591669 A
- US 5589369 A
- US 5545807 A
- US 5565332 A
- US 5573905 A
- US 5567610 A
- US 5229275 A
- WO 9316185 A
- US 5571894 A
- US 5587458 A
- WO 9308829 A
- WO 9404690 A
- US 5731168 A
- US 4676980 A
- WO 9100360 A
- WO 92200373 A
- EP 03089 A
- US 20060067930 A
- US 5208020 A
- US 5712374 A
- EP 402226 A

**Non-patent literature cited in the description**

- **KELLEY, BD et al.** *Biotechnol Bioeng*, 2008, vol. 101 (3), 553-566
- **GAZZANO-SANTORO et al.** *J. Immunol. Methods*, 1996, vol. 202, 163
- **KABAT et al.** Sequences of Proteins of Immunological Interest. 1991
- **CHOTHIA ; LESK**. *J. Mol. Biol.*, 1987, vol. 196, 901-917
- **ZAPATA et al.** *Protein Eng.*, 1995, vol. 8 (10), 1057-1062
- **PLÜCKTHUN**. The Pharmacology of Monoclonal Antibodies. Springer-Verlag, 1994, vol. 113, 269-315
- **HOLLINGER et al.** *Proc. Natl. Acad. Sci. USA*, 1993, vol. 90, 6444-6448
- *Nature*, 1989, vol. 341, 544-546
- *Dev Comp Immunol*, 2006, vol. 30, 43-56
- *Trend Biochem Sci*, 2001, vol. 26, 230-235
- *Trends Biotechnol*, 2003, vol. 21, 484-490
- *Febs Lett*, 1994, vol. 339, 285-290
- **KOHLER et al.** *Nature*, 1975, vol. 256, 495
- **CLACKSON et al.** *Nature*, 1991, vol. 352, 624-628
- **MARKS et al.** *J. Mol. Biol.*, 1991, vol. 222, 581-597
- **MORRISON et al.** *Proc. Natl. Acad. Sci. USA*, 1984, vol. 81, 6851-6855
- **JONES et al.** *Nature*, 1986, vol. 321, 522-525
- **RIECHMANN et al.** *Nature*, 1988, vol. 332, 323-329
- **PRESTA**. *Curr. Op. Struct. Biol.*, 1992, vol. 2, 593-596
- **RAVETCH ; KINET**. *Annu. Rev. Immunol*, 1991, vol. 9, 457-92
- **CLYNES et al.** *Proc. Natl. Acad. Sci. (USA)*, 1998, vol. 95, 652-656
- **DAËRON**. *Annu. Rev. Immunol.*, 1997, vol. 15, 203-234
- **CAPEL et al.** *Immunomethods*, 1994, vol. 4, 25-34
- **HAAS et al.** *J. Lab. Clin. Med.*, 1995, vol. 126, 330-41
- **GUYER et al.** *J. Immunol.*, 1976, vol. 117, 587
- **KIM et al.** *J. Immunol.*, 1994, vol. 24, 249
- **CARTER et al.** *BiolTechnology*, 1992, vol. 10, 163-167
- **GODING**. Monoclonal Antibodies: Principles and Practice. Academic Press, 59-103
- **KOZBOR**. *J. Immunol.*, 1984, vol. 133

- **BRODEUR et al.** Monoclonal Antibody Production Techniques and Applications. Marcel Dekker, Inc., 1987, 51-63
- **MUNSON et al.** *Anal. Biochem.*, 1980, vol. 107, 220
- **GODING**. Monoclonal Antibodies: Principles and Practice. Academic Press, 1986, 59-103
- **SKERRA et al.** *Curr. Opinion in Immunol.*, 1993, vol. 5, 256-262
- **PLÜCKTHUN**. *Immunol. Revs.*, 1992, vol. 130, 151-188
- **MCCAFFERTY et al.** *Nature*, 1990, vol. 348, 552-554
- **MARKS et al.** *BiolTechnology*, 1992, vol. 10, 779-783
- **WATERHOUSE et al.** *Nuc. Acids. Res.*, 1993, vol. 21, 2265-2266
- **MORRISON et al.** *Proc. Natl Acad. Sci. USA*, 1984, vol. 81, 6851
- **RIECHMANN et al.** *Nature*, 1988, vol. 332, 323-327
- **VERHOEYEN et al.** *Science*, 1988, vol. 239, 1534-1536
- **SIMS et al.** *J. Immunol.*, 1993, vol. 151, 2296
- **CHOTHIA et al.** *J. Mol. Biol.*, 1987, vol. 196, 901
- **CARTER et al.** *Proc. Natl. Acad. Sci. USA*, 1992, vol. 89, 4285
- **PRESTA et al.** *J. Immunol.*, 1993, vol. 151, 2623
- **JAKOBOVITS et al.** *Proc. Natl. Acad. Sci. USA*, 1993, vol. 90, 2551
- **JAKOBOVITS et al.** *Nature*, 1993, vol. 362, 255-258
- **BRUGGERMANN et al.** *Year in Immuno.*, 1993, vol. 7, 33
- **MCCAFFERTY et al.** *Nature*, 1990, vol. 348, 552-553
- **JOHNSON, KEVIN S.** ; **CHISWELL, DAVID J.** *Current Opinion in Structural Biology*, 1993, vol. 3, 564-571
- **GRIFFITH et al.** *EMBO J.*, 1993, vol. 12, 725-734
- **MORIMOTO et al.** *Journal of Biochemical and Biophysical Methods*, 1992, vol. 24, 107-117
- **BRENNAN et al.** *Science*, 1985, vol. 229, 81
- **MILLSTEIN et al.** *Nature*, 1983, vol. 305, 537-539
- **TRAUNECKER et al.** *EMBO J.*, 1991, vol. 10, 3655-3659
- **SURESH et al.** *Methods in Enzymology*, 1986, vol. 121, 210
- **KOSTELNY et al.** *J. Immunol.*, 1992, vol. 148 (5), 1547-1553
- **GRUBER et al.** *J. Immunol.*, 1994, vol. 152, 5368
- **TUTT et al.** *J. Immunol.*, 1991, vol. 147, 60
- **CARON et al.** *J. Exp Med.*, 1992, vol. 176, 1191-1195
- **SHOPES, B.** *J. Immunol.*, 1992, vol. 148, 2918-2922
- **WOLFF et al.** *Cancer Research*, 1993, vol. 53, 2560-2565
- **STEVENSON et al.** *Anti-Cancer Drug Design*, 1989, vol. 3, 219-230
- **CUNNINGHAM** ; **WELLS**. *Science*, 1989, vol. 244, 1081-1085
- **A. L. LEHNINGER**. Biochemistry. Worth Publishers, 1975, 73-75
- **VITETTA et al.** *Science*, 1987, vol. 238, 1098
- Remington's Pharmaceutical Sciences. 1990
- **STEWART et al.** *Solid-Phase Peptide Synthesis W.H. Freeman Co.*, 1969
- **MERRIFIELD**. *J. Am. Chem. Soc.*, 1963, vol. 85, 2149-2154
- **SAMBROOK et al.** Molecular Cloning: A Laboratory Manual. Cold Spring Harbor Press, 1989
- **WINNACKER**. From Genes to Clones. VCH Publishers, 1987
- Remington's Pharmaceutical Sciences. 1980